# EUROPÄISCHE PATENTSCHRIFT

(11) **EP 2 740 165 B1**
(45) Veröffentlichungstag und Bekanntmachung des Hinweises auf die Patenterteilung: **07.11.2018**
(21) Anmeldenummer: 12734810.0
(22) Anmeldetag: 05.07.2012
(51) Int. Cl.: H01L 51/54, C09K 11/06

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 03.08.2011 EP 11006384
(43) Veröffentlichungstag der Anmeldung: 11.06.2014
(60) Teilanmeldung: 18194164.2
(73) Patentinhaber: Merck Patent GmbH, 64293 Darmstadt (DE)
(72) Erfinder: PARHAM, Amir Hossain, 65929 Frankfurt am Main (DE); BUESING, Arne, 65929 Frankfurt am Main (DE); PFLUMM, Christof, 64291 Darmstadt (DE); MUJICA-FERNAUD, Teresa, 64289 Darmstadt (DE); STOESSEL, Philipp, 60487 Frankfurt am Main (DE); EBERLE, Thomas, 76829 Landau (DE); VOGES, Frank, 67098 Bad Duerkheim (DE)
(86) Internationale Anmeldenummer: PCT/EP2012/002835
(87) Internationale Veröffentlichungsnummer: WO 2013/017192

(56) Entgegenhaltungen:
- EP-A2- 2 182 040
- WO-A2-2010/110553
- JP-A- 2000 056 491
- JP-A- 2009 040 730
- US-A1- 2005 221 124
- US-A1- 2010 025 669

## Beschreibung

Die vorliegende Erfindung betrifft eine Verbindung der Formel (I), (II) oder (III), die Verwendung der Verbindung in einer elektronischen Vorrichtung, sowie eine elektronische Vorrichtung enthaltend eine Verbindung der Formel (I), (II) oder (III). Weiterhin betrifft die vorliegende Erfindung ein Verfahren zur Herstellung einer Verbindung der Formel (I), (II) oder (III) sowie eine Formulierung enthaltend eine oder mehrere Verbindungen der Formel (I), (II) oder (III).

Die Entwicklung von funktionellen Verbindungen zur Verwendung in elektronischen Vorrichtungen ist aktuell Gegenstand intensiver Forschung. Ziel ist hierbei insbesondere die Entwicklung von Verbindungen, mit denen verbesserte Eigenschaften der elektronischen Vorrichtungen in einem oder mehreren relevanten Punkten erzielt werden können, wie beispielsweise Leistungseffizienz, Lebensdauer oder Farbkoordinaten des emittierten Lichts.

Unter dem Begriff elektronische Vorrichtung werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs) verstanden.

Von besonderem Interesse ist die Bereitstellung von Verbindungen zur Verwendung in den zuletzt genannten, als OLEDs bezeichneten elektronischen Vorrichtungen. Der allgemeine Aufbau sowie das Funktionsprinzip von OLEDs ist dem Fachmann bekannt und unter anderem in US 4539507, US 5151629, EP 0676461 und WO 1998/27136 beschrieben.

Betreffend die Leistungsdaten von OLEDs sind noch weitere Verbesserungen erforderlich, insbesondere in Hinblick auf eine breite kommerzielle Verwendung, beispielsweise in Anzeigevorrichtungen oder als Lichtquellen. Von besonderer Bedeutung sind in diesem Zusammenhang die Lebensdauer, die Effizienz und die Betriebsspannung der OLEDs sowie die realisierten Farbwerte. Insbesondere bei blau emittierenden OLEDs besteht Verbesserungspotential bezüglich der Lebensdauer der Vorrichtungen. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich unzersetzt sublimieren lassen.

In diesem Zusammenhang besteht insbesondere Bedarf an alternativen Lochtransportmaterialien. Bei Lochtransportmaterialien gemäß dem Stand der Technik steigt im Allgemeinen die Spannung mit der Schichtdicke der Lochtransportschicht an. In der Praxis wäre häufig eine höhere Schichtdicke der Lochtransportschicht wünschenswert, dies hat jedoch oftmals eine höhere Betriebsspannung und schlechtere Leistungsdaten zur Folge. In diesem Zusammenhang besteht Bedarf an neuen Lochtransportmaterialien, die eine hohe Ladungsträgerbeweglichkeit aufweisen, so dass dickere Lochtransportschichten mit lediglich geringem Anstieg der Betriebsspannung realisiert werden können.

Im Stand der Technik ist die Verwendung von Arylaminverbindungen und Carbazolverbindungen als Lochtransportmaterialien für OLEDs bekannt. An dieser Stelle soll beispielsweise auf die Offenlegungsschrift EP 1661888 verwiesen werden. Es besteht jedoch weiterhin Bedarf an neuen Lochtransportmaterialien zur Verwendung in OLEDs. Insbesondere besteht Bedarf an Materialien, mit denen die oben genannten Verbesserungen der Leistungsdaten und Eigenschaften der OLEDs erreicht werden können.

In der Offenlegungsschrift WO 2010/064871 werden Verbindungen enthaltend eine Carbazolgruppe sowie eine Fluorenylgruppe offenbart, wobei die genannten Gruppen unmittelbar über eine Aminogruppe miteinander verbunden sind. Die Verbindungen werden zur Verwendung als Dotanden in organischen Elektrolumineszenzvorrichtungen offenbart. Es besteht weiterhin Bedarf an alternativen Verbindungen zur Verwendung als Lochtransportmaterial in OLEDs, insbesondere an solchen, mit denen gute Effizienzen und lange Lebensdauern der Vorrichtungen erreicht werden können.

In der Offenlegungsschrift US 2005/0221124 werden in 2-Position substituierte Fluorenylverbindungen offenbart, welche eine Carbazolgruppe oder Arylaminogruppe tragen. Weiterhin wird in diesem Dokument offenbart, dass die genannten 2-Fluorenylverbindungen zur Verwendung als Lochtransportmaterialien in OLEDs geeignet sind. Insbesondere bei der Verwendung als Lochtransportmaterial in Kombination mit einem phosphoreszierenden Dotanden in der emittierenden Schicht ist es jedoch hoch erwünscht, dass die als Lochtransportmaterial eingesetzte Verbindung ein hochliegendes angeregtes Triplett-Niveau aufweist. Es besteht daher weiterhin Bedarf an alternativen Verbindungen zur Verwendung als Lochtransportmaterial in OLEDs, insbesondere an solchen, mit denen gute Effizienzen und lange Lebensdauern der Vorrichtungen erreicht werden können.

Weiterhin offenbaren WO 2010/110553 A2, EP 2182040 A2, JP 2000-56491 und US 2010/025669 A1 Fluorenyl-Aminverbindungen, und JP 2009-040730 A offenbart Benzo-kondensierte Fluoren-Derivate, die mit einer Aminogruppe substituiert sind.

Weiterhin besteht Bedarf an alternativen Matrixmaterialien zur Verwendung in OLEDs und anderen elektronischen Vorrichtungen. Insbesondere besteht Bedarf an Matrixmaterialien für phosphoreszierende Emitter, die gleichzeitig zu guter Effizienz, hoher Lebensdauer und geringer Betriebsspannung führen. Gerade die Eigenschaften der Matrixmaterialien sind häufig limitierend für die Lebensdauer und die Effizienz der organischen Elektrolumineszenzvorrichtung. Insbesondere ist es für Matrixmaterialien für phosphoreszierende Emitter wünschenswert, dass diese ein hochliegendes angeregtes Triplettniveau aufweisen.

Der vorliegenden Erfindung liegt somit die technische Aufgabe zu Grunde, Verbindungen bereitzustellen, welche sich zur Verwendung in elektronischen Vorrichtungen wie beispielsweise OLEDs eignen, und welche insbesondere als Lochtransportmaterialien und/oder als Matrixmaterialien eingesetzt werden können.

Im Rahmen der vorliegenden Erfindung wurde nun gefunden, dass sich Verbindungen der unten angegebenen Formel (I), (II) oder (III) ausgezeichnet für die oben genannten Verwendungen eignen.

Die erfindungsgemäßen Verbindungen besitzen kennzeichnenderweise eine Fluorenylgruppe, welche in ihrer 1-, 3- oder 4-Position an eine Arylaminogruppe gebunden ist, welche wiederum mit einer weiteren Triarylaminogruppe oder Carbazolgruppe substituiert ist.

Gegenstand der vorliegenden Erfindung ist somit eine Verbindung der Formel (I), (II) oder (III)

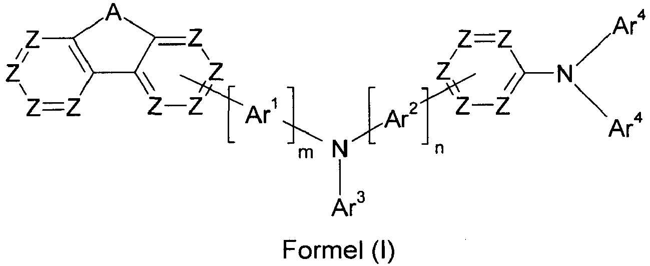

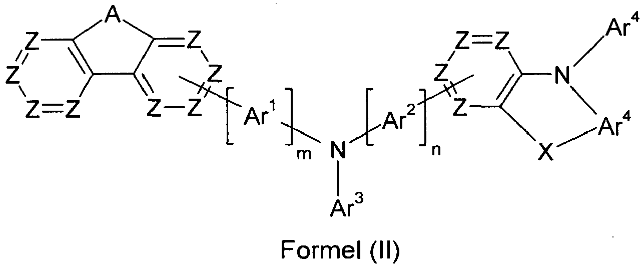

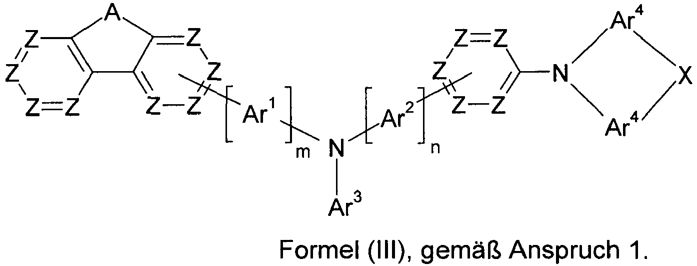

Zur Verdeutlichung wird untenstehend die Strukturformel von Fluoren abgebildet, zusammen mit der Nummerierung der Positionen. Bei Spirobifluorenderivaten ist die Nummerierung analog, mit dem einzigen Unterschied, dass die Position 9 nicht substituiert sein kann.

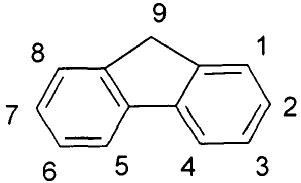

Weiterhin soll hervorgehoben werden, dass für den Fall m=0 bzw. n=0 die betreffende Gruppe Ar¹ bzw. Ar² nicht vorhanden ist und die beiden an diese Gruppe bindenden Molekülteile direkt miteinander verbunden sind.

Ist der Index m bzw. n größer als 1, so bedeutet dies, dass mehrere Gruppen Ar¹ bzw. Ar², genauer gesagt m bzw. n Gruppen Ar¹ bzw. Ar² sequentiell hintereinander gebunden sind, so dass beispielsweise für m=2 und Ar¹ = Phenylen eine Biphenylengruppe entsteht, die die Fluorenylgruppe und die Gruppe N(Ar³) verbindet. Für m=3 und Ar¹ = Phenylen entsteht beispielsweise eine Terphenylengruppe mit hintereinander in Reihe gebundenen Phenylengruppen.

Weiterhin soll angemerkt werden, dass in den Formeln (II) und (III) die Bindung der Gruppe X an die Gruppe Ar⁴ an einer beliebigen Position der Gruppe Ar⁴ erfolgen kann, bevorzugt an einer direkt benachbarten oder an einer nicht mehr als eine Position von einer direkt benachbarten Position entfernten Position, z. B. einer meta-Position, betrachtet von der Bindung der Gruppe Ar⁴ an das Stickstoffatom. Besonders bevorzugt ist die Gruppe X in einer direkt benachbarten Position, betrachtet von der Bindung der Gruppe Ar⁴ an das Stickstoffatom, an die Gruppe Ar⁴ gebunden.

Eine Arylgruppe im Sinne dieser Erfindung enthält 6 bis 60 aromatische Ringatome; eine Heteroarylgruppe im Sinne dieser Erfindung enthält prinzipiell 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenden Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Eine Aralkylgruppe im Sinne dieser Erfindung ist eine mit einer Arylgruppe substituierte Alkylgruppe, wobei der Begriff Arylgruppe wie oben definiert zu verstehen ist und die Alkylgruppe 1 bis 20 C-Atome aufweist, wobei in der Alkylgruppe auch einzelne H-Atome und/oder CH₂-Gruppen durch die bei der Definition der Alkylgruppen genannten Gruppen ersetzt sein können und wobei die Alkylgruppe diejenige Gruppe darstellt, welche an den Rest der Verbindung bindet. Entsprechend stellt eine Heteroaralkylgruppe eine mit einer Heteroarylgruppe substituierte Alkylgruppe dar, wobei der Begriff Heteroarylgruppe wie oben definiert zu verstehen ist und die Alkylgruppe 1 bis 20 C-Atome aufweist, wobei in der Alkylgruppe auch einzelne H-Atome und/oder CH₂-Gruppen durch die bei der Definition der Alkylgruppen genannten Gruppen ersetzt sein können und wobei die Alkylgruppe diejenige Gruppe darstellt, welche an den Rest der Verbindung bindet.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht:

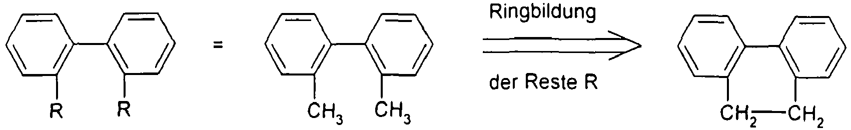

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

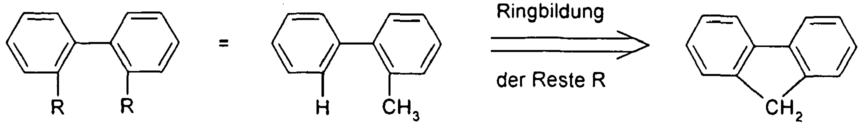

Eine Verbindung der Formel (I) kann erfindungsgemäß eine Verbindung der folgenden Formeln (I-A) bis (I-C) darstellen:

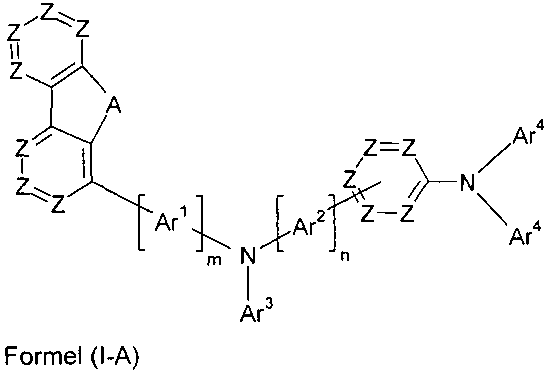

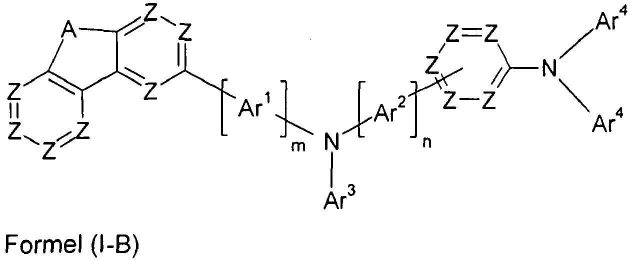

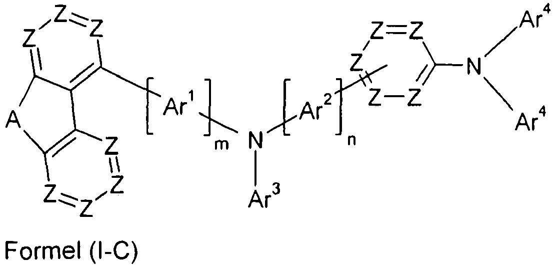

Eine Verbindung der Formel (II) kann erfindungsgemäß eine Verbindung der folgenden Formeln (II-A) bis (II-C) darstellen:

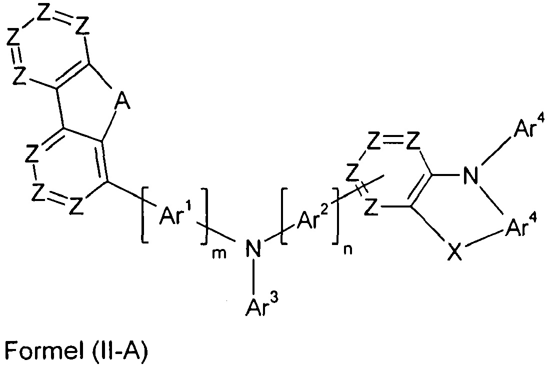

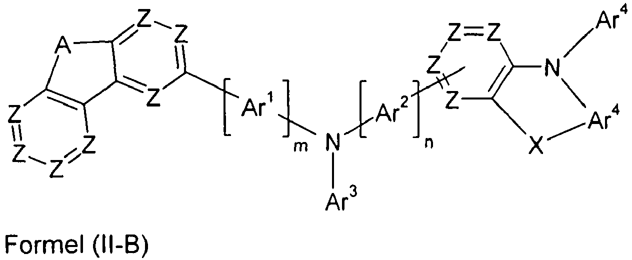

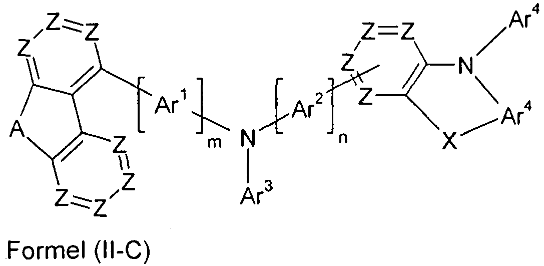

Eine Verbindung der Formel (III) kann erfindungsgemäß eine Verbindung der folgenden Formeln (III-A) bis (III-C) darstellen:

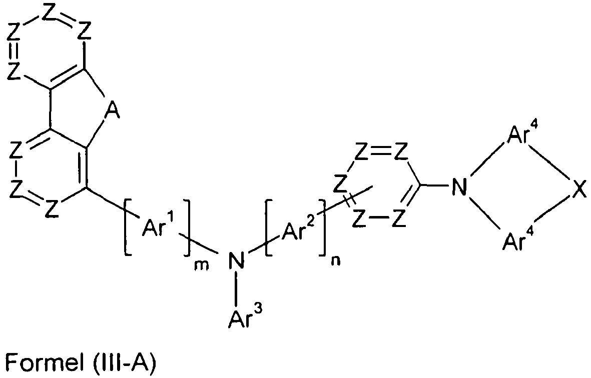

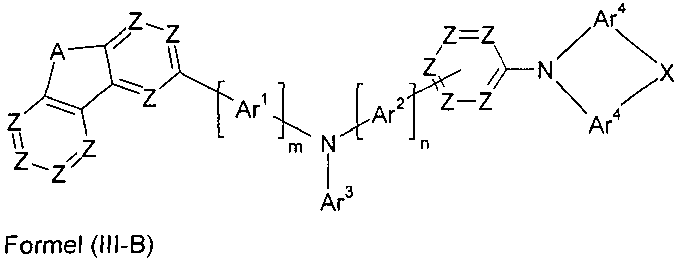

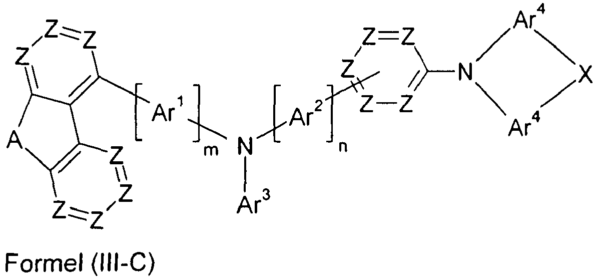

Bevorzugt sind Verbindungen der Formeln (I-B), (II-B) und (III-B).

Es gelten gemäß der vorliegenden Erfindung die folgenden Bevorzugungen für Verbindungen der Formel (I), (II) oder (III).

Bevorzugt ist die Gruppe Ar¹ oder, für den Fall dass m=0 gilt, die Gruppe N(Ar³), in 3-Position am Fluorenyl-Ringsystem gebunden.

Weiterhin bevorzugt bilden Reste R¹, welche Bestandteil einer Gruppe Z sind, für Verbindungen der Formel (II) keine Ringe miteinander. Besonders bevorzugt bilden Reste R¹, welche Bestandteile einer Gruppe Z sind, für Verbindungen der Formel (II) für den Fall, dass X eine Einfachbindung darstellt, keine Ringe miteinander.

Für Ar¹ gilt, dass dieses bevorzugt ein aromatisches oder heteroaromatisches Ringsystem mit 5 bis 24 aromatischen Ringatomen darstellt. Besonders bevorzugt ist Ar¹ ein aromatisches Ringsystem mit 6 bis 18 aromatischen Ringatomen, ganz besonders bevorzugt 6 bis 12 aromatischen Ringatomen. Am stärksten bevorzugt ist Ar¹ eine Arylengruppe mit 6 bis 10 aromatischen Ringatomen. Es gilt allgemein, dass die angegebenen Gruppen mit einem oder mehreren Resten R¹ substituiert sein können.

Für Ar² gilt, dass dieses bevorzugt eine Arylengruppe mit 6 bis 10 aromatischen Ringatomen oder eine Heteroarylengruppe mit 5 bis 14 aromatischen Ringatomen darstellt. Besonders bevorzugt ist Ar² eine Phenylengruppe. Es gilt allgemein, dass die angegebenen Gruppen mit einem oder mehreren Resten R¹ substituiert sein können. Es ist erfindungsgemäß bevorzugt, dass Reste R¹ an Gruppen Ar² keine Ringe miteinander bilden.

Ar⁴ ist bevorzugt eine Arylgruppe mit 6 bis 10 aromatischen Ringatomen. Besonders bevorzugt ist Ar⁴ eine Phenylgruppe. Es gilt allgemein, dass die angegebenen Gruppen mit einem oder mehreren Resten R¹ substituiert sein können.

Die Gruppe X ist bevorzugt ausgewählt aus einer Einfachbindung, C(R¹)₂, O oder S. Ganz besonders bevorzugt ist X eine Einfachbindung.

Bevorzugt ist der Rest R¹ bei jedem Auftreten gleich oder verschieden ausgewählt aus H, einer geradkettigen Alkylgruppe mit 1 bis 20 C-Atomen oder einer verzweigten oder cyclischen Alkylgruppe mit 3 bis 20 C-Atomen, wobei die oben genannten Gruppen jeweils mit einem oder mehreren Resten R² substituiert sein können, oder ein aromatisches Ringsystem mit 5 bis 20 aromatischen Ringatomen, das jeweils mit einem oder mehreren Resten R² substituiert sein kann, wobei zwei oder mehr Reste R¹ miteinander verknüpft sein können und einen Ring bilden können.

Bevorzugt ist der Rest R² bei jedem Auftreten gleich oder verschieden ausgewählt aus H, einer geradkettigen Alkylgruppe mit 1 bis 20 C-Atomen oder einer verzweigten oder
cyclischen Alkylgruppe mit 3 bis 20 C-Atomen, wobei die oben genannten Gruppen jeweils mit einem oder mehreren Resten R³ substituiert sein können, oder ein aromatisches Ringsystem mit 5 bis 20 aromatischen Ringatomen, das jeweils mit einem oder mehreren Resten R³ substituiert sein kann, wobei zwei oder mehr Reste R² miteinander verknüpft sein können und einen Ring bilden können.

Weiterhin ist es bevorzugt, dass m gleich 0 oder 1 ist. Besonders bevorzugt ist m gleich 0.

Für Formel (II) und (III) ist es allgemein bevorzugt, dass n gleich 1, 2 oder 3 ist. Für Formel (II) ist es besonders bevorzugt, dass n gleich 1 ist.

Weiterhin ist es allgemein für die Formeln (I), (II) und (III) bevorzugt, dass n gleich 1, 2 oder 3 ist, besonders bevorzugt gleich 1 oder 2, ganz besonders bevorzugt gleich 1.

Es ist für die erfindungsgemäßen Verbindungen allgemein bevorzugt, dass keine Heteroarylgruppe mit mehr als 10 aromatischen Ringatomen vorhanden ist. Besonders bevorzugt ist keine Heteroarylgruppe mit mehr als 6 aromatischen Ringatomen vorhanden. Ganz besonders bevorzugt enthalten die erfindungsgemäßen Verbindungen keine Heteroarylgruppe gleich welcher Größe.

Weiterhin ist es für die erfindungsgemäßen Verbindungen bevorzugt, dass keine kondensierte Arylgruppe mit mehr als 14 aromatischen Ringatomen in der Verbindung vorhanden ist. Besonders bevorzugt ist keine kondensierte Arylgruppe mit mehr als 10 aromatischen Ringatomen vorhanden.

Weiterhin ist es bevorzugt, dass in Verbindungen der Formel (I) die Gruppe Ar² bzw. die Gruppe N(Ar³) in para-Position zum Stickstoffatom an den unten gezeigten Teil der Verbindung gebunden ist

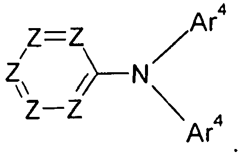

Weiterhin ist es bevorzugt, dass in Verbindungen der Formel (II) die Gruppe Ar² bzw. die Gruppe N(Ar³) in para-Position zum Stickstoffatom an den unten gezeigten Teil der Verbindung gebunden ist

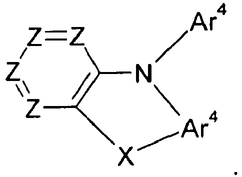

Weiterhin ist es bevorzugt, dass in Verbindungen der Formel (III) die Gruppe Ar² bzw. die Gruppe N(Ar³) in para-Position zum Stickstoffatom an den unten gezeigten Teil der Verbindung gebunden ist

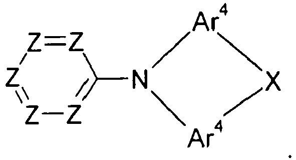

Weiterhin bevorzugt ist die Verbindung der Formel (I), (II) oder (II) asymmetrisch. Weiterhin bevorzugt kann die Verbindung nicht in einer spiegelsymmetrischen Strukturformel dargestellt werden.

Weiterhin ist es bevorzugt, dass die Verbindung der Formel (I), (II) oder (III) zusätzlich zu den beiden abgebildeten Triarylaminogruppen keine weitere Triarylaminogruppe aufweist. Es ist ebenfalls bevorzugt, dass die Verbindung kein weiteres Carbazolderivat enthält außer solchen, welche sich in Formel (II) oder (III) für X als Einfachbindung ergeben.

Bevorzugte Ausführungsformen von Formel (I) entsprechen den folgenden Formeln (I-25) bis (I-48) 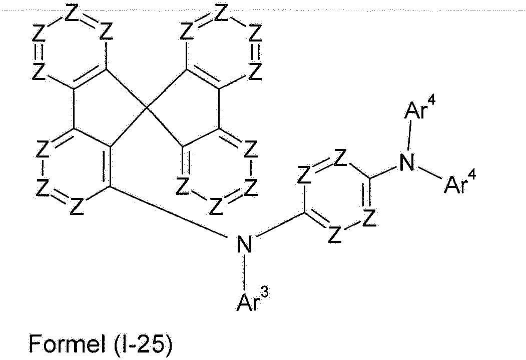 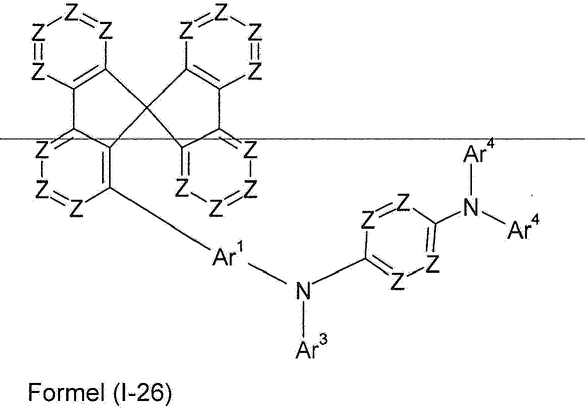 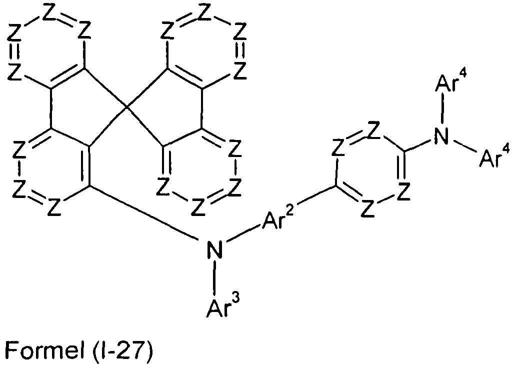 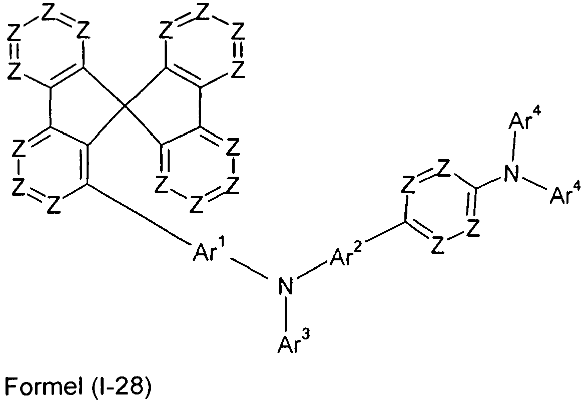 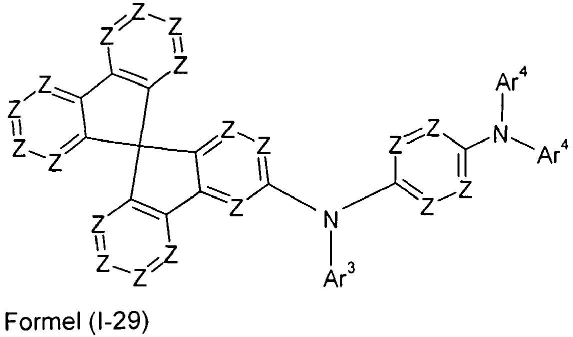 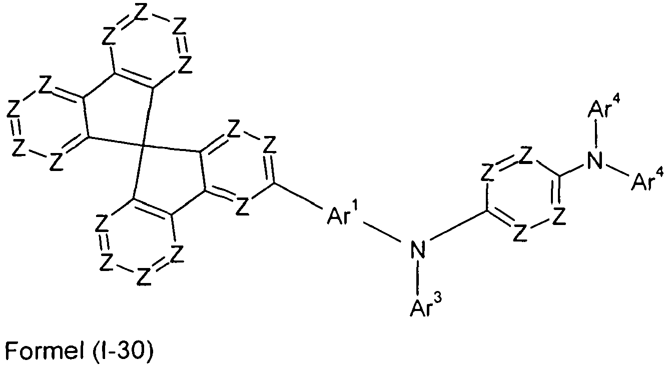 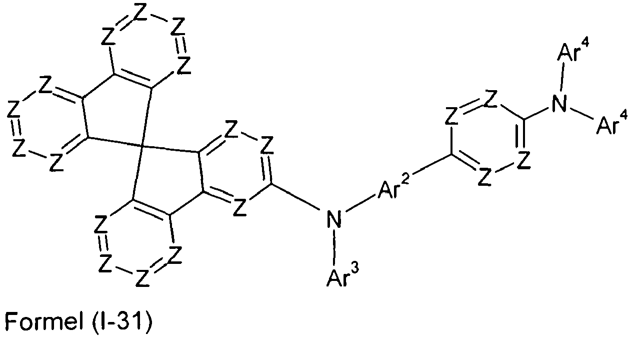 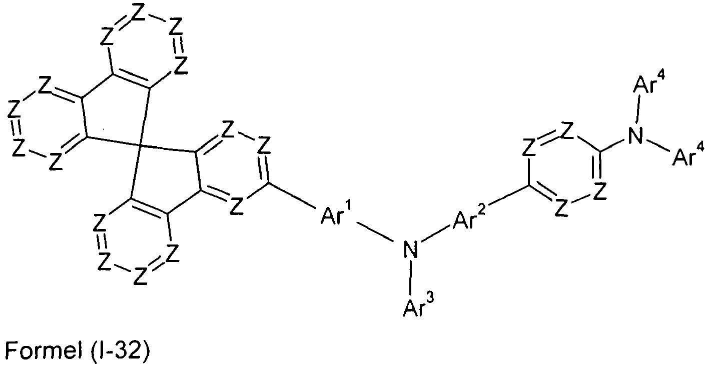 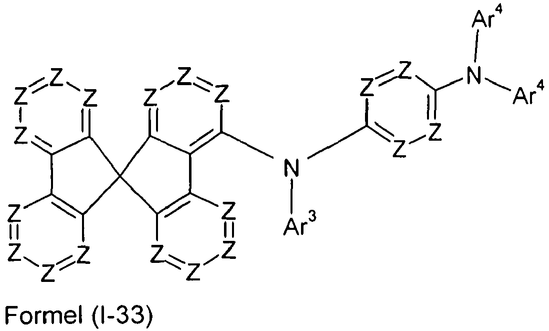 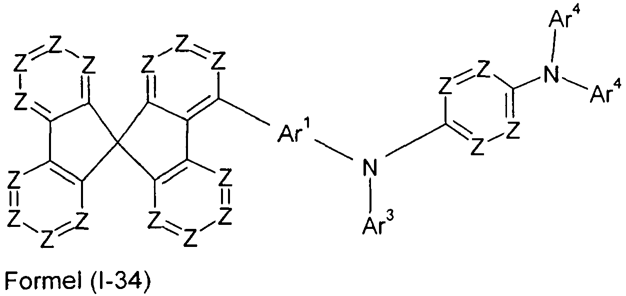 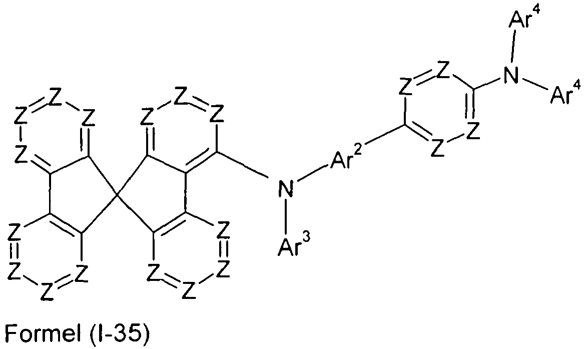 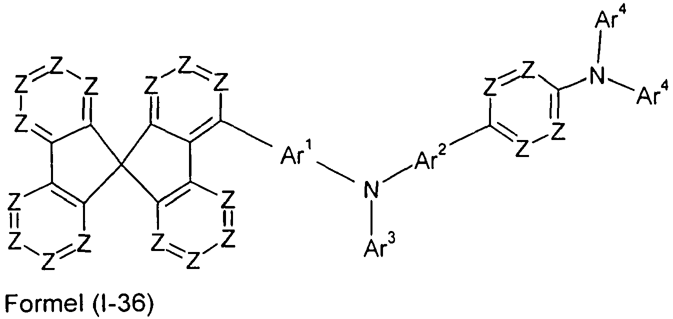 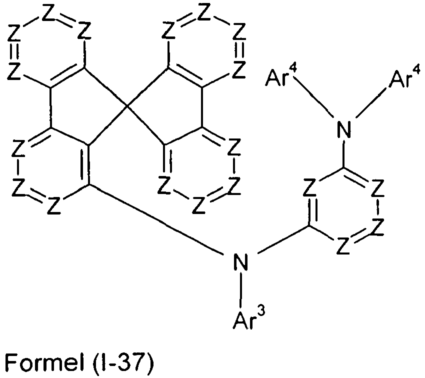 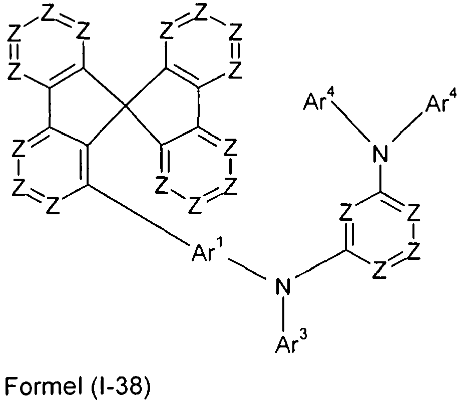 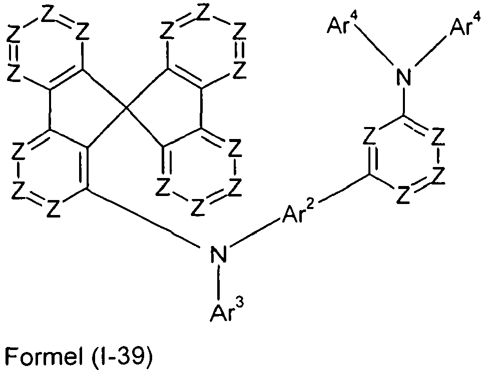 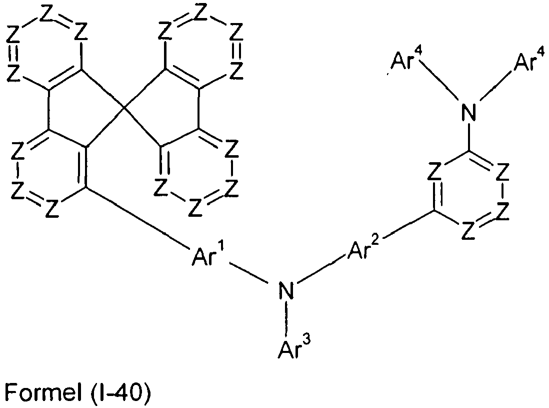 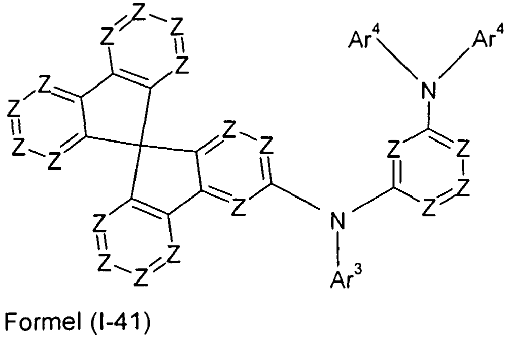 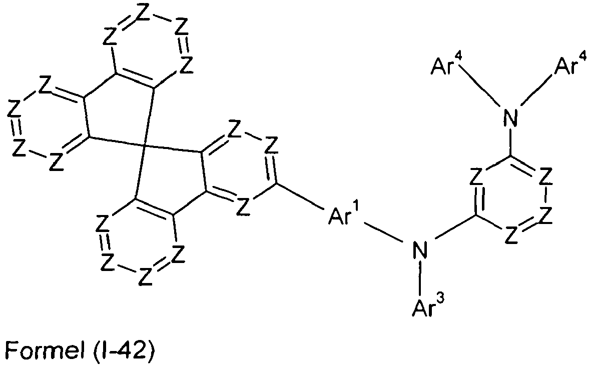 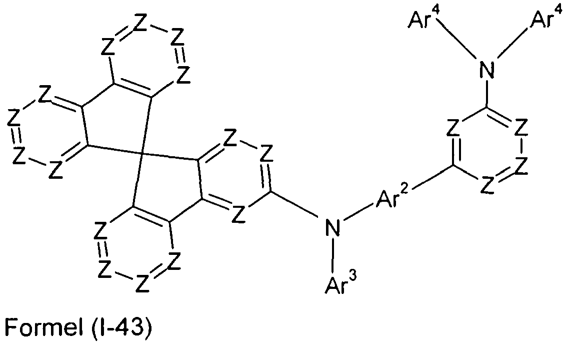 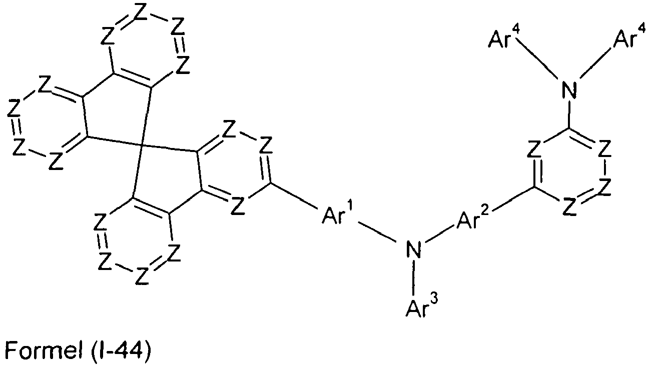 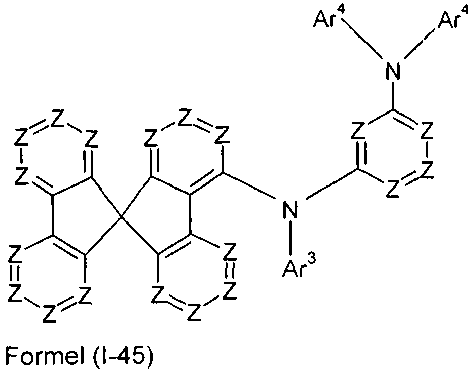 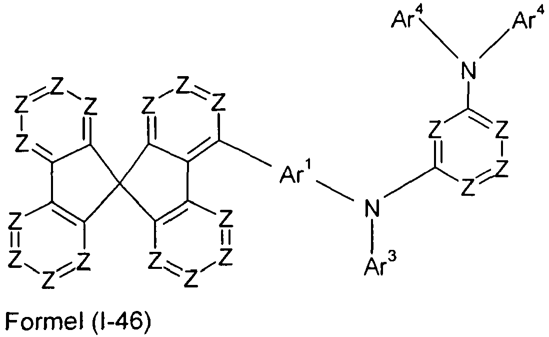 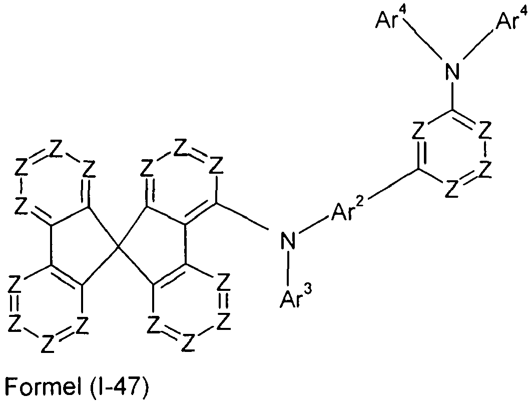 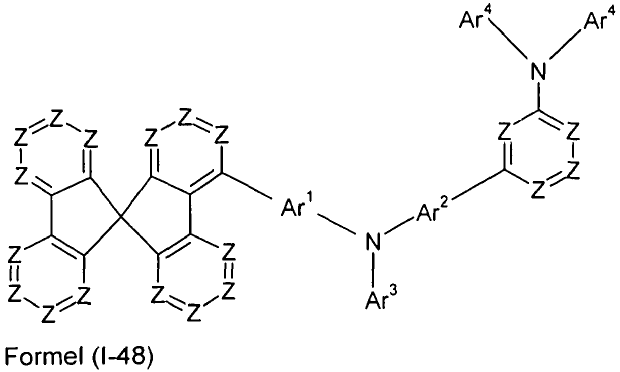 wobei die auftretenden Symbole wie oben definiert sind. Weiterhin gelten die oben angegebenen bevorzugten Ausführungsformen der Gruppen Ar¹, Ar², Ar³, Ar⁴, Z, R¹ und R² auch hier als bevorzugt.

Bevorzugte Ausführungsformen von Formel (II) entsprechen den folgenden Formeln (II-1) bis (II-24) 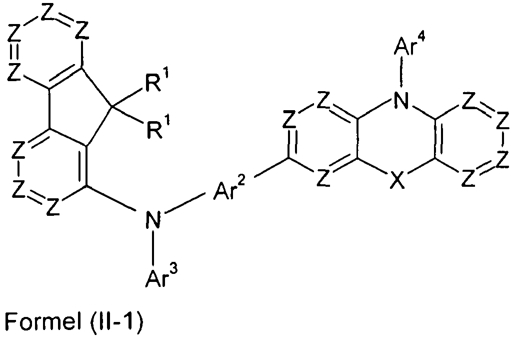 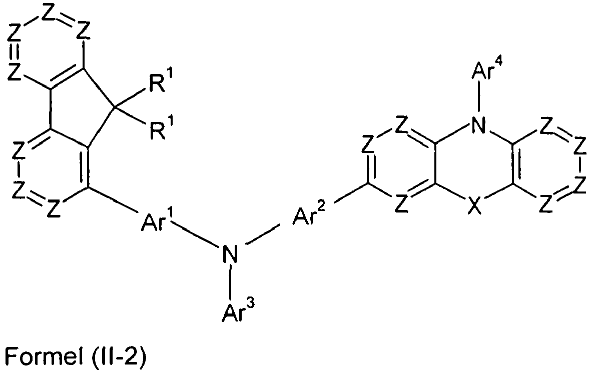 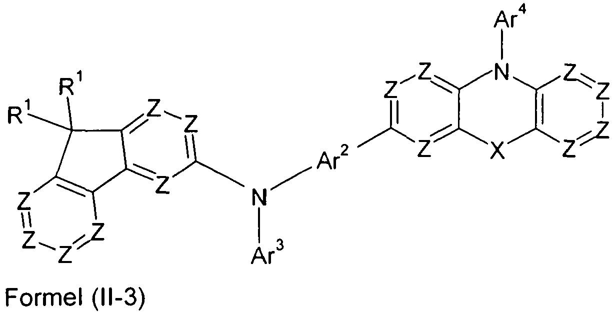 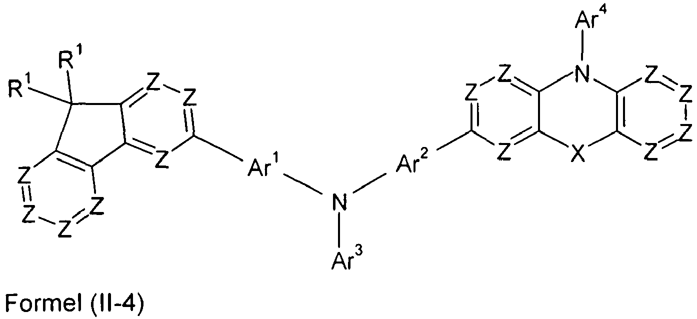 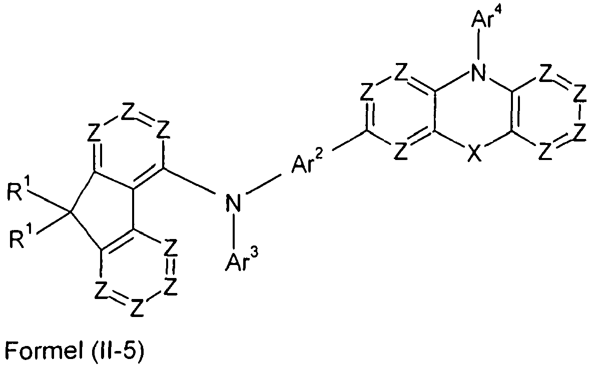 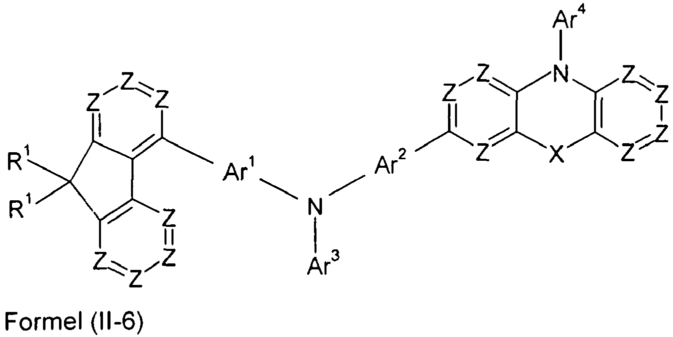 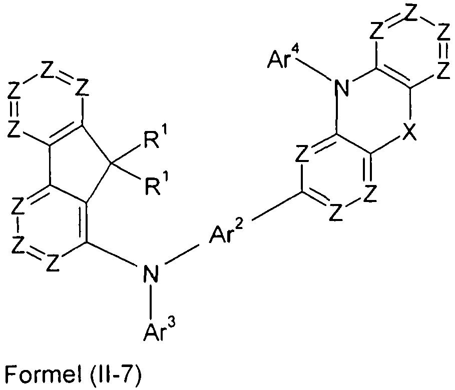 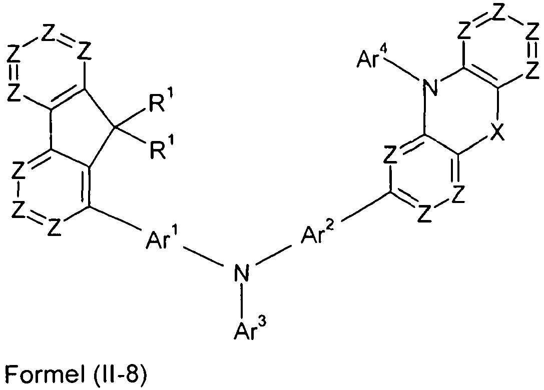 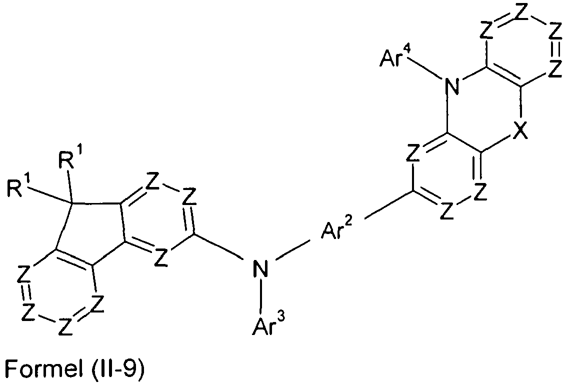 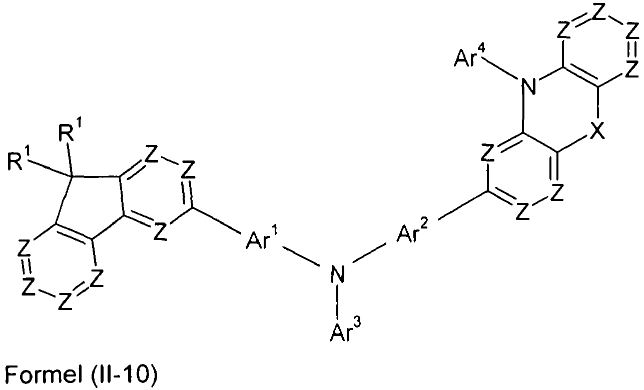 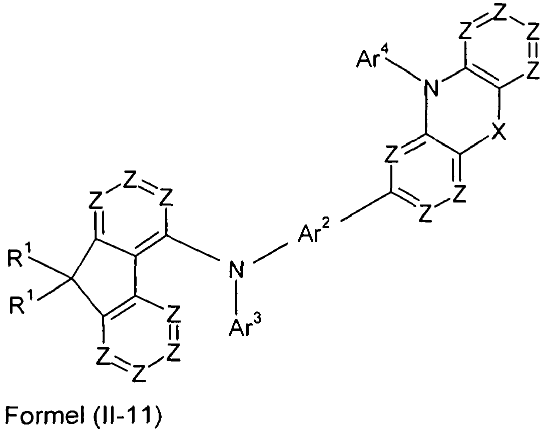 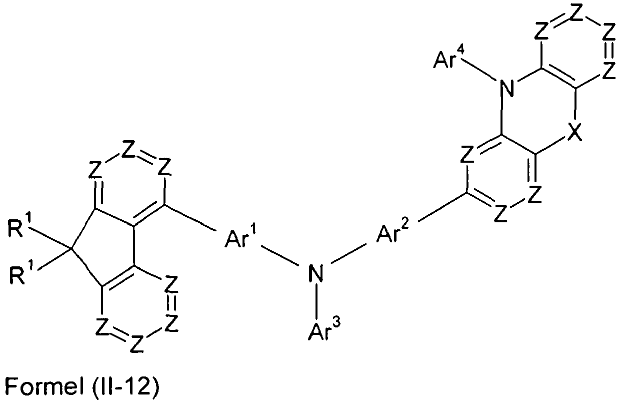 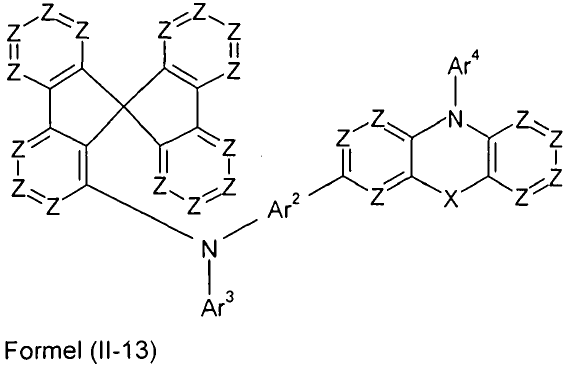 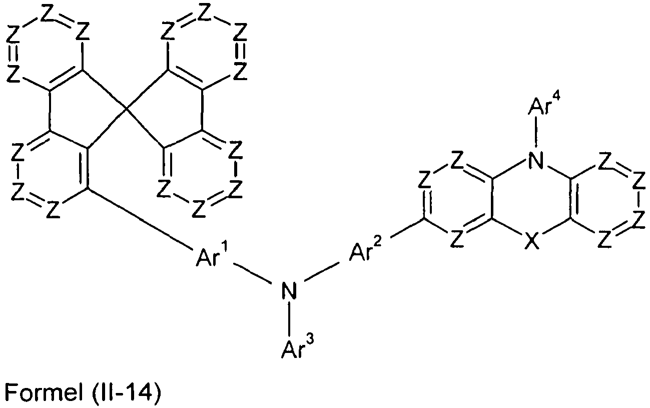 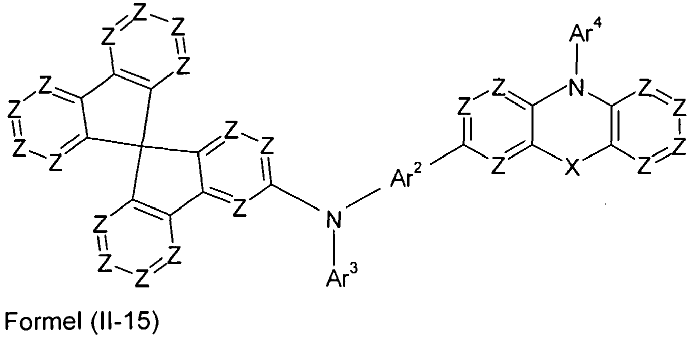 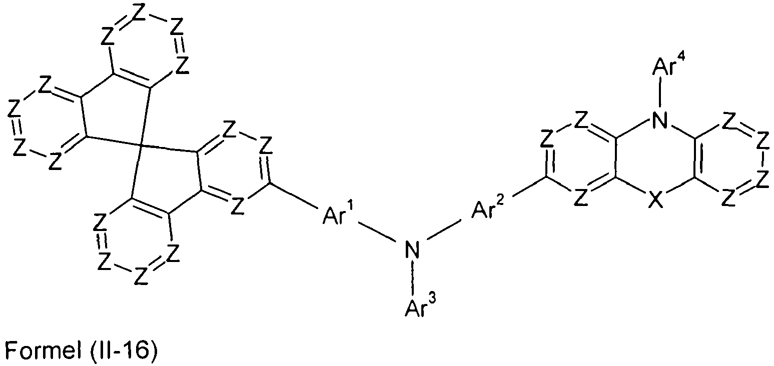 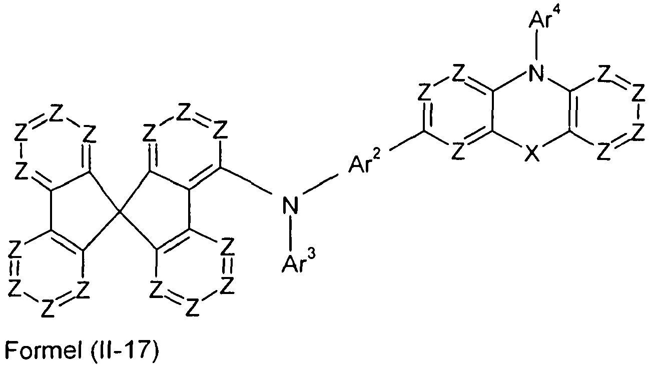 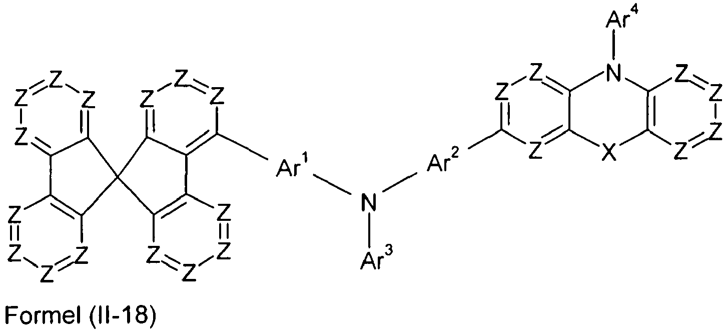 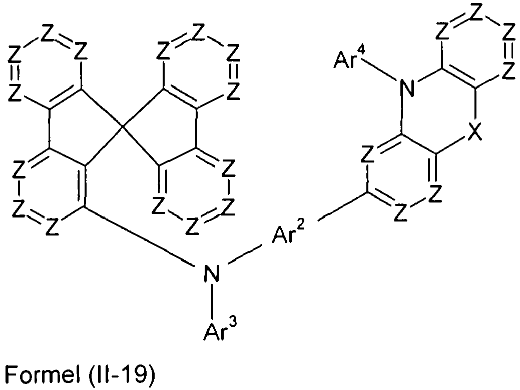 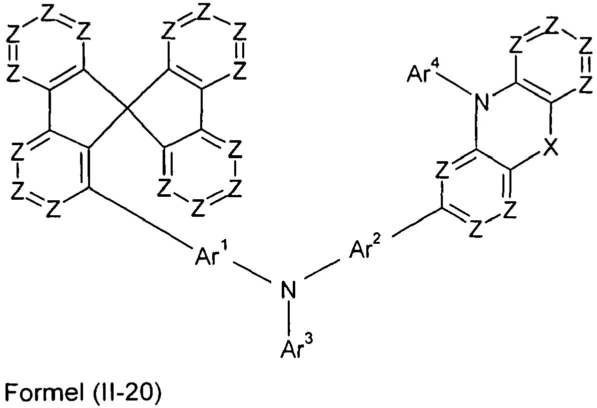 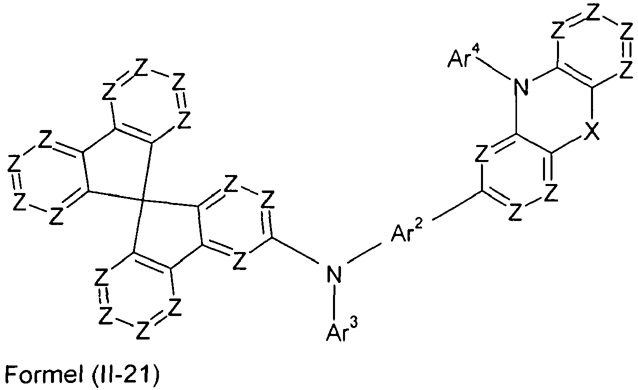 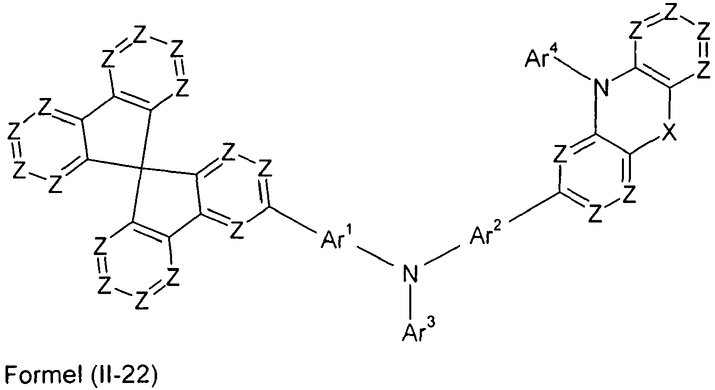 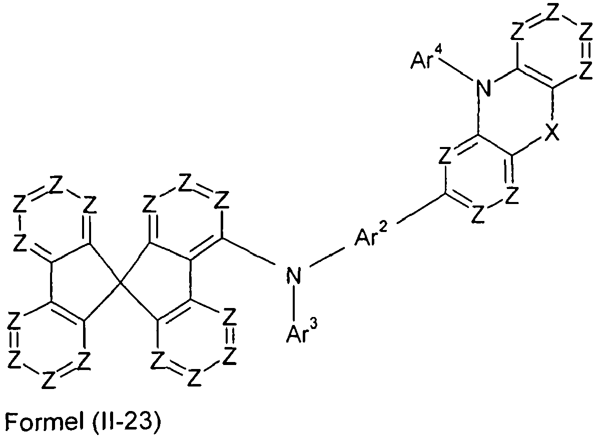 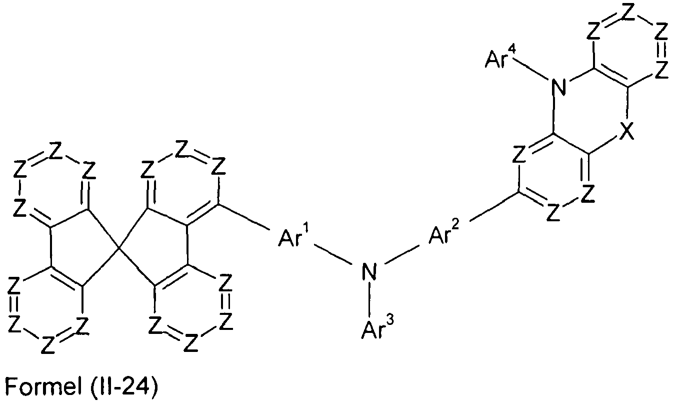 wobei die auftretenden Symbole wie oben definiert sind. Weiterhin gelten die oben angegebenen bevorzugten Ausführungsformen der Gruppen Ar¹, Ar², Ar³, Ar⁴, X, Z, R¹ und R² auch hier als bevorzugt.

Bevorzugte Ausführungsformen von Verbindungen gemäß Formel (III) entsprechen den folgenden Formeln (III-1) bis (III-48) 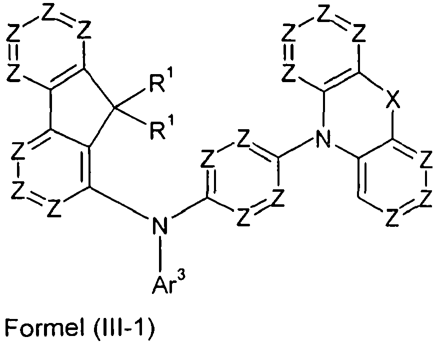 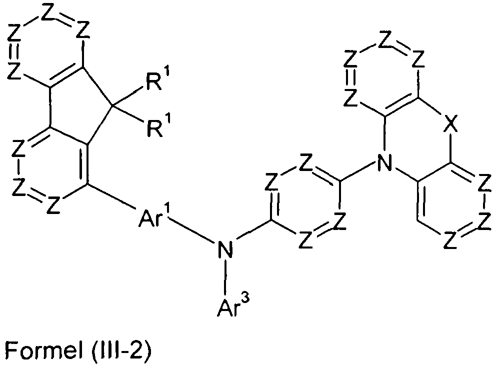 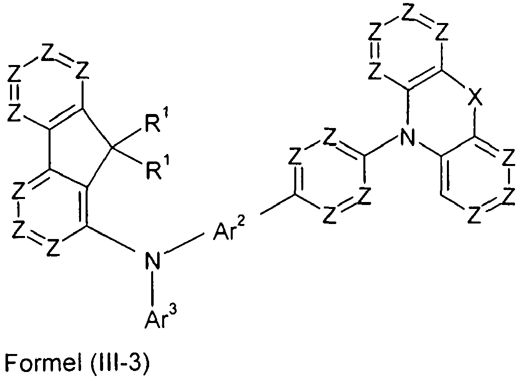 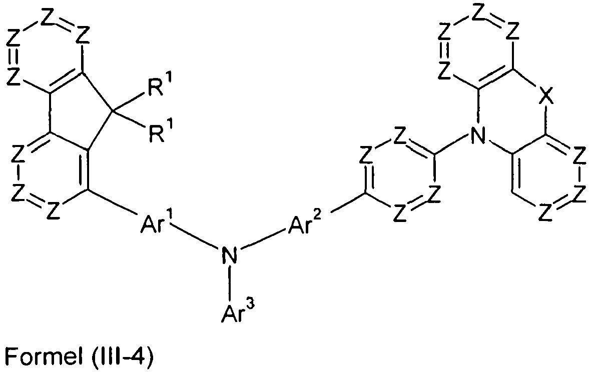 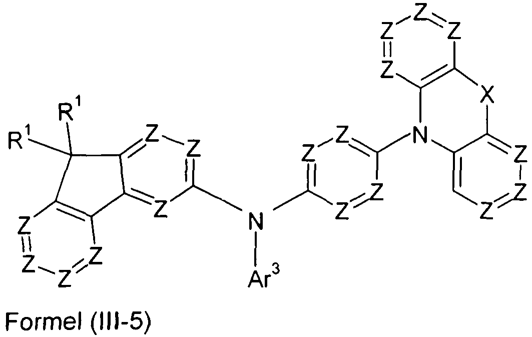 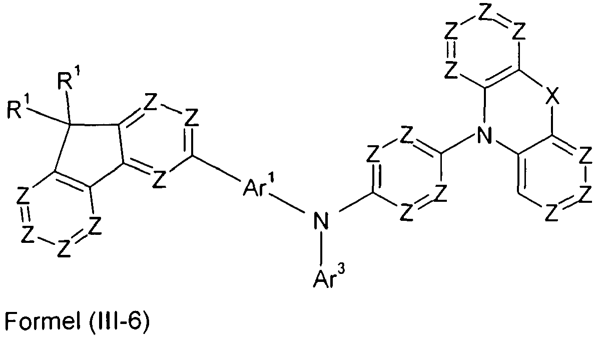 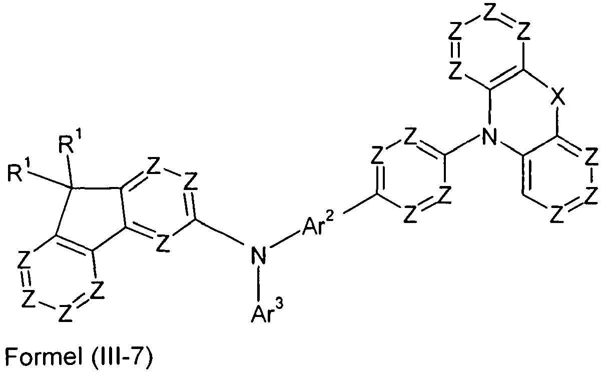 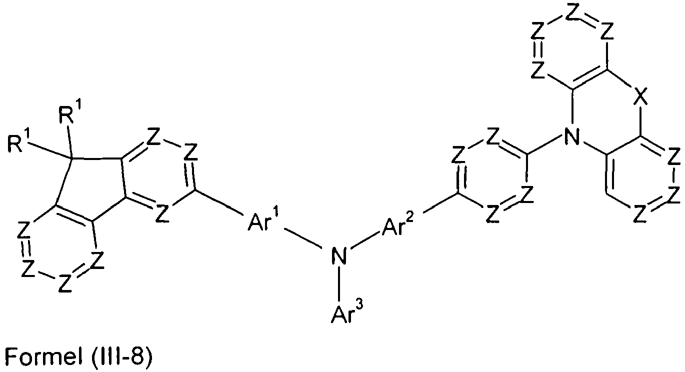 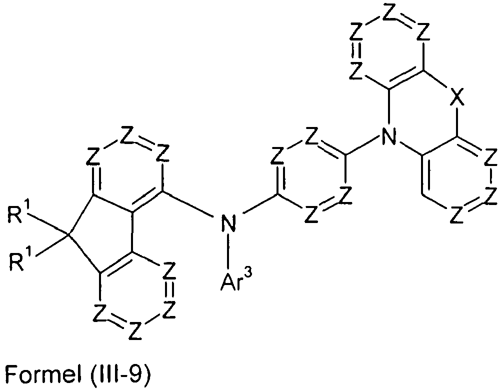 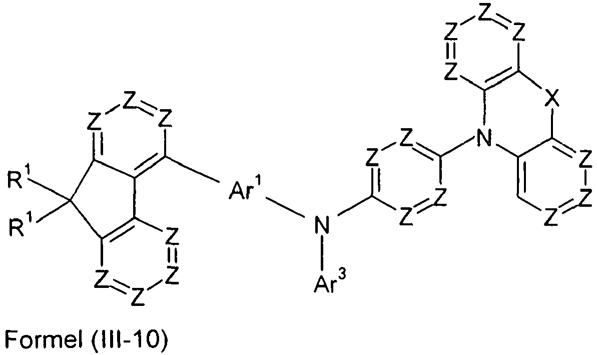 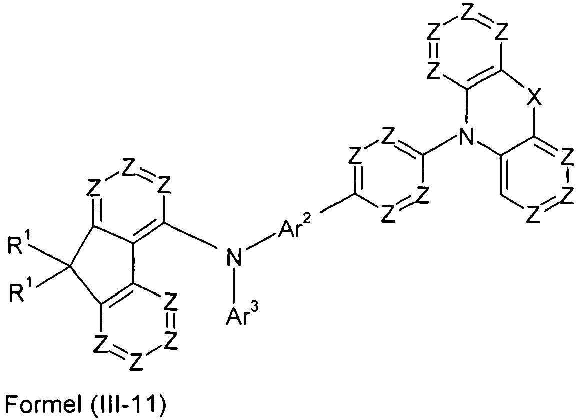 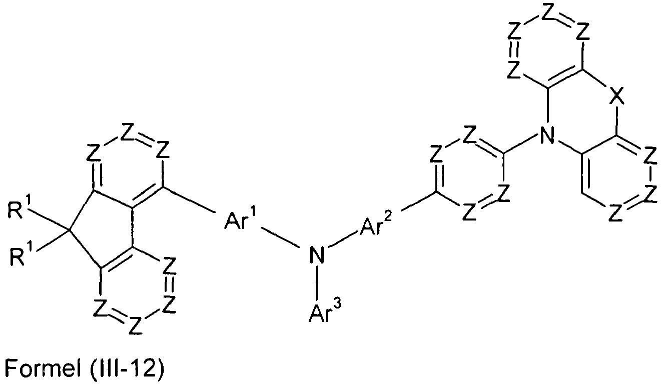 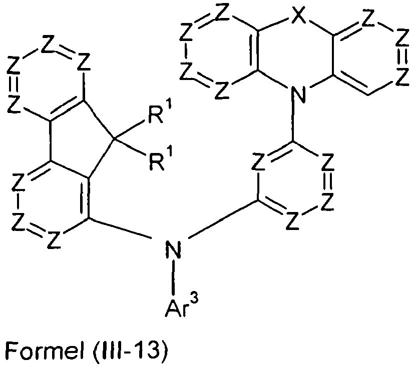 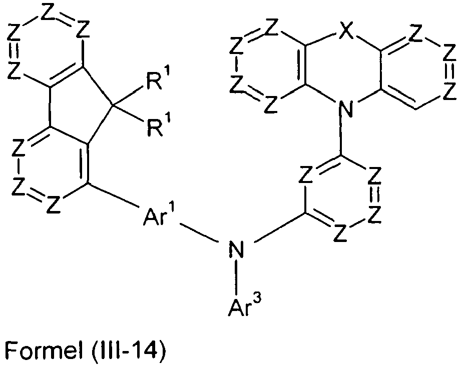 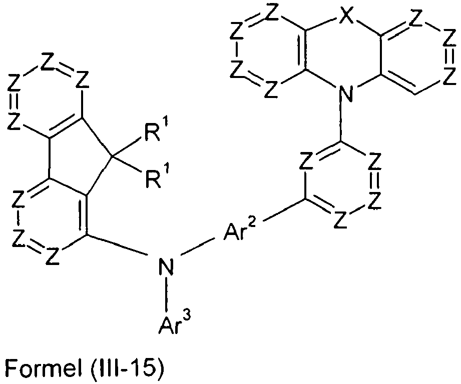 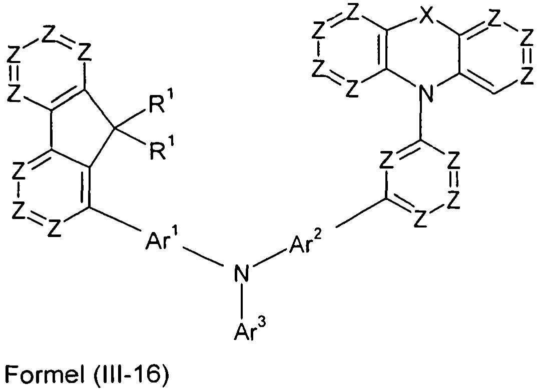 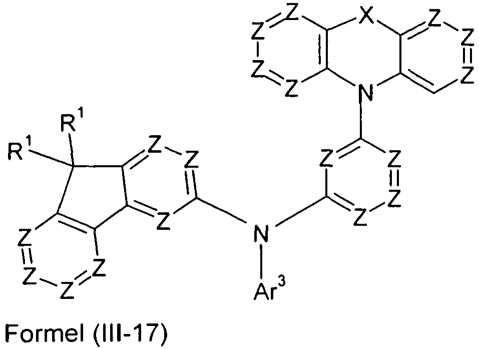 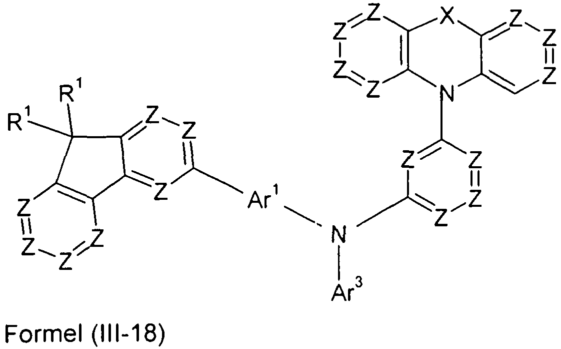 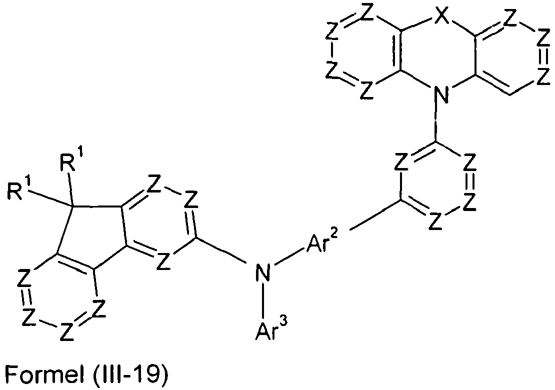 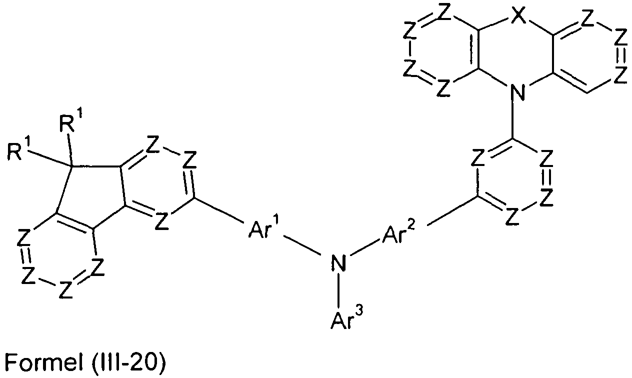 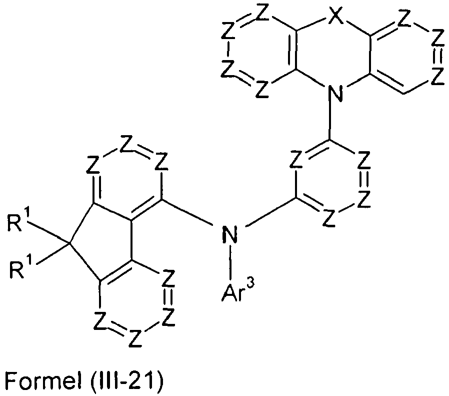 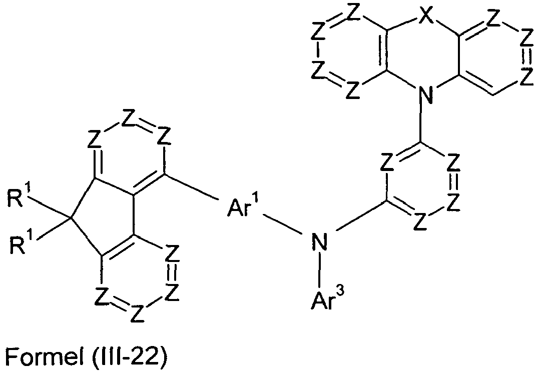 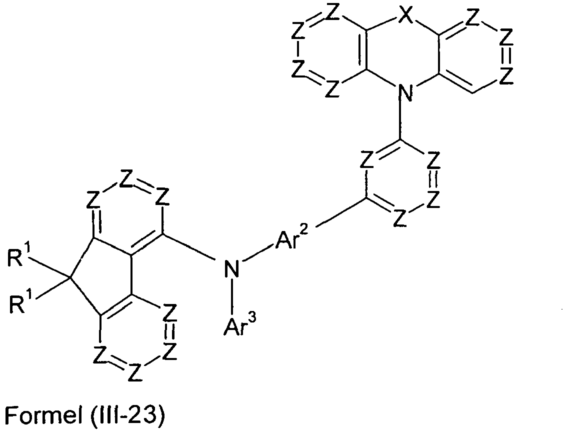 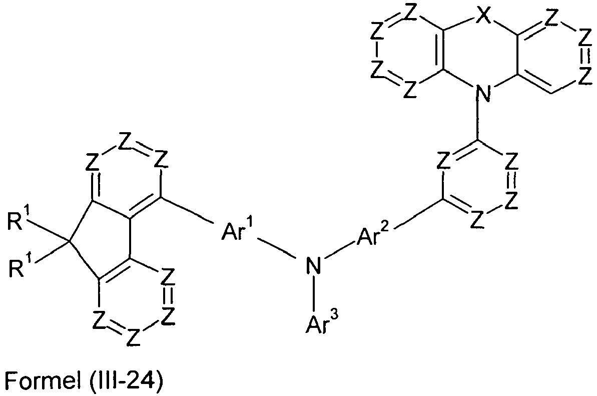 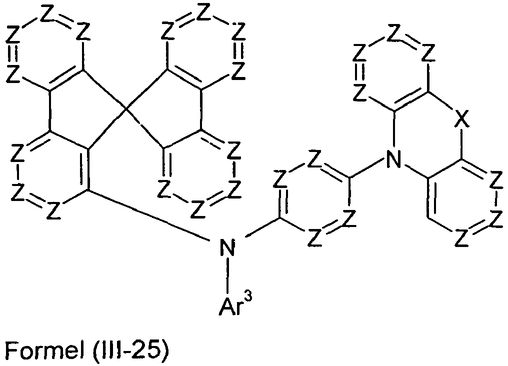 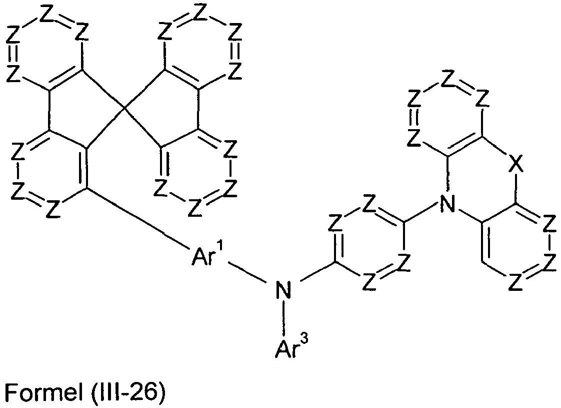 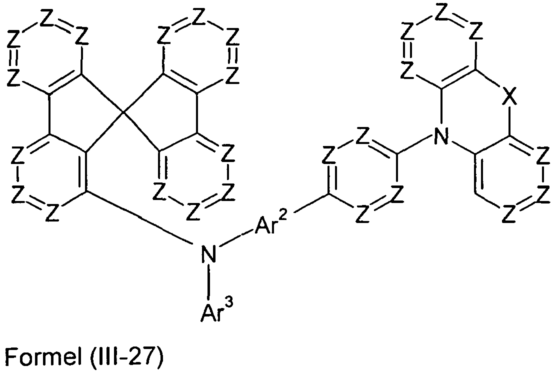 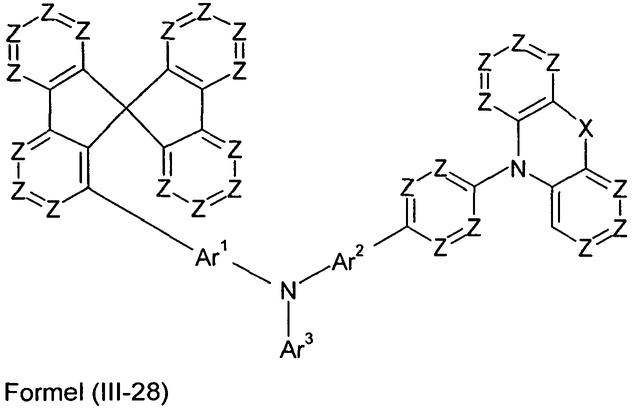 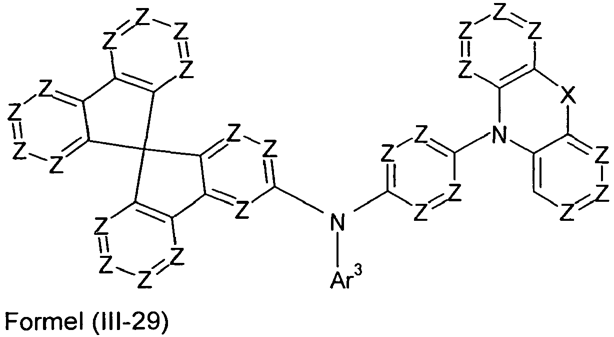 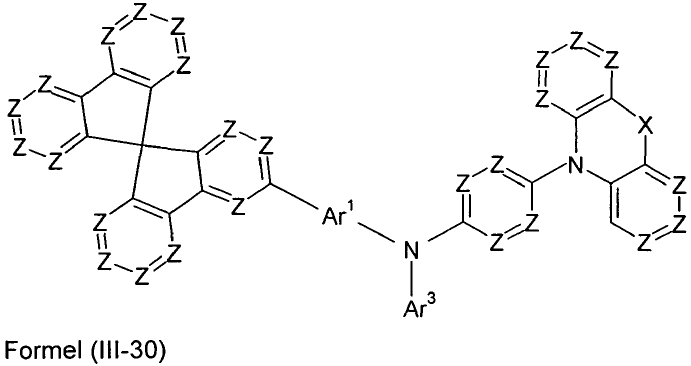 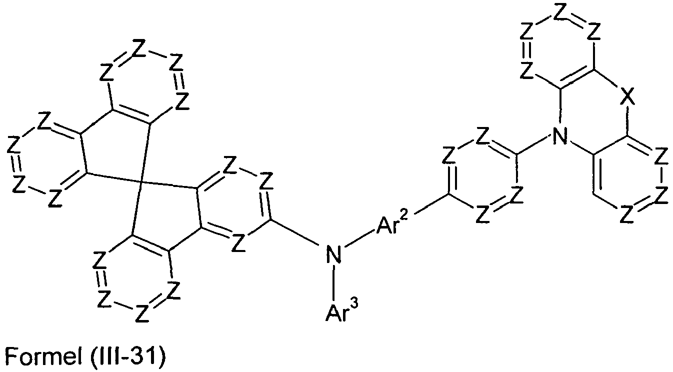 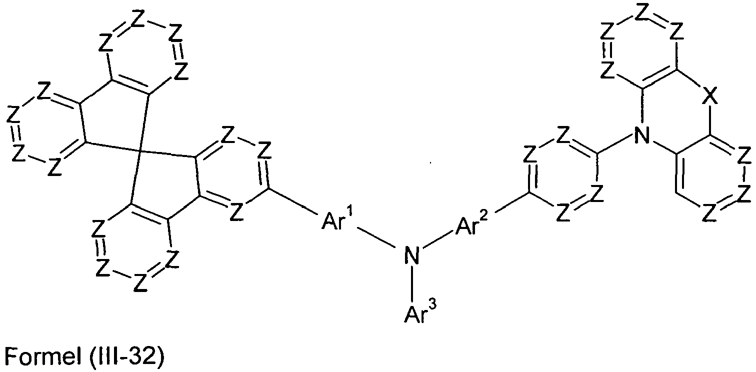 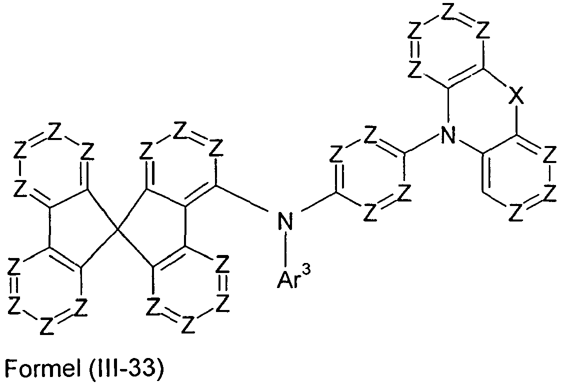 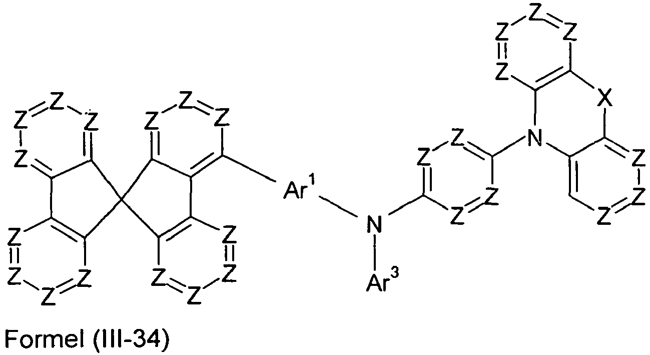 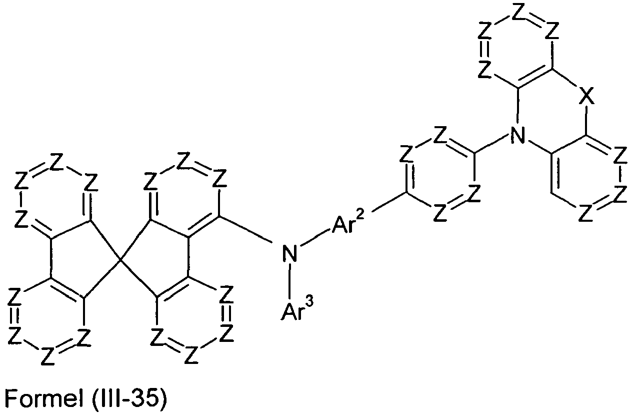 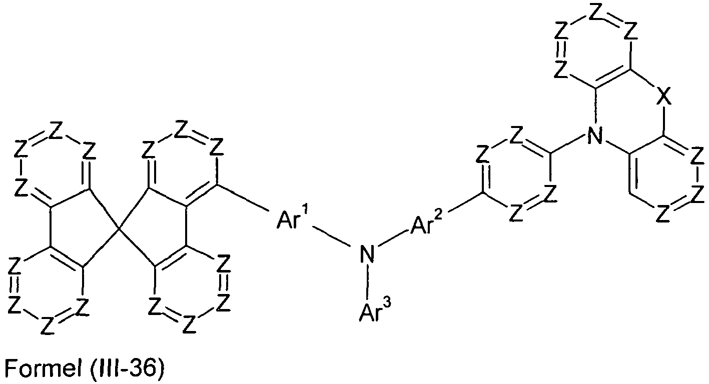 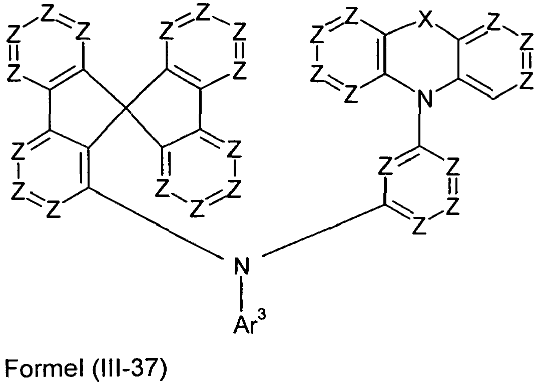 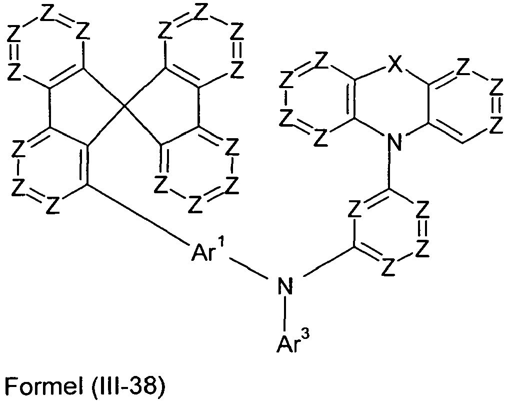 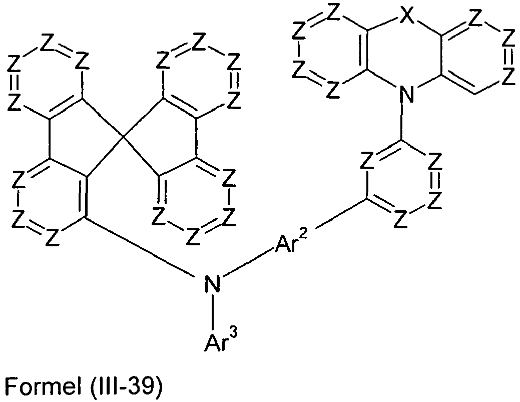 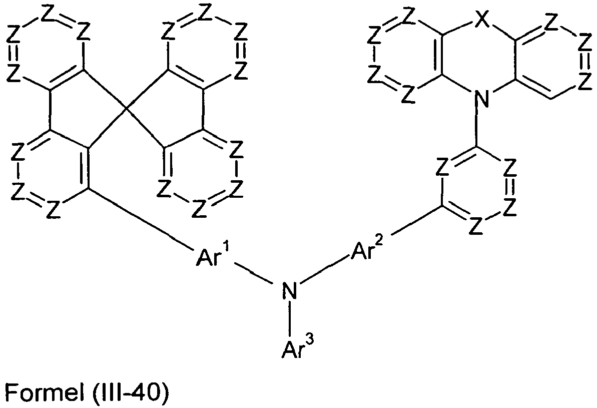 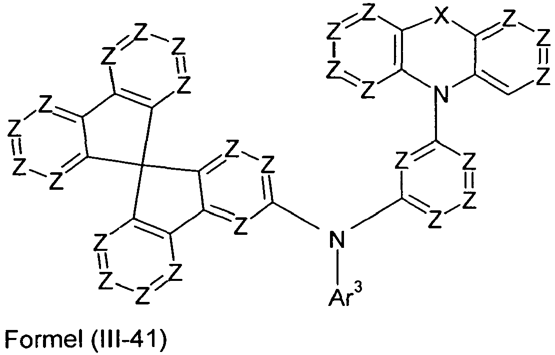 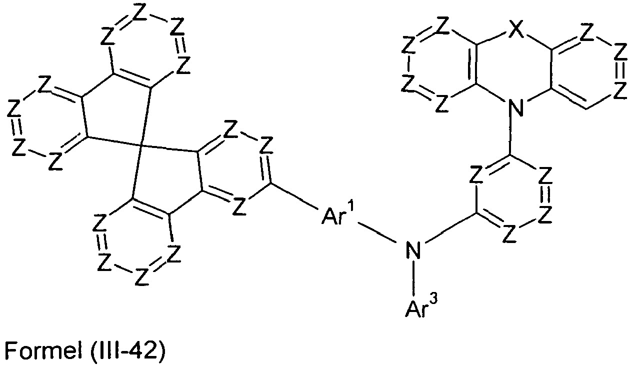 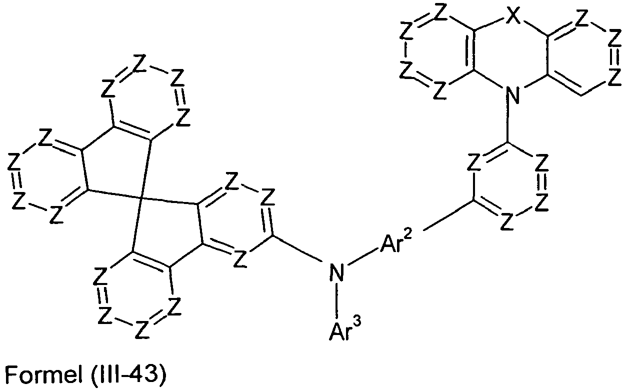 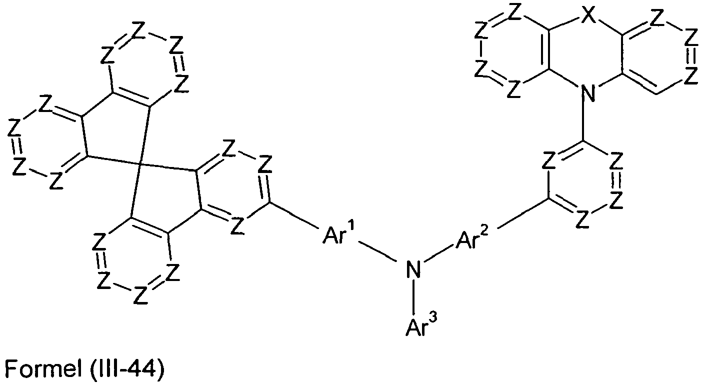 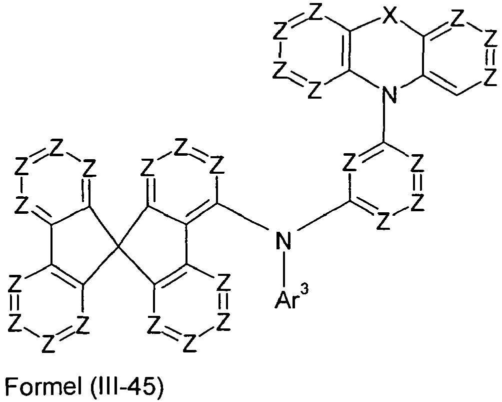 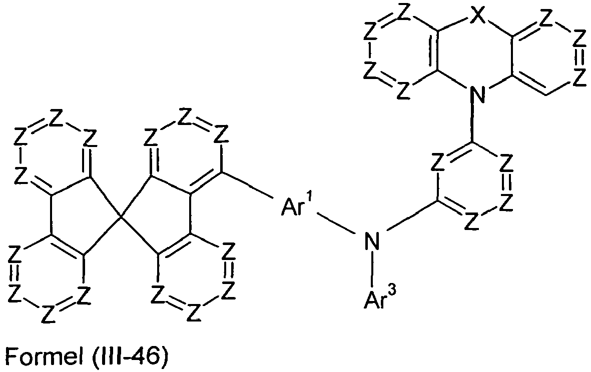 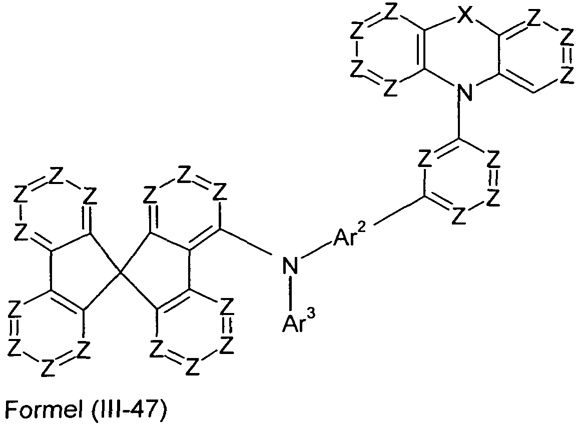 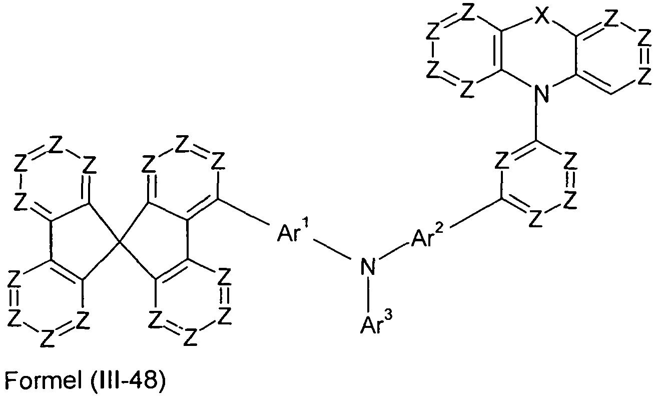 wobei die auftretenden Symbole wie oben definiert sind. Weiterhin gelten die oben angegebenen bevorzugten Ausführungsformen der Gruppen Ar¹, Ar², Ar³, X, Z, R¹ und R² auch hier als bevorzugt.

Explizite Beispiele für erfindungsgemäße Verbindungen sind in der folgenden Tabelle aufgeführt, wobei die mit * markierten Verbindungen nicht anspruchsgemäß sind.

| | | |
|---|---|---|
| 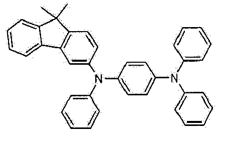 | 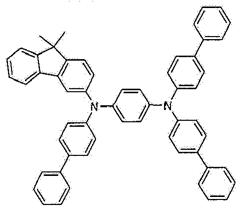 | 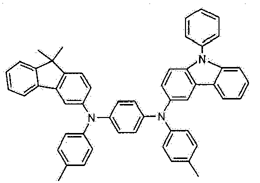 |
| 1* | 2* | 3* |
| 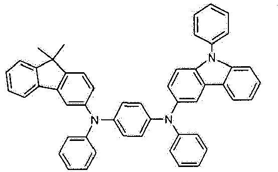 | 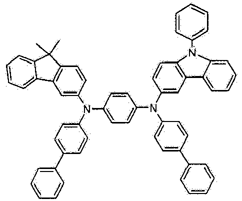 | 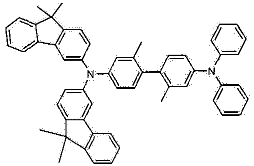 |
| 4* | 5* | 6* |
| 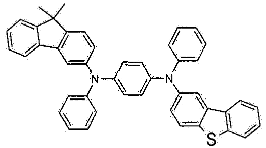 | 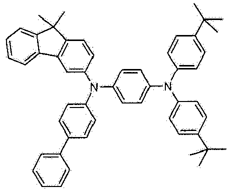 | 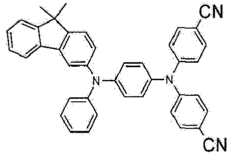 |
| 7* | 8* | 9* |
| 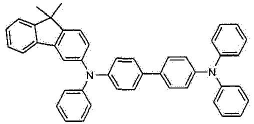 | 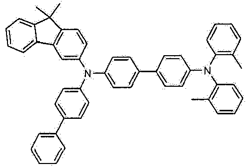 | 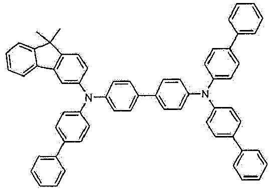 |
| 10* | 11* | 12* |
| 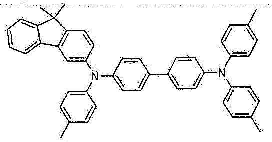 | 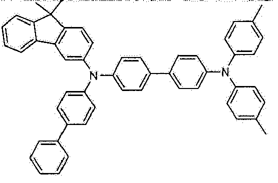 | 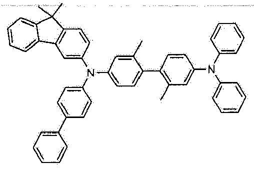 |
| 13* | 14* | 15* |
| 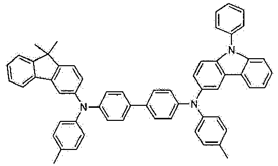 | 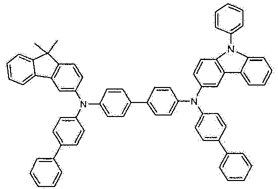 | 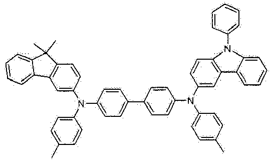 |
| 16* | 17* | 18* |
| 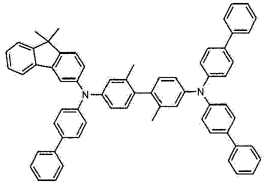 | 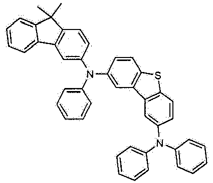 | 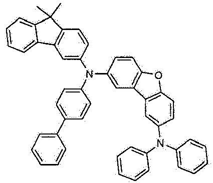 |
| 19* | 20* | 21* |
| 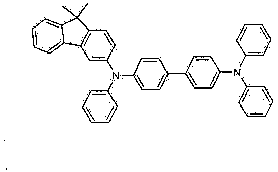 | 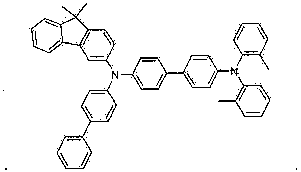 | 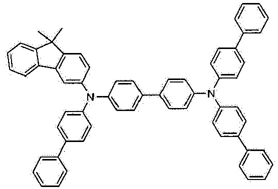 |
| 22* | 23* | 24* |
| 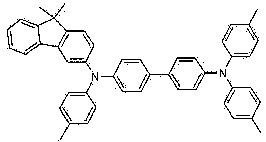 | 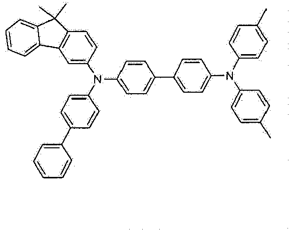 | 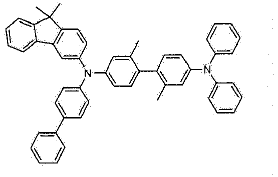 |
| 25* | 26* | 27* |
| 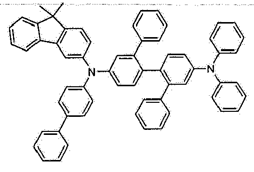 | 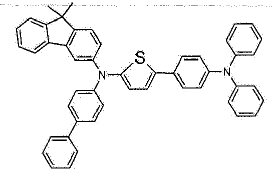 | 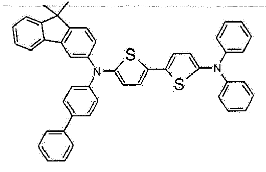 |
| 28* | 29* | 30* |
| 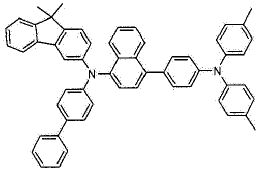 | 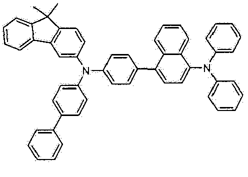 | 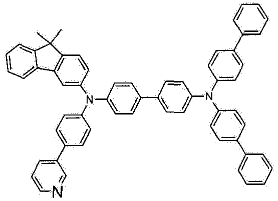 |
| 31* | 32* | 33* |
| 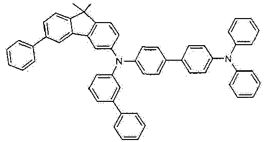 | 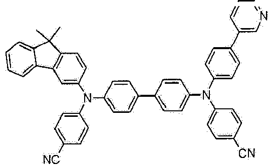 | 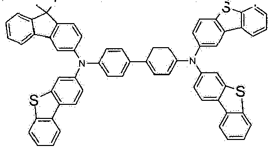 |
| 34* | 35* | 36* |
| 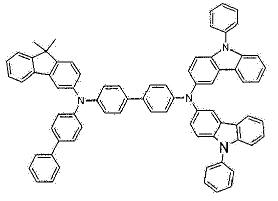 | 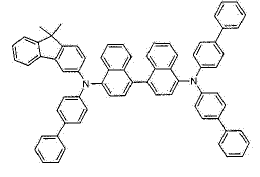 | 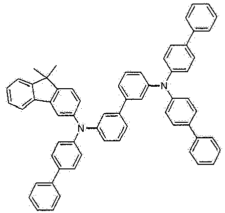 |
| 37* | 38* | 39* |
| 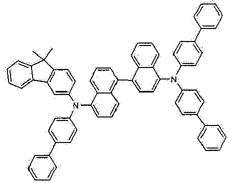 | 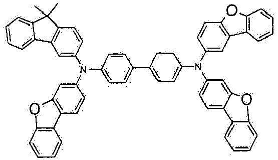 | 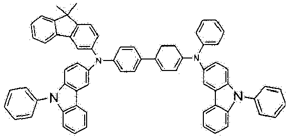 |
| 40* | 41* | 42* |
| 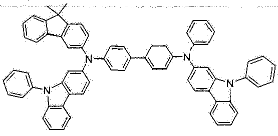 | 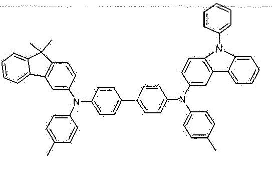 | 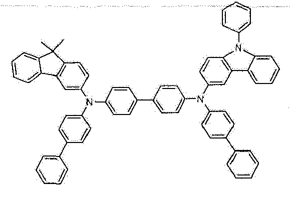 |
| 43* | 44* | 45* |
| 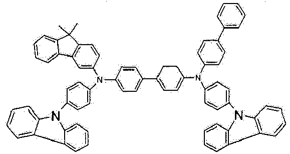 | 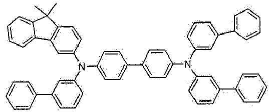 | 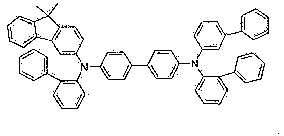 |
| 46* | 47* | 48* |
| 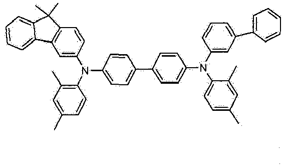 | 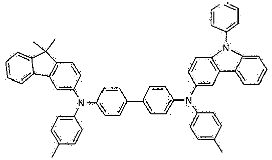 | 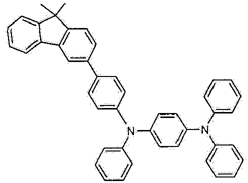 |
| 49* | 50* | 51* |
| 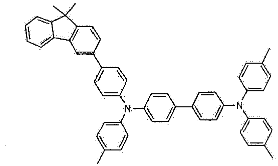 | 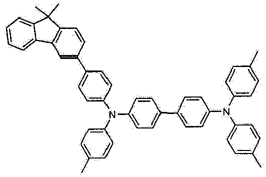 | 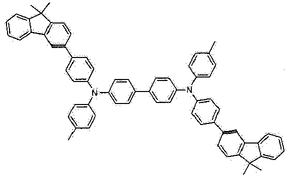 |
| 52* | 53* | 54* |
| 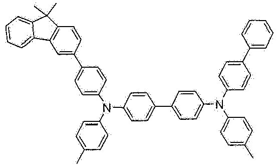 | 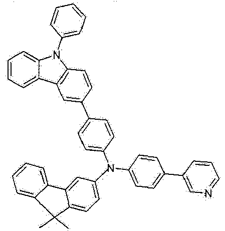 | 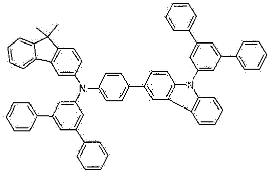 |
| 55* | 56 | 57 |
| 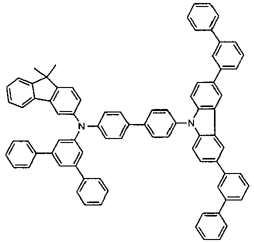 | 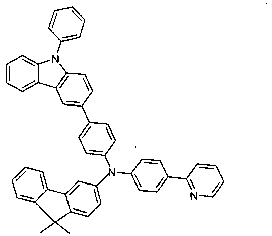 | 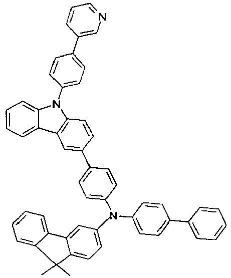 |
| 58 | 59 | 60* |
| 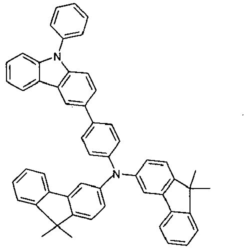 | 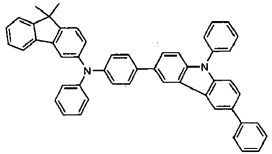 | 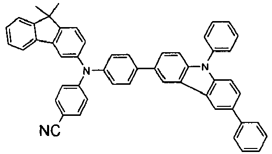 |
| 61 | 62 | 63* |
| 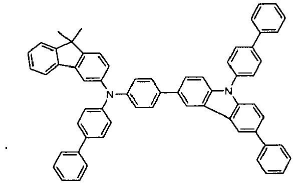 | 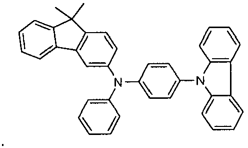 | 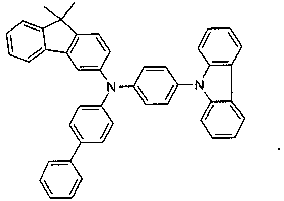 |
| 64 | 65 | 66 |
| 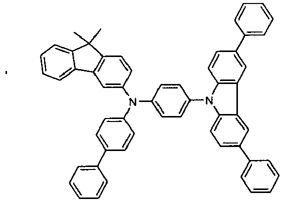 | 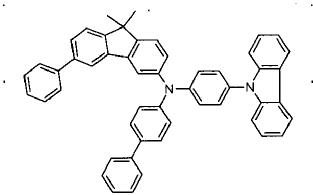 | 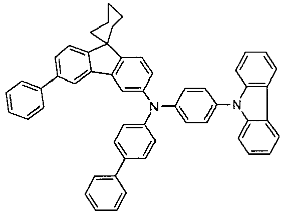 |
| 67 | 68 | 69 |
| 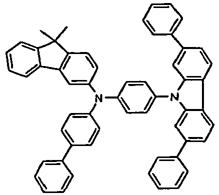 | 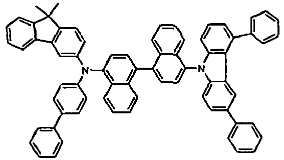 | 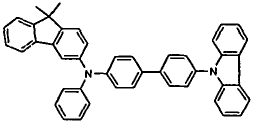 |
| 70 | 71 | 72 |
| 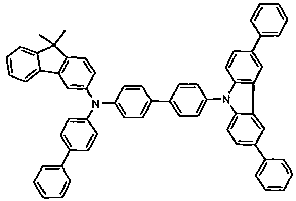 | 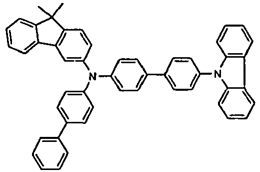 | 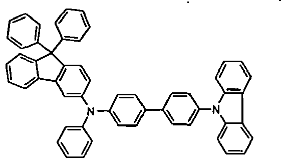 |
| 73 | 74 | 75 |
| 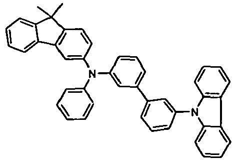 | 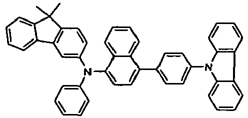 | 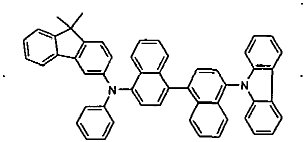 |
| 76 | 77 | 78 |
| 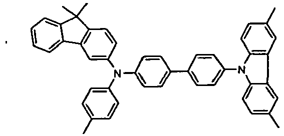 | 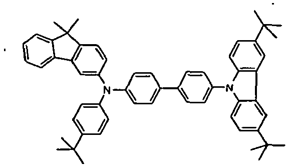 | 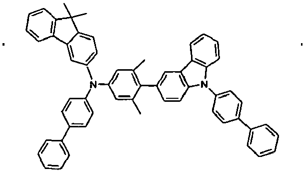 |
| 79 | 80 | 81 |
| 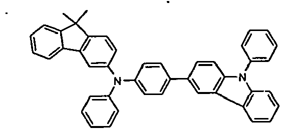 | 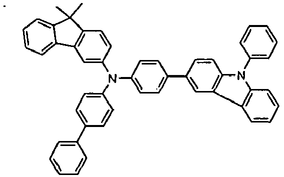 | 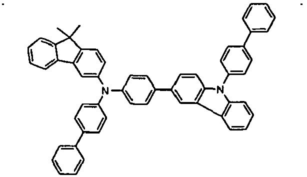 |
| 82 | 83 | 84 |
| 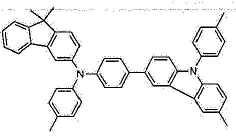 | 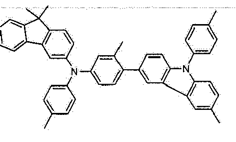 | 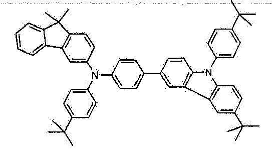 |
| 85 | 86 | 87 |
| 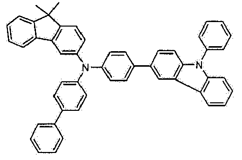 | 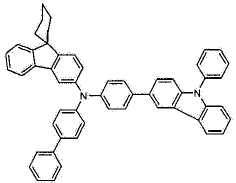 | 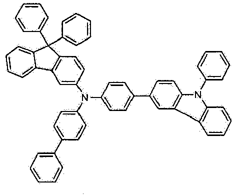 |
| 88 | 89 | 90 |
| 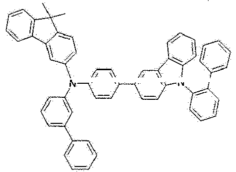 | 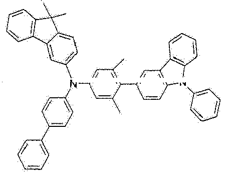 | 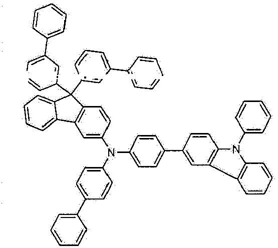 |
| 91 | 92 | 93 |
| 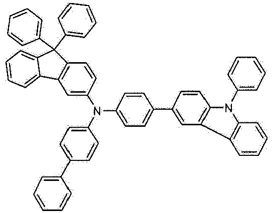 | 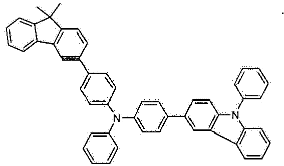 | 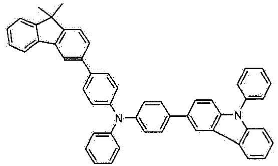 |
| 94 | 95 | 96 |
| 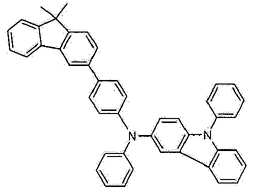 | 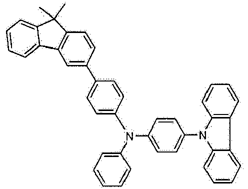 | 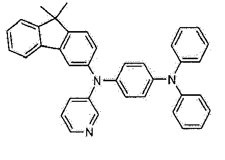 |
| 97 | 98 | 99* |
| 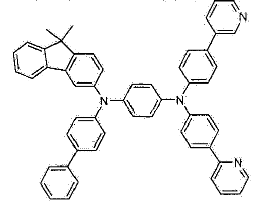 | 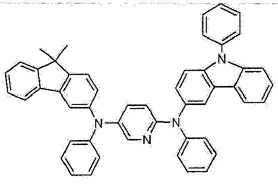 | 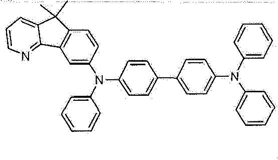 |
| 100* | 101* | 102* |
| 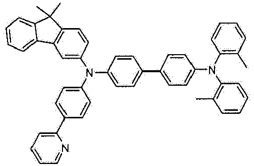 | 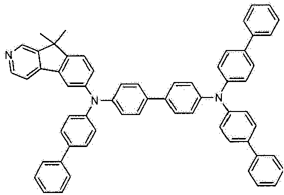 | 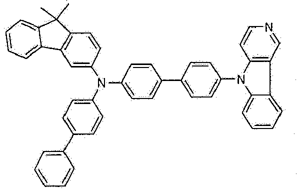 |
| 103* | 104* | 105* |
| 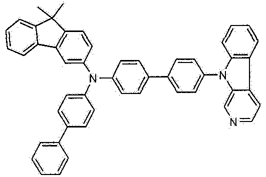 | 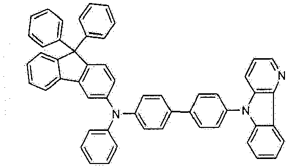 | 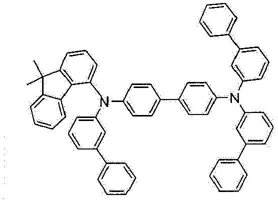 |
| 106* | 107* | 108* |
| 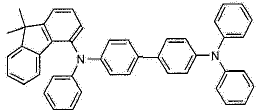 | 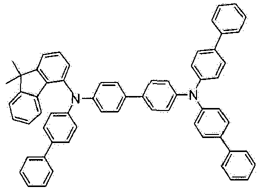 | 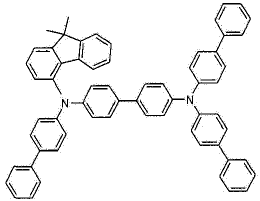 |
| 109* | 110* | 111* |
| 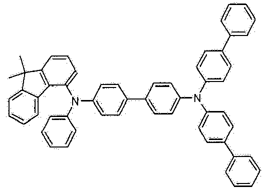 | 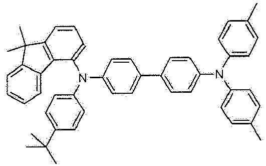 | 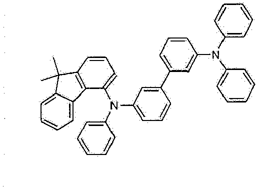 |
| 112* | 113* | 114* |
| 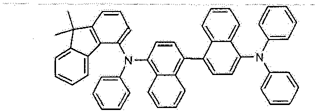 | 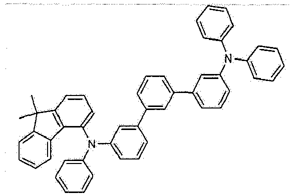 | 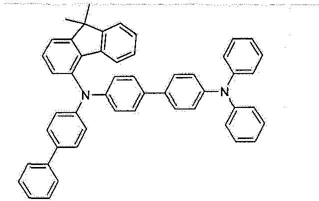 |
| 115* | 116* | 117* |
| 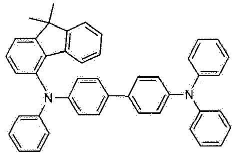 | 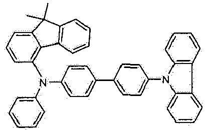 | 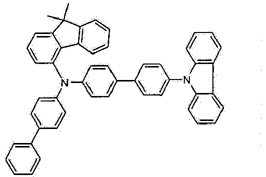 |
| 118* | 119 | 120 |
| 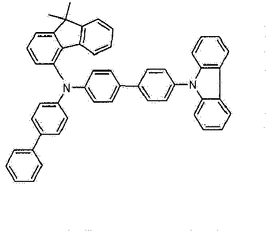 | 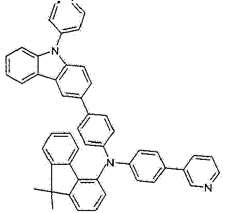 | 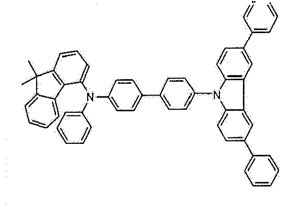 |
| 121 | 122 | 123 |
| 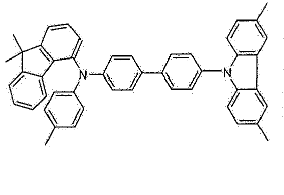 | 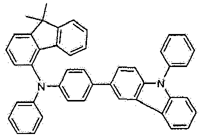 | 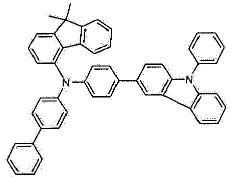 |
| 124 | 125 | 126 |
| 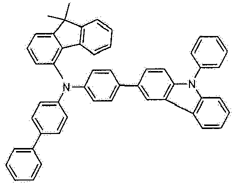 | 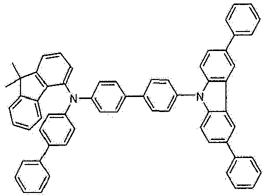 | 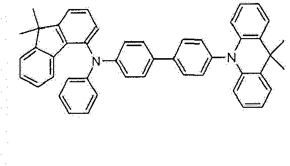 |
| 127 | 128 | 129 |
| 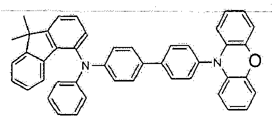 | 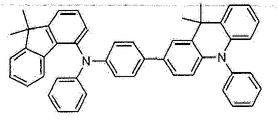 | 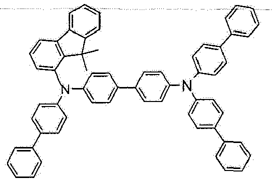 |
| 130 | 131 | 132* |
| 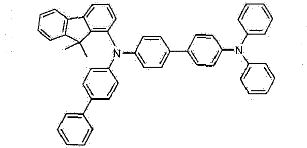 | 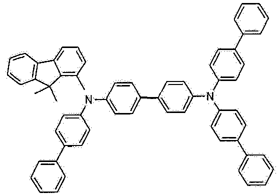 | 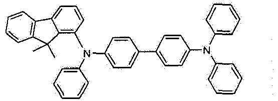 |
| 133* | 134* | 135* |
| 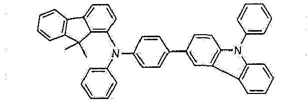 | 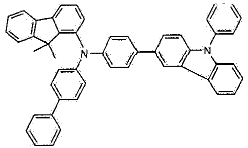 | 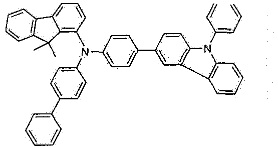 |
| 136 | 137 | 138 |
| 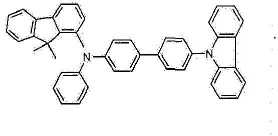 | 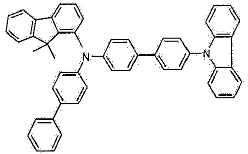 | 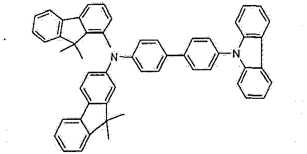 |
| 139 | 140 | 141 |
| 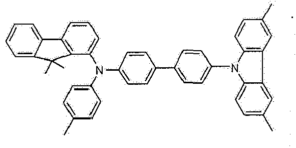 | 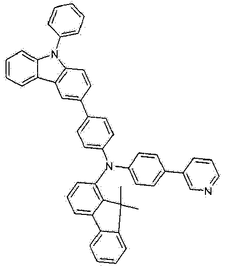 | 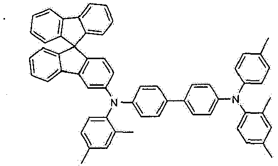 |
| 142 | 143 | 144 |
| 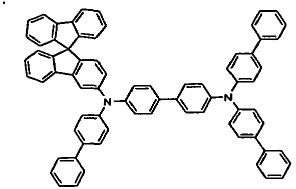 | 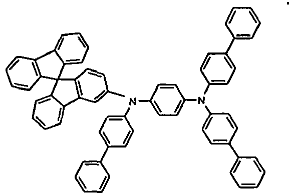 | 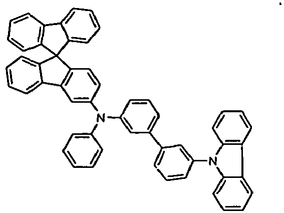 |
| 145 | 146 | 147 |
| 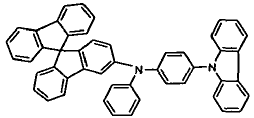 | 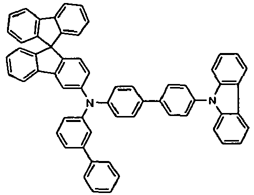 | 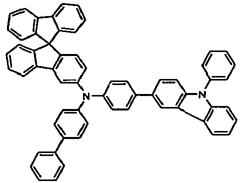 |
| 148 | 149 | 150 |
| 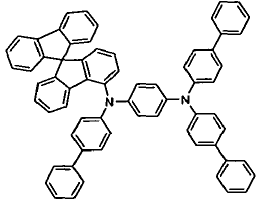 | 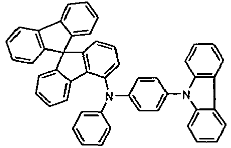 | 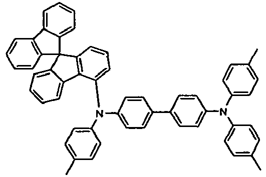 |
| 151 | 152 | 153 |
| 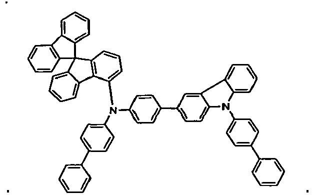 | 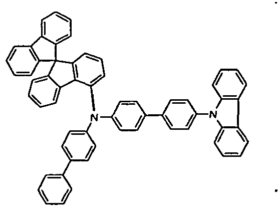 | 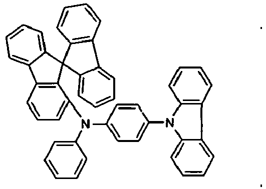 |
| 154 | 155 | 156 |
| 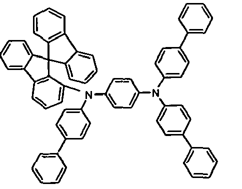 | 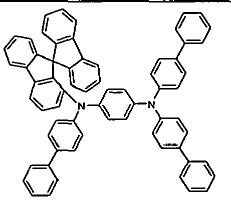 | 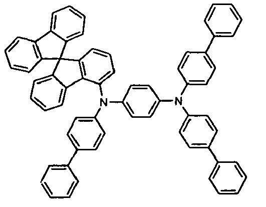 |
| 157 | 158 | 159 |
| 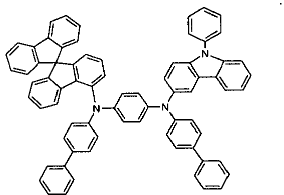 | 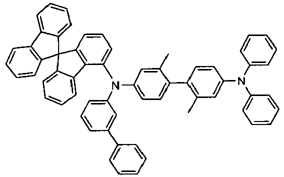 | 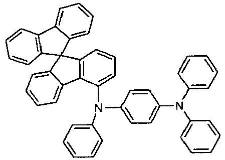 |
| 160* | 161 | 162 |
| 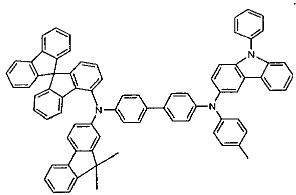 | 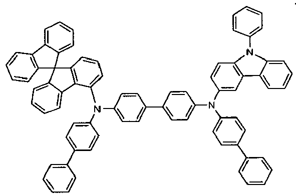 | 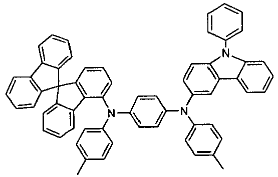 |
| 163* | 164* | 165* |
| 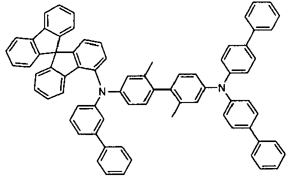 | 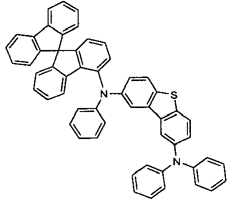 | 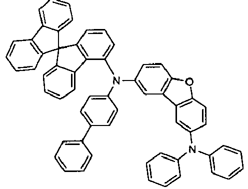 |
| 166 | 167 | 168 |
| 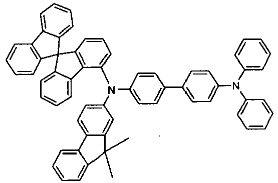 | 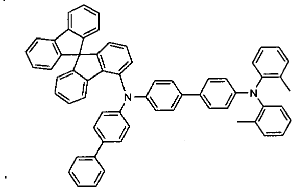 | 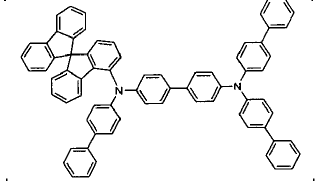 |
| 169 | 170 | 171 |
| 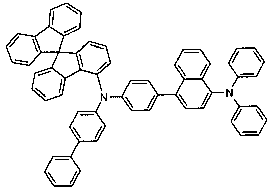 | 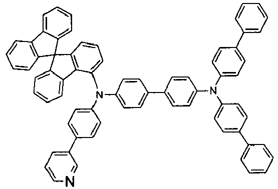 | 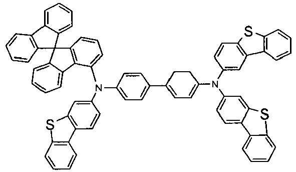 |
| 172 | 173 | 174* |
| 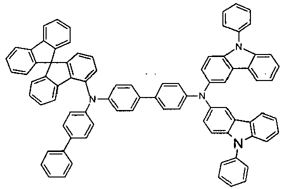 | 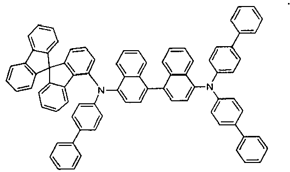 | 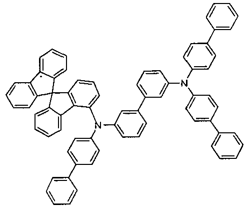 |
| 175* | 176 | 177 |
| 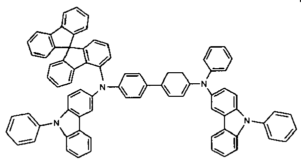 | 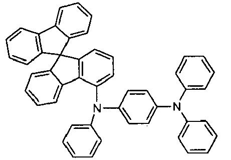 | 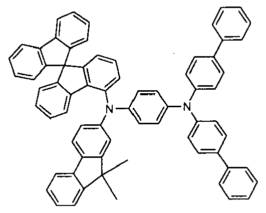 |
| 178* | 179 | 180 |
| 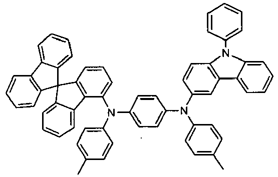 | 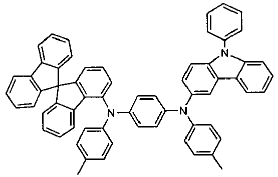 | 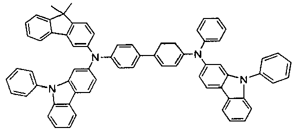 |
| 181* | 182* | 183* |
| 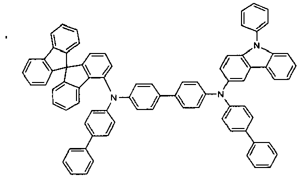 | 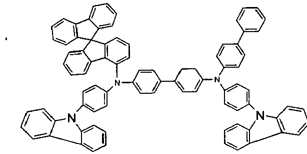 | 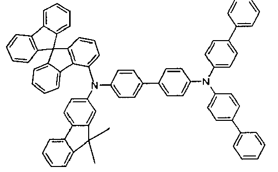 |
| 184* | 185* | 186 |
| 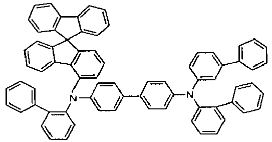 | 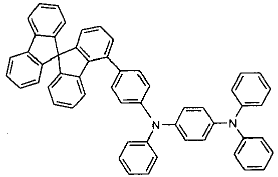 | 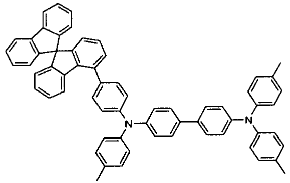 |
| 187 | 188 | 189 |
| 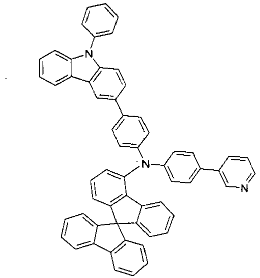 | 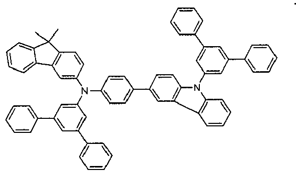 | 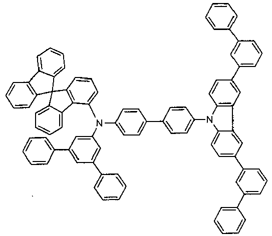 |
| 190 | 191 | 192 |
| 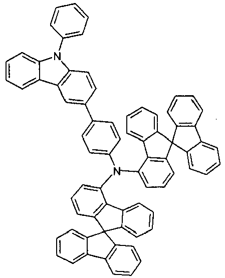 | 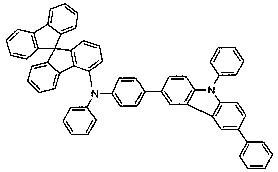 | 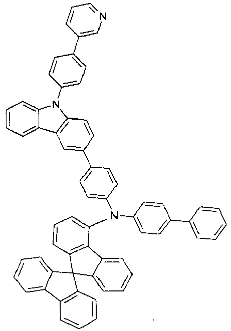 |
| 193 | 194 | 195* |
| 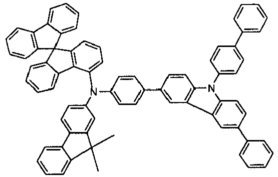 | 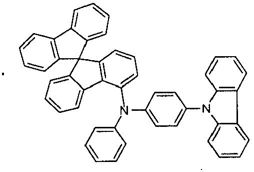 | 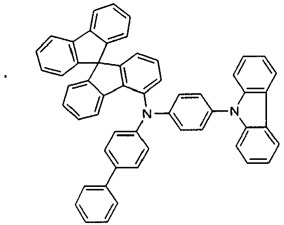 |
| 196 | 197 | 198 |
| 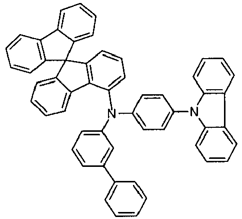 | 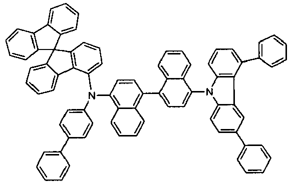 | 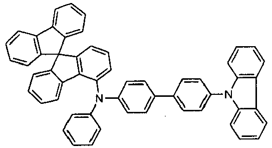 |
| 199 | 200 | 201 |
| 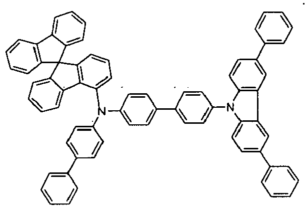 | 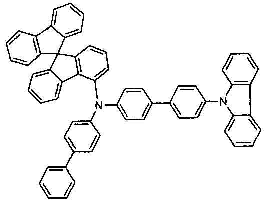 | 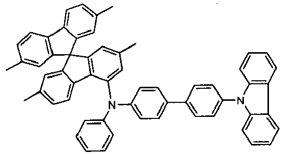 |
| 202 | 203 | 204 |
| 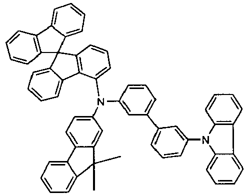 | 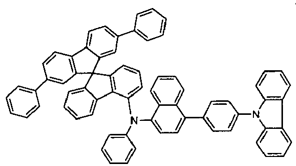 | 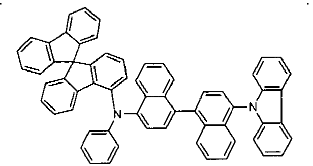 |
| 205 | 206 | 207 |
| 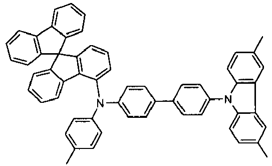 | 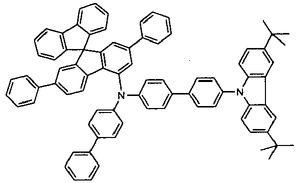 | 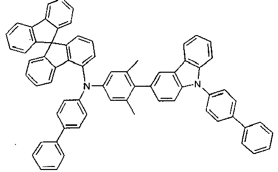 |
| 208 | 209 | 210 |
| 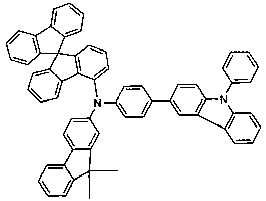 | 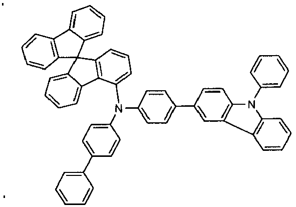 | 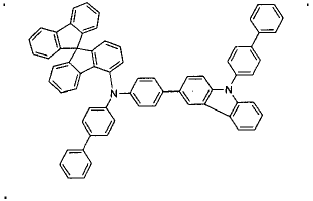 |
| 211 | 212 | 213 |
| 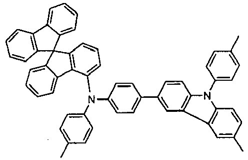 | 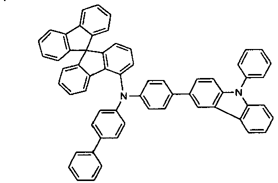 | 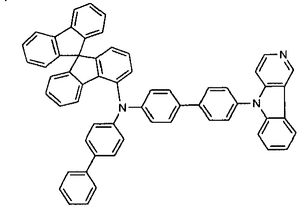 |
| 214 | 215 | 216* |
| 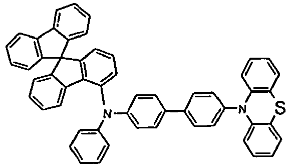 | 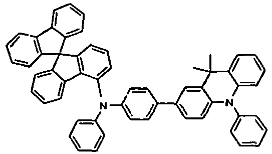 | 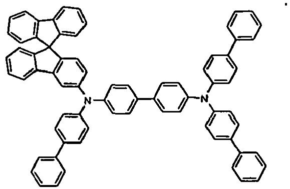 |
| 217 | 218 | 219 |
| 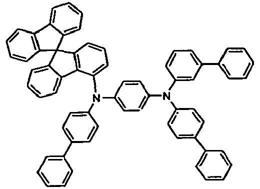 | 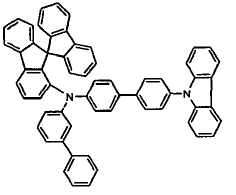 | 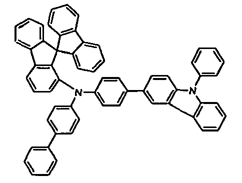 |
| 220 | 221 | 222 |
| 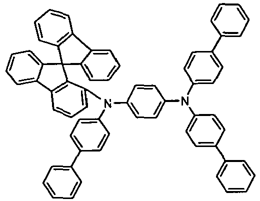 | 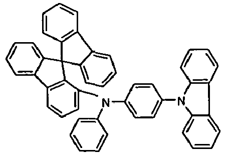 | 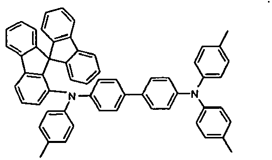 |
| 223 | 224 | 225 |
| 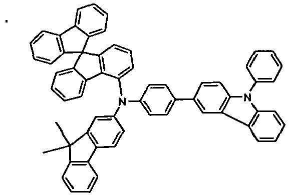 | 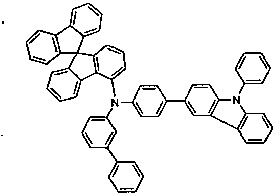 | 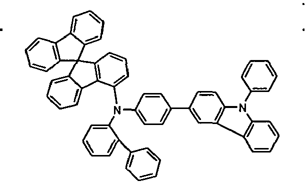 |
| 226 | 227 | 228 |
| 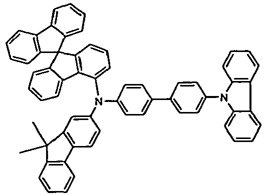 | 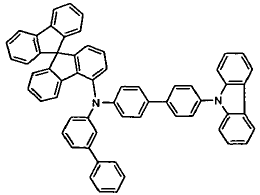 | 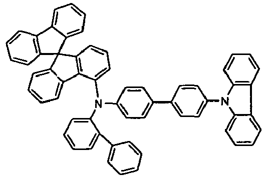 |
| 229 | 230 | 231 |
| 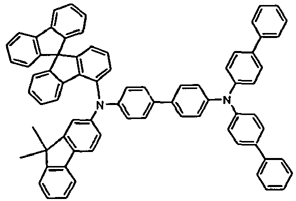 | 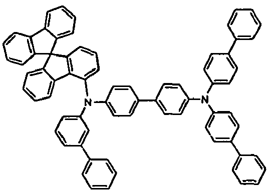 | 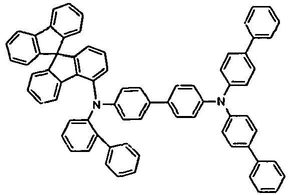 |
| 232 | 233 | 234 |
| 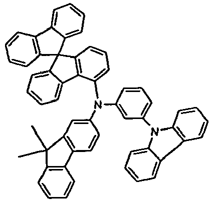 | 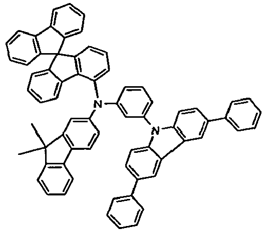 | 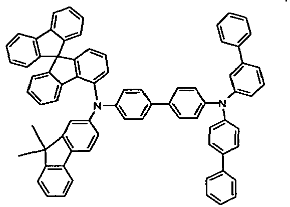 |
| 235 | 236 | 237 |
| 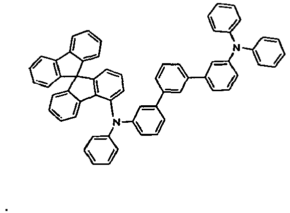 | 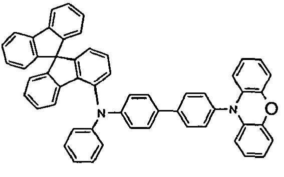 | |
| 238 | 239 | |

Die Synthese der erfindungsgemäßen Verbindungen kann gemäß im Stand der Technik bekannten Verfahren und Reaktionstypen, beispielsweise Halogenierung, Buchwald-Kupplung und Suzuki-Kupplung erfolgen.

Ein bevorzugter Syntheseweg zur Herstellung der erfindungsgemäßen Verbindungen wird im Folgenden dargestellt. Der Syntheseweg umfasst zwei Kupplungsreaktionen: zunächst wird das Fluoren- bzw. Spirobifluorenderivat in einer ersten Buchwald-Kupplung mit einem Amin der Formel Ar³-NH₂ (vgl. Formeln (I) bis (III) der erfindungsgemäßen Verbindungen) umgesetzt. Schließlich erfolgt eine zweite Buchwald-Kupplung, um den Molekülteil enthaltend die zweite Arylamino- bzw. Carbazolgruppe einzuführen.

Der Syntheseweg wird im Folgenden beispielhaft anhand einer Verbindung der Formel (I) dargestellt (Schema 1). Es soll jedoch betont werden, dass auf diesem Syntheseweg gleichermaßen auch erfindungsgemäße Verbindungen der Formel (II) oder (III) hergestellt werden können. Analog zu den dargestellten Fluorenyl-Ausgangsverbindungen können auch Spirobifluorenyl-Verbindungen eingesetzt werden, so dass erfindungsgemäße Verbindungen enthaltend Spirobifluoren-Einheiten erhalten werden. 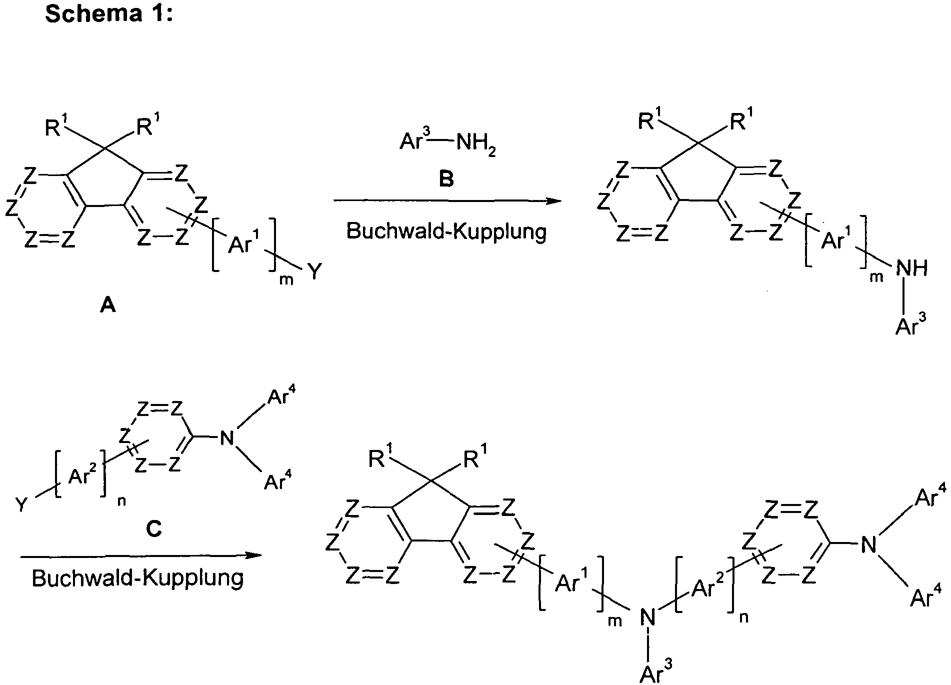 Y = Abgangsgruppe, beispielsweise Halogen

Synthesewege für die Ausgangsverbindungen **A**, **B** und **C**, welche in der Synthese der erfindungsgemäßen Verbindungen eingesetzt werden (vgl. Schema 1), sind dem Fachmann bekannt. Weiterhin werden in den Ausführungsbeispielen einige explizite Syntheseverfahren im Detail dargestellt.

Gegenstand der vorliegenden Erfindung ist somit ein Verfahren zur Herstellung einer Verbindung gemäß Formel (I), (II) oder (III), dadurch gekennzeichnet, dass ein Fluorenyl- oder Spirobifluorenyl-Derivat in einer ersten Kupplungsreaktion mit einer Arylaminoverbindung umgesetzt wird, und das erhaltene Produkt in einer zweiten Kupplungsreaktion mit einer Triarylamino- oder Carbazolverbindung umgesetzt wird.

Die Kupplungsreaktionen stellen dabei bevorzugt Buchwald-Kupplungen dar.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität.

Weiterer Gegenstand der Erfindung sind daher Oligomere, Polymere oder Dendrimere enthaltend eine oder mehrere Verbindungen gemäß Formel (I), (II) oder (III), wobei die Bindung(en) zum Polymer, Oligomer oder Dendrimer an beliebigen, in Formel (I), (II) bzw. (III) mit R¹ substituierten Positionen lokalisiert sein können. Je nach Verknüpfung der erfindungsgemäßen Verbindung ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß Formel (I), (II) oder (III) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I), (II) oder (III) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein. Für die Wiederholeinheiten gemäß Formel (I), (II) oder (III) in Oligomeren, Dendrimeren und Polymeren gelten dieselben Bevorzugungen wie oben für die erfindungsgemäßen Verbindungen beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I), (II) oder (III) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Für die Verarbeitung der Verbindungen gemäß Formel (I), (II) oder (III) aus flüssiger Phase, beispielsweise durch Spin-Coating oder durch Druckverfahren, sind Formulierungen der Verbindungen erforderlich. Diese Formulierungen können beispielsweise Lösungen, Dispersionen oder Miniemulsionen sein.

Gegenstand der Erfindung ist daher weiterhin eine Formulierung, insbesondere eine Lösung, Dispersion oder Miniemulsion, enthaltend mindestens eine Verbindung gemäß Formel (I), (II) oder (III) oder mindestens ein Polymer, Oligomer oder Dendrimer enthaltend mindestens eine Einheit gemäß Formel (I), (II) oder (III) sowie mindestens ein Lösungsmittel, bevorzugt ein organisches Lösungsmittel. Wie solche Lösungen hergestellt werden können, ist dem Fachmann bekannt und beispielsweise in den Anmeldungen WO 2002/072714 und WO 2003/019694 und der darin zitierten Literatur beschrieben.

Die Verbindungen gemäß Formel (I), (II) oder (III) eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und in unterschiedlichen Schichten der organischen Elektrolumineszenzvorrichtung eingesetzt. Bevorzugt werden die Verbindungen als Lochtransportmaterialien in einer Lochtransport- oder Lochinjektionsschicht, als Matrixmaterialien in einer emittierenden Schicht, als Elektronenblockiermaterialien, als Excitonenblockiermaterialien und/oder als Materialien für eine Zwischenschicht (interlayer) eingesetzt.

Weitere Gegenstände der Erfindung sind daher die Verwendung der Verbindungen gemäß Formel (I), (II) oder (III) in elektronischen Vorrichtungen sowie elektronische Vorrichtungen selbst, welche eine oder mehrere Verbindungen gemäß Formel (I), (II) oder (III) enthalten. Dabei sind die elektronischen Vorrichtungen bevorzugt ausgewählt aus der Gruppe bestehend aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und besonders bevorzugt organischen Elektrolumineszenzvorrichtungen (OLEDs). Besonders bevorzugt sind organische Elektrolumineszenzvorrichtungen, enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (I), (II) oder (III) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die organische Elektrolumineszenzvorrichtung kann auch mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, gelbes, orangefarbenes oder rotes Licht emittieren. Insbesondere bevorzugt sind Dreischichtsysteme, also Systeme mit drei emittierenden Schichten, wobei mindestens eine dieser Schichten mindestens eine Verbindung gemäß Formel (I), (II) oder (III) enthält und wobei die drei Schichten blaue, grüne und orangefarbene oder rote Emission zeigen (für den prinzipiellen Aufbau siehe z. B. WO 2005/011013). Alternativ und/oder zusätzlich können die erfindungsgemäßen Verbindungen auch in der Lochtransportschicht und/oder in einer Zwischenschicht (interlayer) vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist erfindungsgemäß bevorzugt, wenn die Verbindung gemäß Formel (I), (II) oder (III) in einer elektronischen Vorrichtung enthaltend einen oder mehrere phosphoreszierende Dotanden eingesetzt wird. Dabei kann die Verbindung in unterschiedlichen Schichten, bevorzugt in einer Lochtransportschicht, einer Lochinjektionsschicht oder in einer emittierenden Schicht verwendet werden. Die Verbindung gemäß Formel (I), (II) oder (III) kann aber auch erfindungsgemäß in einer elektronischen Vorrichtung enthaltend einen oder mehrere fluoreszierende Dotanden eingesetzt werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I), (II) oder (III) in organischen Elektrolumineszenzvorrichtungen einsetzen.

Explizite Beispiele für geeignete phosphoreszierende Emitterverbindungen können weiterhin einer im Folgenden abgebildeten Tabelle entnommen werden.

In einer bevorzugten Ausführungsform der Erfindung werden die Verbindungen gemäß Formel (I), (II) oder (III) als Lochtransportmaterial eingesetzt. Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Die Lochtransportschicht kann direkt an die Emissionschicht angrenzen. Wenn die Verbindungen gemäß Formel (I), (II) oder (III) als Lochtransportmaterial oder als Lochinjektionsmaterial verwendet werden, kann es bevorzugt sein, wenn sie mit Elektronenakzeptor-Verbindungen dotiert sind, beispielsweise mit F₄-TCNQ oder mit Verbindungen, wie sie in EP 1476881 oder EP 1596445 beschrieben werden. In einer weiteren bevorzugten Ausführungsform der Erfindung wird eine Verbindung gemäß Formel (I), (II) oder (III) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt. Wird die Verbindung gemäß Formel (I), (II) oder (III) als Lochtransportmaterial in einer Lochtransportschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen in der Lochtransportschicht eingesetzt werden.

In einer weiteren Ausführungsform der vorliegenden Erfindung werden die Verbindungen der Formel (I), (II) oder (III) als Matrixmaterial in Kombination mit einem oder mehreren Dotanden, vorzugsweise phosphoreszierenden Dotanden, eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer bevorzugten Ausführungsform der Erfindung werden die Verbindungen gemäß Formel (I), (II) oder (III) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Die beiden unterschiedlichen Matrixmaterialien können dabei in einem Verhältnis von 1:10 bis 1:1 vorhanden sein, bevorzugt in einem Verhältnis von 1:4 bis 1:1. Die Mixed-Matrix-Systeme können einen oder mehrere Dotanden umfassen. Die Dotandverbindung bzw. die Dotandverbindungen zusammen haben erfindungsgemäß einen Anteil von 0.1 bis 50.0 Vol.-% an der Gesamtmischung und bevorzugt einen Anteil von 0.5 bis 20.0 Vol.-% an der Gesamtmischung. Entsprechend haben die Matrixkomponenten zusammen einen Anteil von 50.0 bis 99.9 Vol-% an der Gesamtmischung und bevorzugt einen Anteil von 80.0 bis 99.5 Vol.-% an der Gesamtmischung.

Bevorzugt werden Mixed-Matrix-Systeme in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems eingesetzt werden können, sind aromatische Ketone, aromatische Phosphinoxide oder aromatische Sulfoxide oder Sulfone, z. B. gemäß WO 04/013080, WO 04/093207, WO 06/005627 oder WO 10/006680, Triarylamine, Carbazolderivate, z. B. CBP (N,N-Bis-carbazolylbiphenyl) oder die in WO 05/039246, US 2005/0069729, JP 2004/288381, EP 1205527 oder WO 08/086851 offenbarten Carbazolderivate, Indolocarbazolderivate, z. B. gemäß WO 07/063754 oder WO 08/056746, Azacarbazolderivate, z. B. gemäß EP 1617710, EP 1617711, EP 1731584, JP 2005/347160, bipolare Matrixmaterialien, z. B. gemäß WO 07/137725, Silane, z. B. gemäß WO 05/111172, Azaborole oder Boronester, z. B. gemäß WO 06/117052, Triazinderivate, z. B. gemäß der Anmeldung WO 2010/015306, WO 2007/063754 oder WO 2008/056746, Zinkkomplexe, z. B. gemäß EP 652273 oder WO 09/062578, Diazasilol- bzw. Tetraazasilol-Derivate, z. B. gemäß WO 10/054729, Diazaphosphol-Derivate, z. B. gemäß WO 10/054730, oder Indenocarbazolderivate, z. B. gemäß WO 10/136109.

Bevorzugte phosphoreszierende Dotanden zur Verwendung in Mixed-Matrix-Systemen enthaltend die erfindungsgemäßen Verbindungen sind die in einer folgenden Tabelle aufgeführten phosphoreszierenden Dotanden.

Gemäß einer weiteren bevorzugten Ausführungsform der Erfindung werden die Verbindungen gemäß Formel (I), (II) oder (III) in einer Zwischenschicht (interlayer) eingesetzt. Zwischenschichten werden bevorzugt in organischen Elektrolumineszenzvorrichtungen enthaltend mehrere emittierende Schichten eingesetzt, beispielsweise in weiß emittierenden OLEDs, welche jeweils eine rot emittierende, eine grün emittierende und eine blau emittierende Schicht enthalten. Besonders bevorzugt sind Zwischenschichten zwischen zwei emittierenden Schichten angeordnet. Eine Zwischenschicht enthaltend eine erfindungsgemäße Verbindung ist gemäß einer bevorzugten Ausführungsform der Erfindung zwischen der blau emittierenden Schicht und der grün emittierenden Schicht einer weißes Licht emittierenden OLED, welche eine rot emittierende, eine grün emittierende und eine blau emittierende Schicht enthält, angeordnet. Besonders bevorzugt ist die blau emittierende Schicht dabei eine fluoreszierende Schicht, und die grün emittierende Schicht ist eine phosphoreszierende Schicht.

Die in der folgenden Tabelle aufgeführten Verbindungen stellen besonders geeignete phosphoreszierende Dotanden dar.

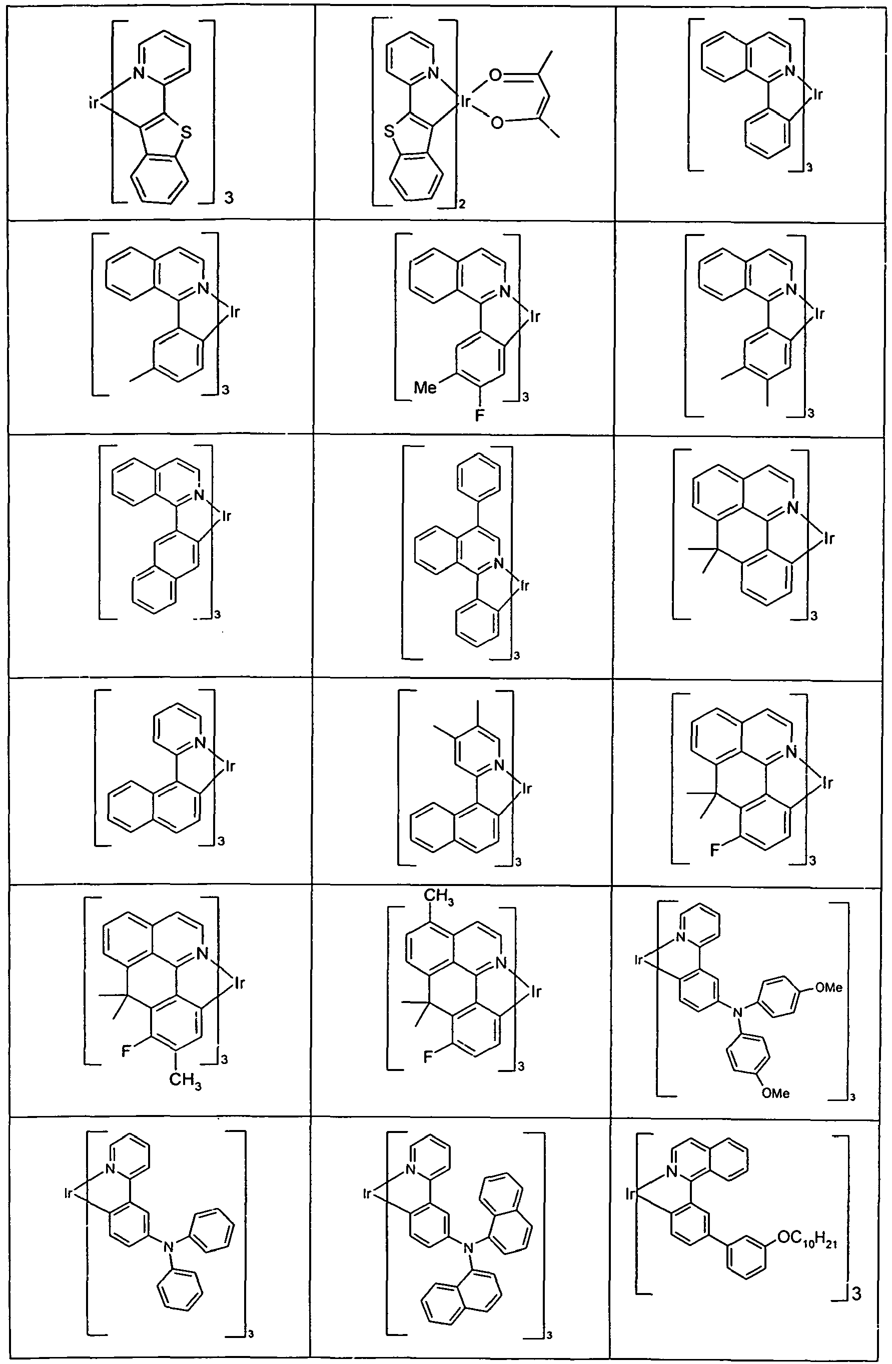

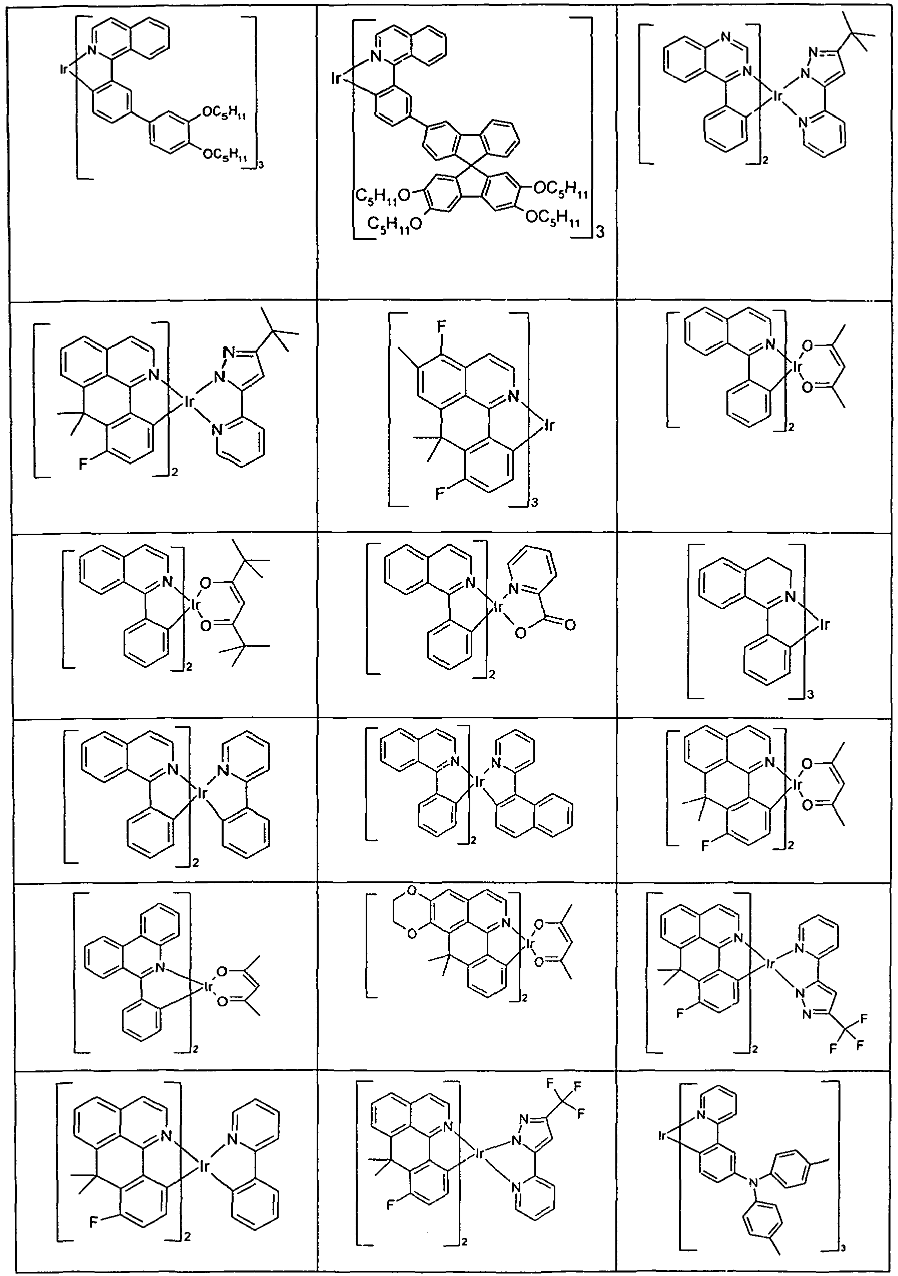

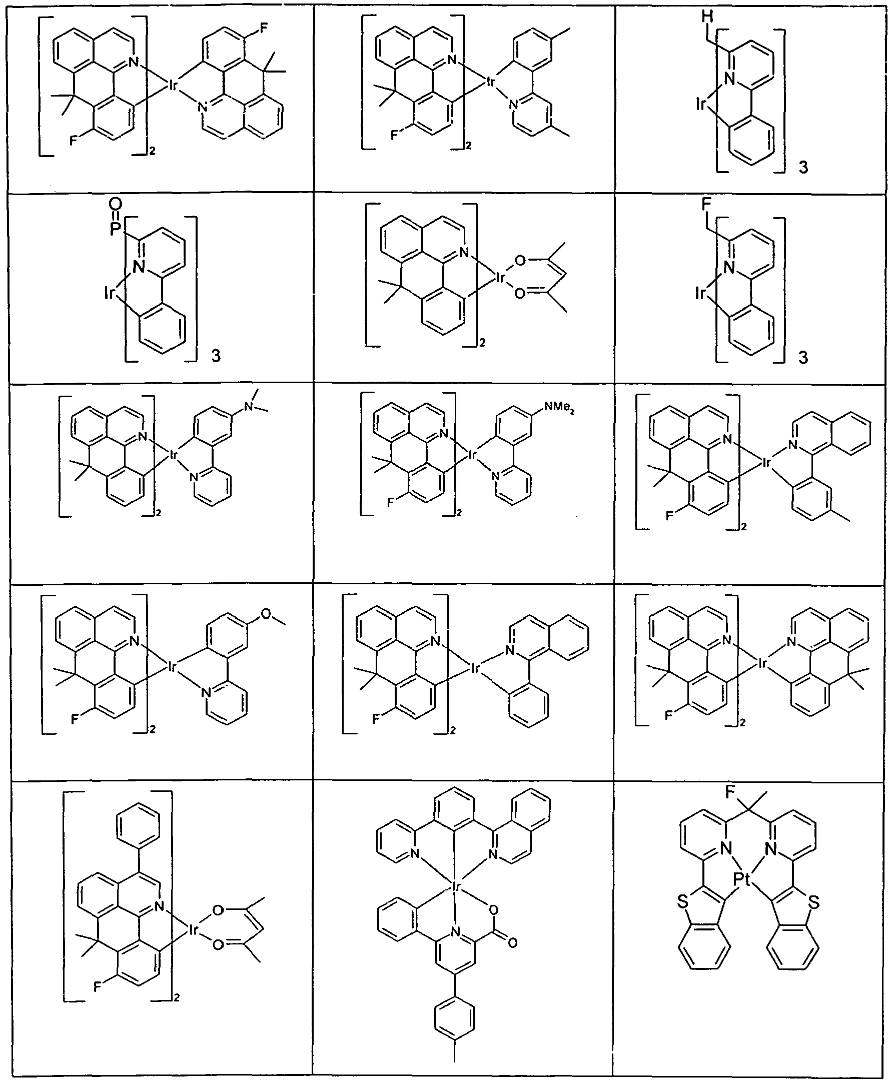

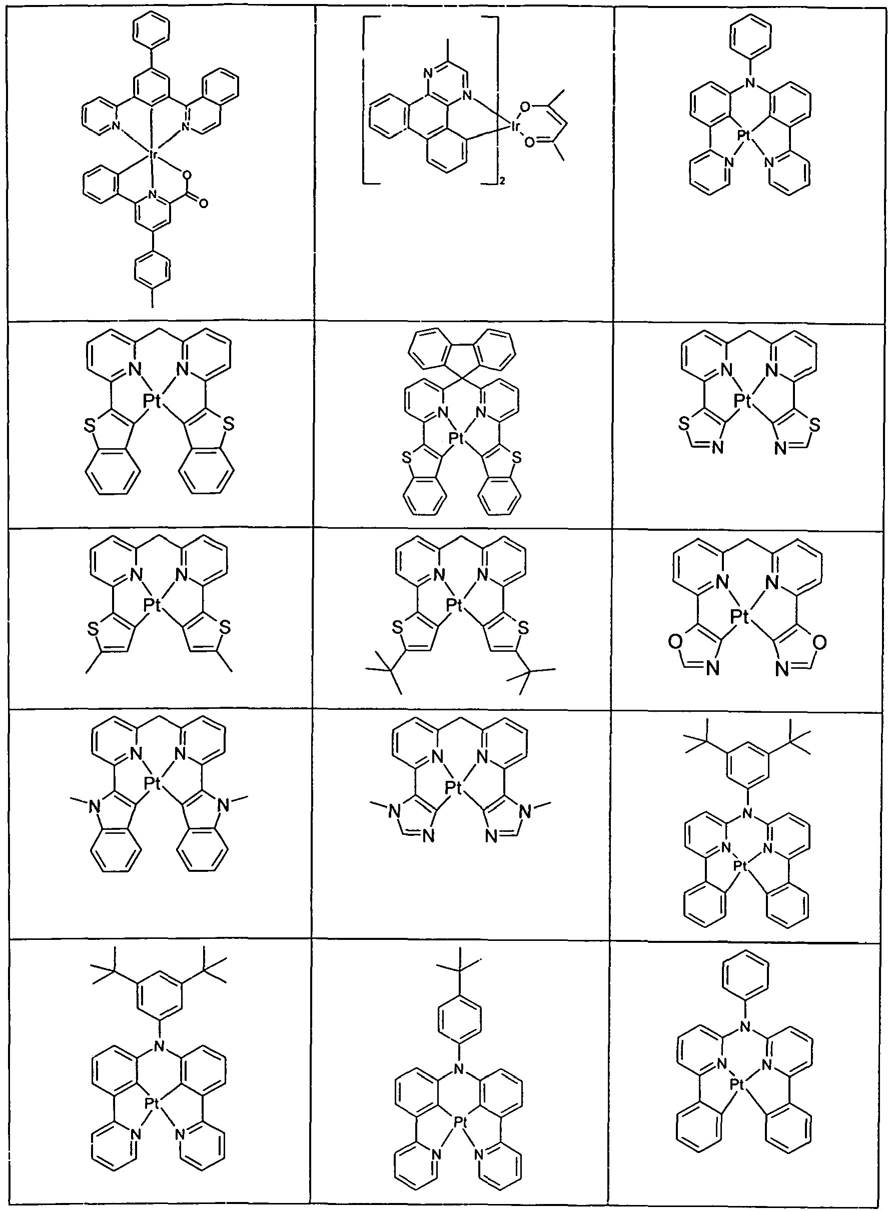

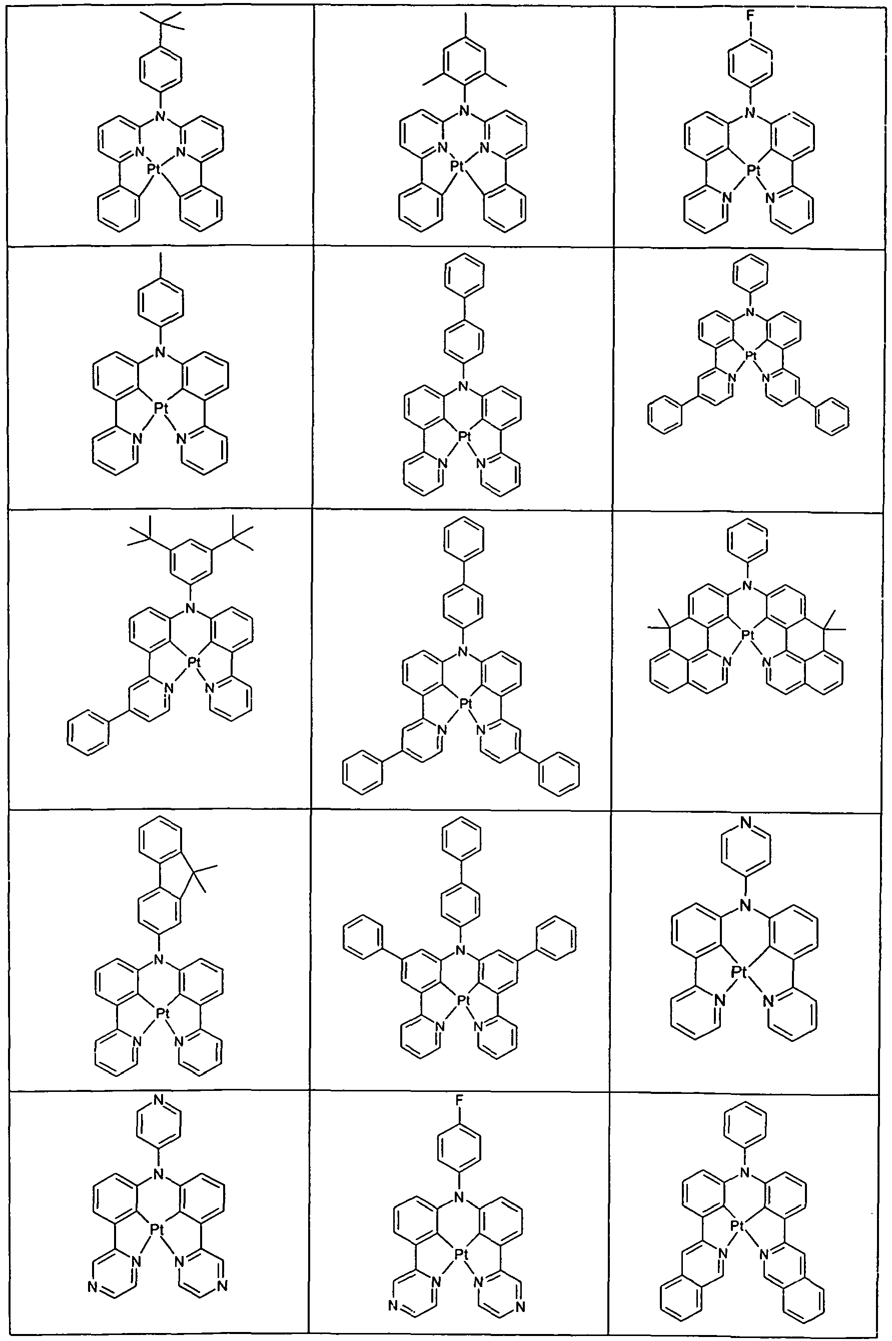

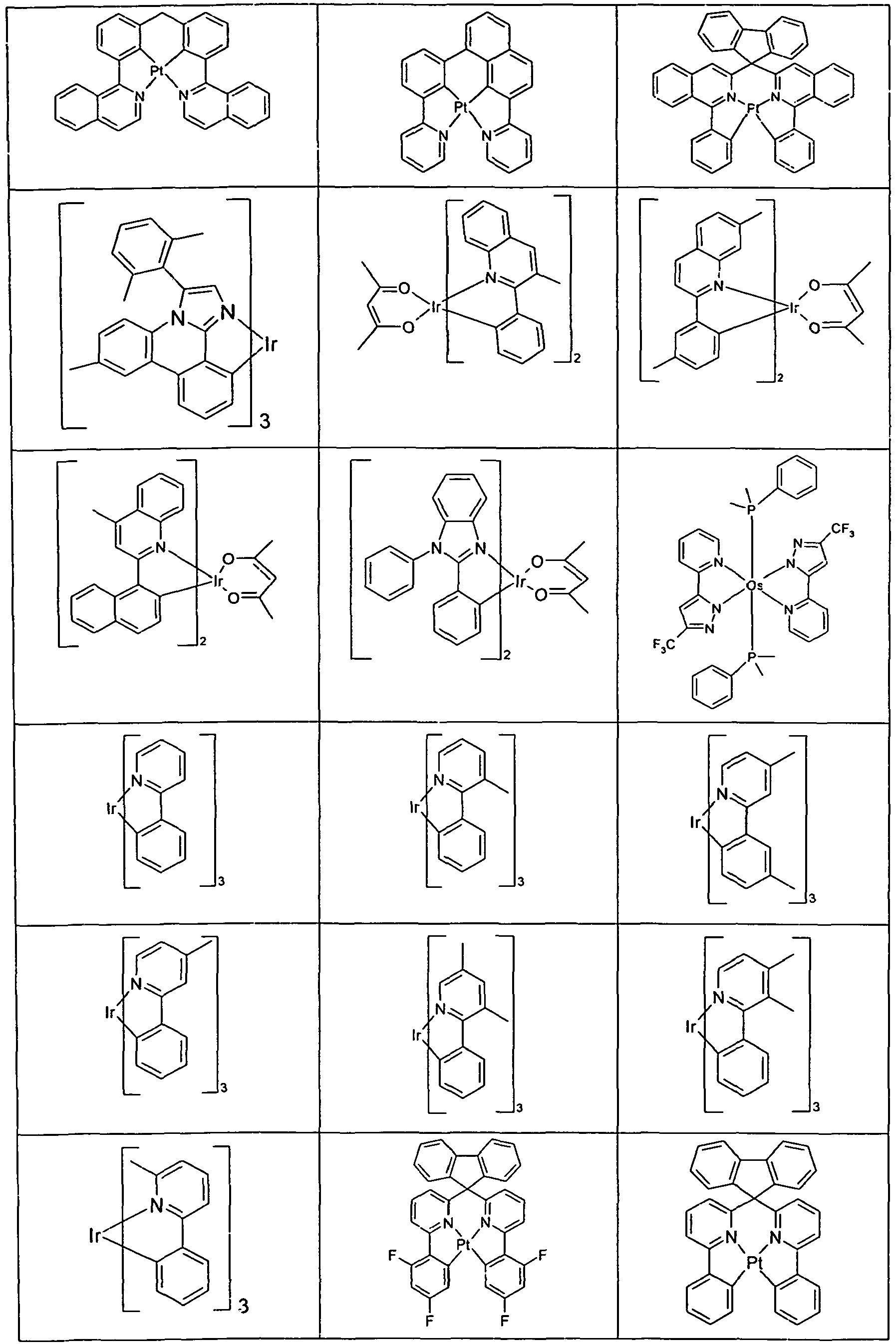

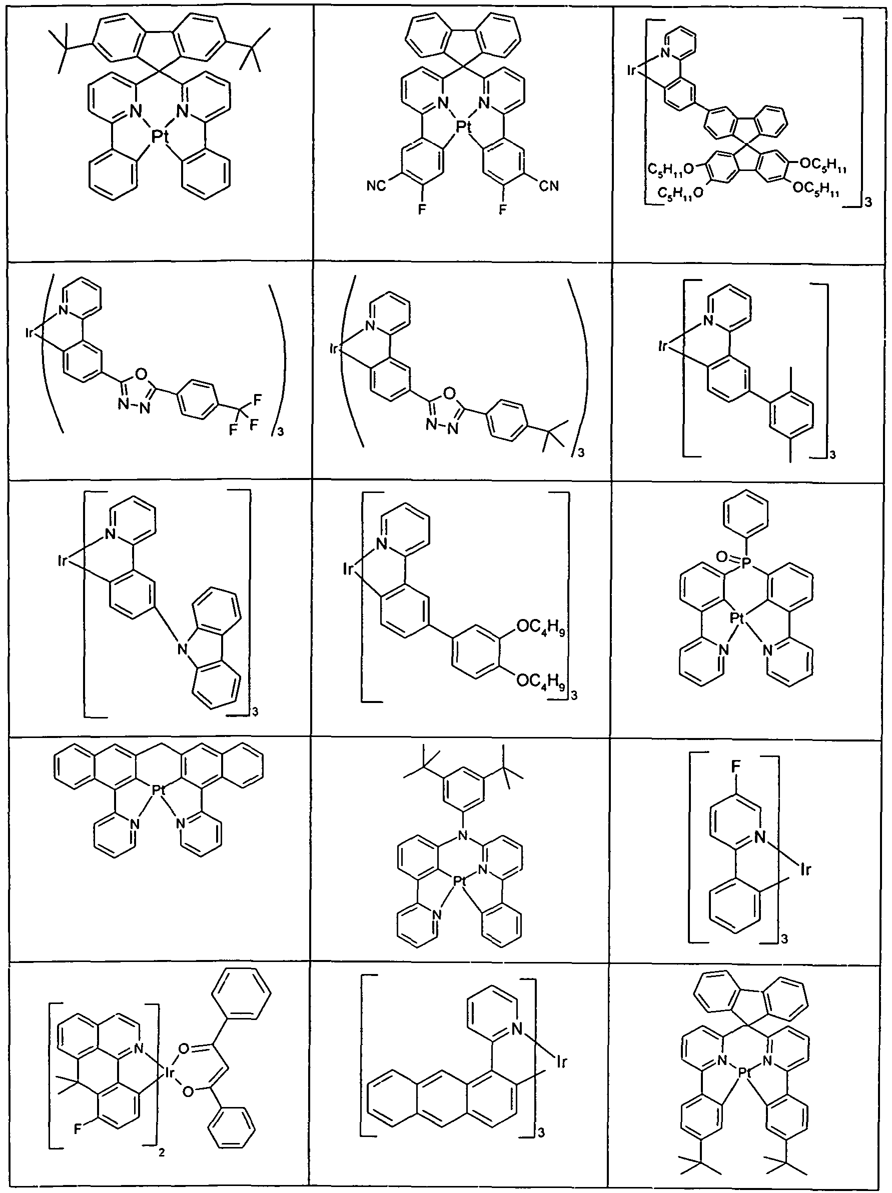

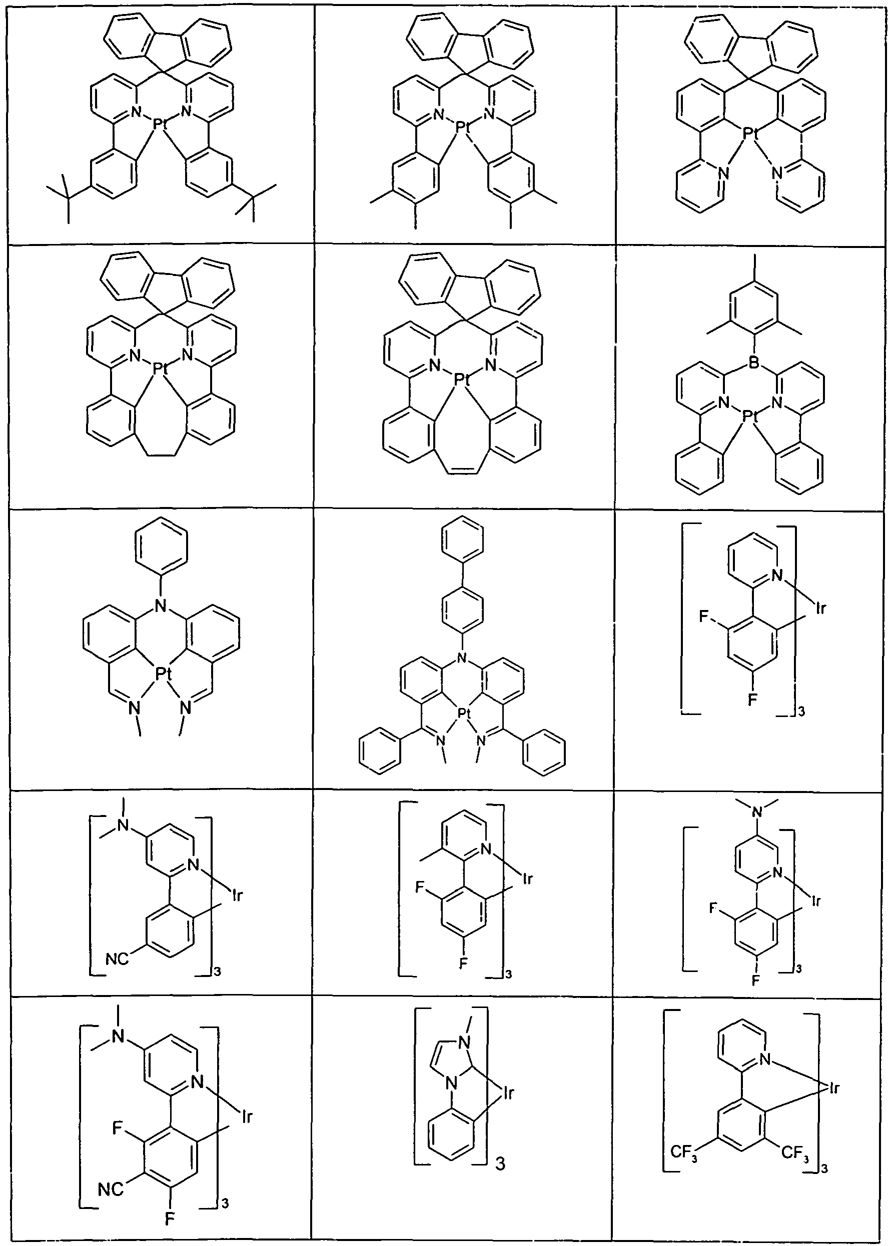

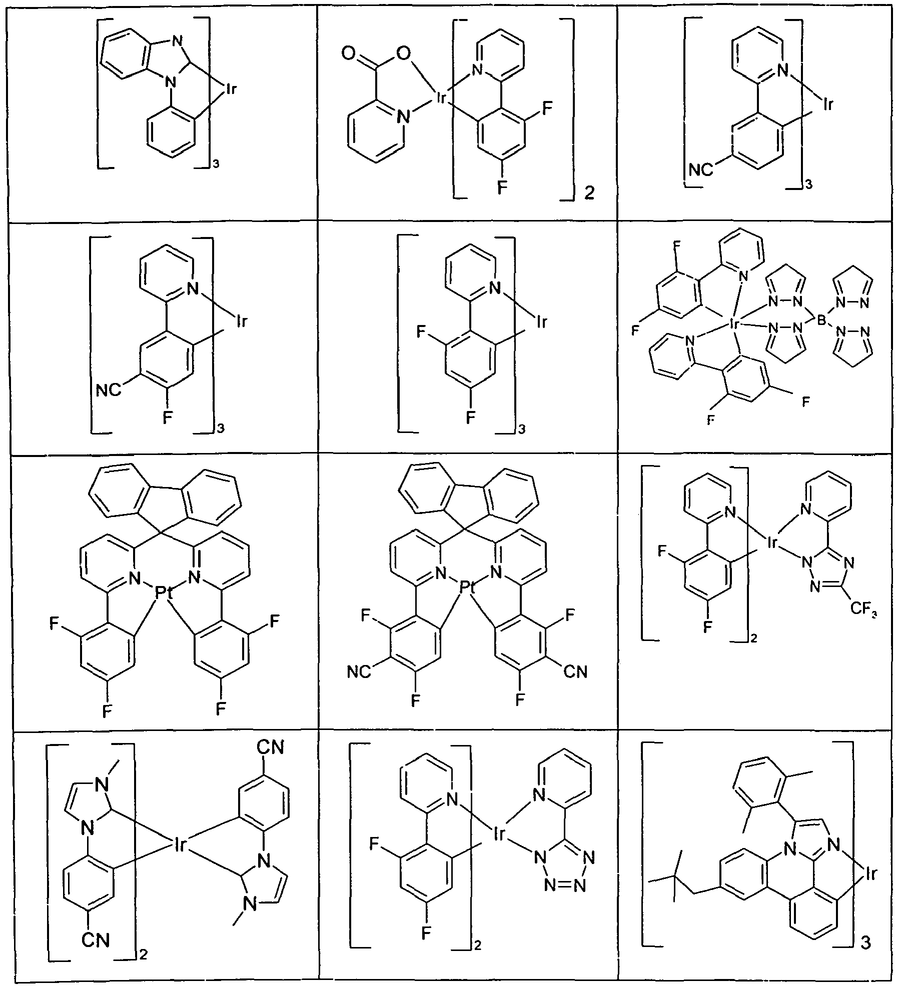

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte fluoreszierende Dotanden sind gewählt aus Indenofluorenaminen bzw. -diaminen, beispielsweise gemäß WO 2006/122630, Benzoindenofluorenaminen bzw. -diaminen, beispielsweise gemäß WO 2008/006449, und Dibenzoindenofluorenaminen bzw. -diaminen, beispielsweise gemäß WO 2007/140847. Weiterhin bevorzugt sind die in WO 2010/012328 offenbarten kondensierten Kohlenwasserstoffe.

Geeignete fluoreszierende Dotanden sind weiterhin die in JP 2006/001973, WO 2004/047499, WO 2006/098080, WO 2007/065678, US 2005/0260442 und WO 2004/092111 offenbarten Derivate dieser Strukturen.

Als Matrixmaterialien, bevorzugt für fluoreszierende Dotanden, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den erfindungsgemäßen Verbindungen Carbazolderivate (z. B. CBP (N,N-Biscarbazolylbiphenyl) oder Verbindungen gemäß WO 2005/039246, US 2005/0069729, JP 2004/288381, EP 1205527 oder WO 2008/086851), Triarylamine, Azacarbazole (z. B. gemäß EP 1617710, EP 1617711, EP 1731584, JP 2005/347160), Indolocarbazolderivate, z. B. gemäß WO 2007/063754 oder WO 2008/056746, Ketone (z. B. gemäß WO 2004/093207 oder WO 2010/006680), Phosphinoxide, Sulfoxide und Sulfone (z. B. gemäß WO 2005/003253), Oligophenylene, aromatische Amine (z. B. gemäß US 2005/0069729), bipolare Matrixmaterialien (z. B. gemäß WO 2007/137725), Silane (z. B. gemäß WO 2005/111172), Azaborole oder Boronester, z. B. gemäß WO 2006/117052, Triazinderivate, z. B. gemäß WO 2010/015306, WO 2007/063754 oder WO 2008/056746, Zinkkomplexe (z. B. gemäß WO 2009/062578) Aluminiumkomplexe (z. B. BAlq), Diazasilol- und Tetraazasilol-Derivate, z. B. gemäß WO 2010/054730, Indenocarbazolderivate, z. B. gemäß WO 2010/136109 und WO 2011/000455 oder Diazaphosphole, z. B. gemäß WO 2010/054730.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden. Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, AI, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder AI, wobei dann in der Regel Kombinationen der Metalle, wie beispielsweise Ca/Ag, Ba/Ag oder Mg/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink-Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I), (II) oder (III) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Die organischen Elektrolumineszenzvorrichtungen enthaltend eine oder mehrere erfindungsgemäße Verbindungen können in Displays, als Lichtquellen in Beleuchtungsanwendungen sowie als Lichtquellen in medizinischen und/oder kosmetischen Anwendungen (z.B. Lichttherapie) eingesetzt werden.

Die erfindungsgemäßen Verbindungen zeichnen sich insbesondere dadurch aus, dass sie bei Verwendung in organischen Elektrolumineszenzvorrichtungen gute Leistungseffizienzen, geringe Betriebsspannungen und lange Lebensdauern der Vorrichtungen bewirken.

Weiterhin sind die Verbindungen oxidationsstabil, temperaturstabil und weisen eine hohe Glasübergangstemperatur auf, was sowohl für die Prozessierbarkeit, beispielsweise aus Lösung oder aus der Gasphase, als auch für die Verwendung in elektronischen Vorrichtungen vorteilhaft ist.

Weiterhin weisen die Verbindungen ein hochliegendes angeregtes Triplettniveau auf, was insbesondere bei Verwendung in einer emittierenden Schicht in Kombination mit einer phosphoreszierenden Emitterverbindung hoch erwünscht ist. Insbesondere weisen die Verbindungen ein höheres angeregtes Triplettniveau auf als die entsprechenden 2-Fluorenyl-Derivate.

Weiterhin weisen die Verbindungen eine hohe Lochbeweglichkeit auf, was insbesondere bei der Verwendung als Lochtransportmaterial bzw. Lochinjektionsmaterial hoch erwünscht ist.

Die Erfindung wird durch die nachfolgenden Anwendungsbeispiele näher erläutert, wobei die Erfindung nicht auf den Umfang der Beispiele beschränkt ist.

### Ausführungsbeispiele

### Synthesen der Verbindungen 1 bis 13 (nicht erfindungsgemäße Verbindungen sind mit * markiert):

Die nachfolgenden Synthesen werden, sofern nicht anders angegeben, unter einer Schutzgasatmosphäre durchgeführt. Die Edukte können von den Firmen ALDRICH bzw. ABCR (Palladium(II)acetat, Tri-o-tolylphosphin, Anorganika, Lösemittel) bezogen werden. Die Synthese von 3-Bromfluorenon (Tetrahedron, 51,7, 2039-54; 1995) und 3-Bromfluoren kann gemäß der Literatur (Tetrahedron Letters, 51,37, 4894-4897; 2010) erfolgen, ebenfalls die Synthese von Bis-biphenyl-4-yl-(4'-bromo-biphenyl-4-yl)-amin (JP 2010-111605).

### Vorstufe A: 3-Bromo-9H-fluoren

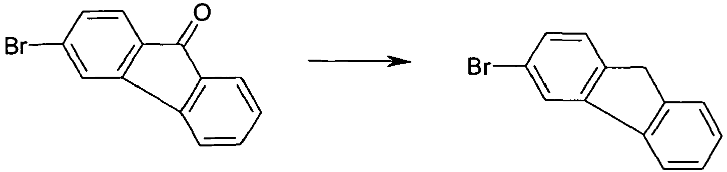

Eine gut gerührte, unter Rückfluss kochende Suspension von 64 g (250 mmol) 3-Bromfluoren in einem Gemisch von 1000 ml Toluol und 2000 ml Ethanol wird mit 49 ml (1000 mmol) Hydrazinhydrat und dann mit 3 g frisch bereitetem Raney-Nickel versetzt. Nach 2 h unter Rückfluss lässt man die Mischung erkalten, entfernt das Lösemittel im Vakuum, nimmt den Rückstand in 1000 ml warmem Chloroform auf, filtriert die Lösung über Kieselgel, engt die klare Lösung auf 100 ml ein und gibt 300 ml Ethanol zu. Nach 12 h Stehenlassen werden die farblosen Kristalle abgesaugt und anschließend zweimal aus Chloroform/Ethanol umkristallisiert. Ausbeute: 60 g (240 mmol), 98 % d. Th.; Reinheit: 97 % nach ¹H-NMR.

### Vorstufe B: 3-Brom-9,9-dimethyl-9H-fluoren

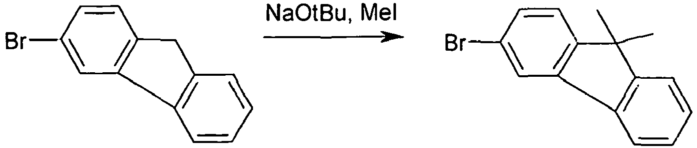

37 g (152 mmol) 3-Bromo-9H-fluoren werden in einem ausgeheizten Kolben in 600 mL getrocknetem DMSO gelöst. Bei Raumtemperatur werden 43.9 g (457 mmol) NaO*^{t}*Bu zugegeben. Die nunmehr blaue Suspension wird auf eine Innentemperatur von 80 °C gebracht. Bei dieser Temperatur werden zur jetzt violetten Lösung 64.8 g (457 mmol) lodmethan so zugetropft, dass die Innentemperatur 90 °C nicht übersteigt (Dauer: ca. 30 min). Der Ansatz wird für weitere 30 min bei 80-90 °C Innentemperatur gehalten, dann auf 1500 mL Eiswasser gegossen und ca. 20 min gerührt. Der ausgefallene Feststoff wird abgesaugt und nacheinander mit ca. 200 mL H₂O und Methanol gewaschen. Ausbeute: 39 g (144 mmol), 96 % d. Th.; Reinheit: 95 % n. ¹H-NMR.

### Vorstufe C: 3-Brom-9,9'-spirobifluoren

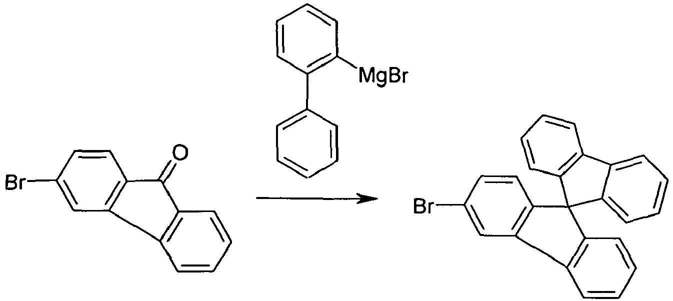

Das Grignardreagenz, bereitet aus 9.9 g (400 mmol) Magnesiumspänen und 93.2 g (68 ml, 400 mmol) 2-Brombiphenyl in 500 ml trockenem Diethylether, wird im Verlauf von 2 h zu einer siedenden Lösung von 103 g (400 mmol) 3-Brom-9-fluorenon in 100 ml trockenem Diethylether getropft. Nach beendeter Zugabe wird 3 Stunden weiter zum Sieden erhitzt. Nach Abkühlung über Nacht wird der ausgefallene Niederschlag abgesaugt und mit kaltem Ether gewaschen. Der Rückstand wird in einer Lösung von 15 g Ammoniumchlorid in 250 ml Eiswasser hydrolysiert. Nach 1 h wird der gebildete Alkohol abgesaugt, mit Wasser gewaschen und trockengesaugt.

Das getrocknete Fluorenol wird für die Ringschlußreaktion in 100 ml Eisessig, nach Zugabe von 3 Tropfen HCl conc. 6 Stunden gekocht. Man lässt über Nacht kristallisieren, saugt das gebildete Produkt ab und wäscht mit Eisessig und Wasser. Ausbeute: 141 g (356 mmol), 95 % d. Th.; Reinheit: 96 % n. ¹H-NMR.

### Vorstufe D: Synthese von 9,9-Dimethyl -9,9'-spirobifluoren-3-boronsäure

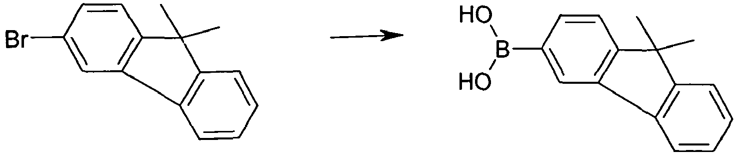

70 g (259 mmol) 3-Brom-9,9-dimethyl-9H-fluoren werden in 1500 mL trockenem THF gelöst, bei -70 °C werden 135 mL (337 mmol) einer 2.5 M Lösung von n-Butyllithium in Cyclohexan zugetropft und nach 1 h werden 37 mL Trimethylborat (336 mmol) zugetropft. Man lässt innerhalb 1 h auf Raumtemperatur kommen und entfernt das Lösungsmittel. Der Rückstand, der nach ¹H-NMR einheitlich ist, wird ohne weitere Reinigung in die Folgereaktion eingesetzt. Die Ausbeute beträgt 55 g (230 mmol), entsprechend 90 % der Theorie.

Analog werden die Vorstufen **E** und **F** erhalten:

| **Bsp.** | **Edukt 1** | **Produkt** | **Ausbeute** |
|---|---|---|---|
| **E** | 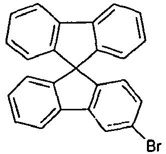 | 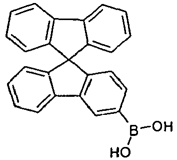 | 88 % |
| **F** | 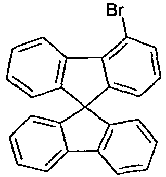 | 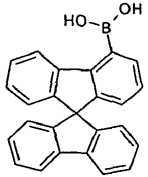 | 69% |
| | 1161009-88-6 | | |

### Vorstufe G: 3-(4-Brom-phenyl)-9,9-dimethyl-9H-fluoren

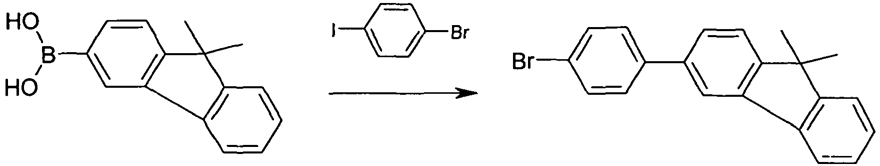

45 g (190 mmol) 9,9-Dimethyl -9,9'-spirobifluoren-3-boronsäure, 53 g (190 mmol) Iodbrombenzol und 13 g (123 mmol) Natriumcarbonat werden in 180 mL Toluol, 180 mL Dioxan und 60 mL Wasser suspendiert. Zu dieser Suspension werden 3.0 mg (2.6 mmol) Pd(PPh₃)₄ gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan/*iso*-Propanol umkristallisiert und abschließend im Hochvakuum sublimiert. Die Reinheit beträgt 99.9 %. Die Ausbeute beträgt 51 g (147 mmol), entsprechend 78 % der Theorie.

Analog werden die Vorstufen **H** und **I** erhalten:

| **Bsp.** | **Edukt 1** | **Produkt** | **Ausbeute** |
|---|---|---|---|
| **H** | 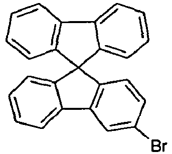 | 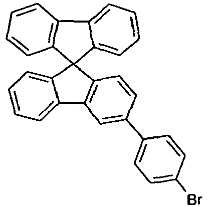 | 81 % |
| **I** | 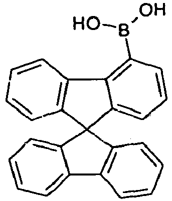 | 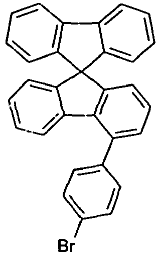 | 66% |

### Vorstufe J: Synthese von (9,9-Dimethyl-9H-fluoren-3-yl)-phenyl-amin

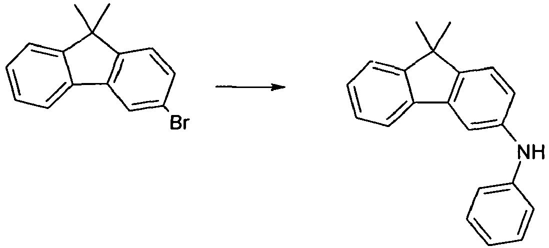

50 g (183 mmol) 3-Brom-9,9-dimethyl-9H-fluoren, 20ml (220 mmol) Anilin, 1.5 g (2.7 mmol) DPPF, 0.5 g Palladium(II)acetat und 45 g (486 mmol) Natrium-tert-butylat werden in 1.5 L Toluol 18 h unter Schutzatmosphäre zum Sieden erhitzt. Das Gemisch wird im Anschluss zwischen Toluol und Wasser verteilt, die organische Phase wird dreimal mit Wasser gewaschen und über Na₂SO₄ getrocknet und einrotiert. Der verbleibende Rückstand wird aus Heptan/Essigester umkristallisiert. Die Ausbeute beträgt 31.2 g (110 mmol, 52%). Ausbeute: 46 g (165 mmol), 78 % d. Th.; Reinheit: 97% n. ¹H-NMR.

Analog werden die Verbindungen **K** bis **X** erhalten:

| **Bsp.** | **Edukt 1** | **Edukt 2** | **Produkt** | **Ausbeute** |
|---|---|---|---|---|
| **K** | 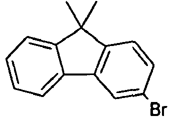 | 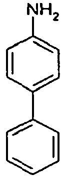 | 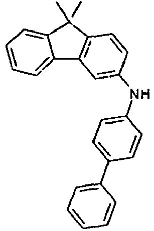 | 81 % |
| | | 3315-50-2 | | |
| **L** | 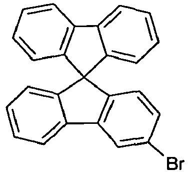 | 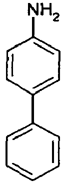 | 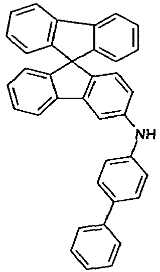 | 85% |
| | | 3315-50-2 | | |
| **M** | 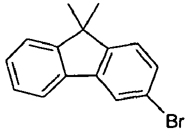 | 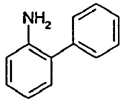 | 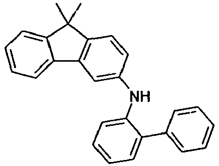 | 73% |
| | | 90-41-5 | | |
| **N** | 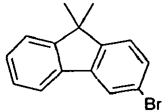 | 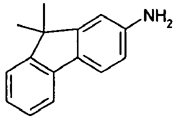 | 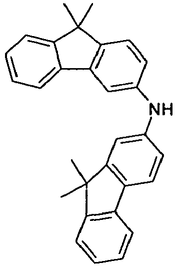 | 75% |
| | | 108714-73-4 | | |
| **O** | 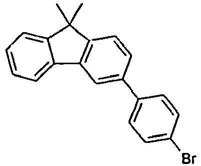 | 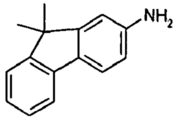 | 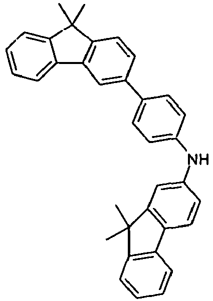 | 70% |
| **P** | 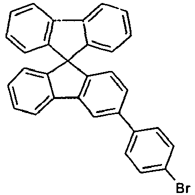 | 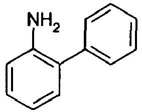 | 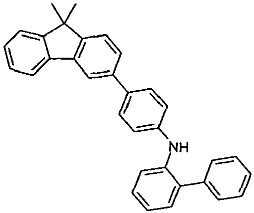 | 71% |
| **Q** | 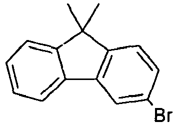 | 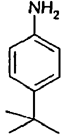 | 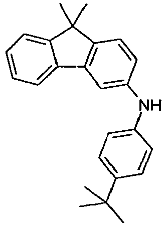 | 86% |
| **R** | 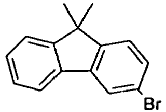 | 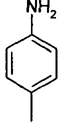 | 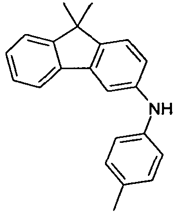 | 89% |
| **S** | 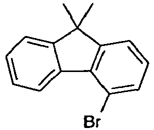 | 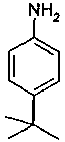 | 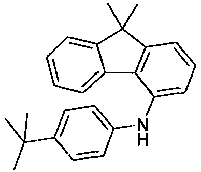 | 72% |
| | 942615-32-9 | | | |
| **T** | 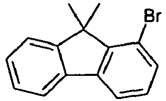 | 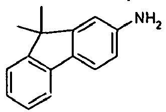 | 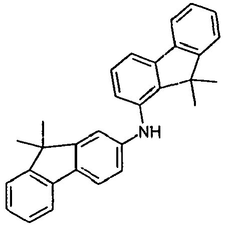 | 75% |
| | 1225053-54-2 | | | |
| **U** | 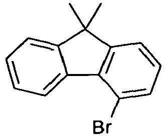 | 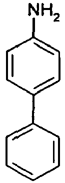 | 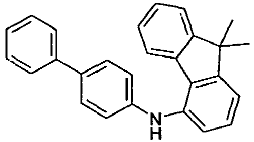 | 79% |
| | 942615-32-9 | 3315-50-2 | | |
| **V** | 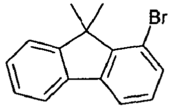 | 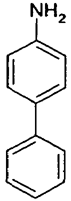 | 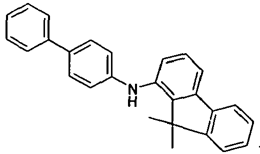 | 72% |
| | 942615-32-9 | 3315-50-2 | | |
| **W** | 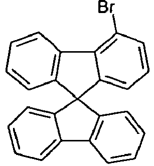 | 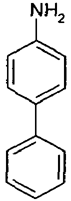 | 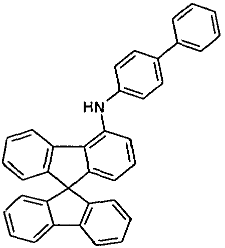 | 70% |
| | | 3315-50-2 | | |
| **X** | 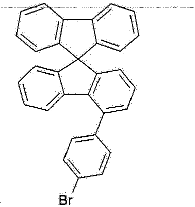 | 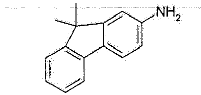 | 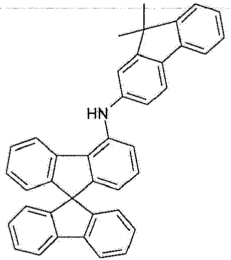 | 65% |
| | | 108714-73-4 | | |

### Beispielverbindung 1 *:

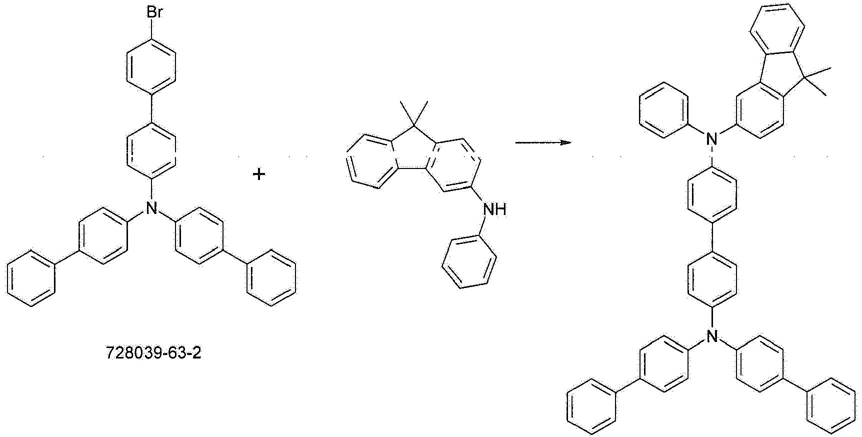

Eine entgaste Lösung von 48 g (87 mmol) Bis-biphenyl-4-yl-(4'-brombiphenyl-4-yl)-amin und 23 g (80 mmol) (9,9-Dimethyl-9H-fluoren-3-yl)-phenylamin in 1000 mL Dioxan wird 1 h mit N₂ gesättigt. Danach wird die Lösung zuerst mit 0.9 mL (4.3 mmol) P(*t*Bu)₃, dann mit 0.48 g (2.1 mmol) Palladium(II)acetat versetzt. Anschließend wird 12.6 g (131 mmol) NaOtBu im festen Zustand zugegeben. Die Reaktionsmischung wird 18 h unter Rückfluss erhitzt. Nach Abkühlen auf Raumtemperatur werden vorsichtig 1000 mL Wasser zugesetzt. Die organische Phase wird mit 4 x 50 mL Wasser gewaschen, über MgSO₄ getrocknet und das Lösungsmittel im Vakuum entfernt. Das reine Produkt erhält man durch Umkristallisation und abschließende Sublimation. Ausbeute: 46 g (61 mmol), 71% d. Th., Reinheit nach HPLC 99.9%.

Analog werden die Verbindungen **2** bis **18** erhalten:

| **Bsp.** | **Edukt 1** | **Edukt 2** | **Produkt** | **Ausbeute** |
|---|---|---|---|---|
| **2** | 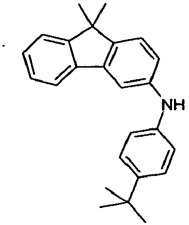 | 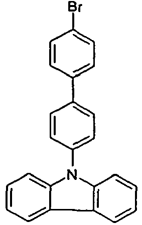 | 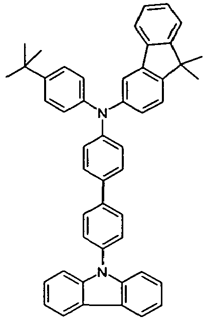 | 83 % |
| | | **212385-73-4** | | |
| **3** | 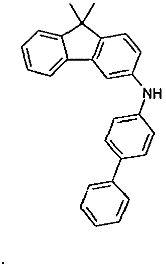 | 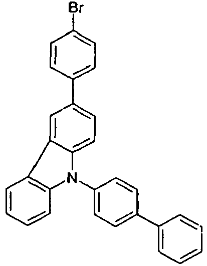 | 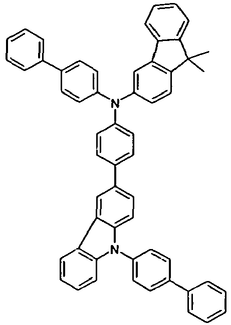 | 80% |
| | | **1028648-25-0** | | |
| **4** | 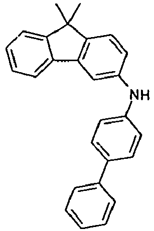 | 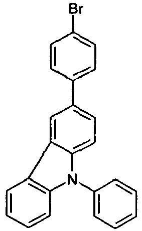 | 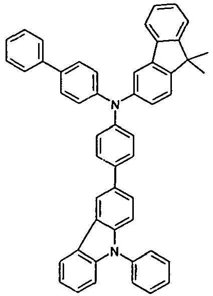 | 84% |
| | | **1028647-93-9** | | |
| **5** | 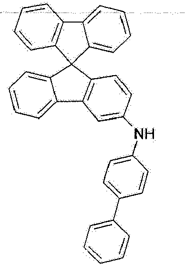 | 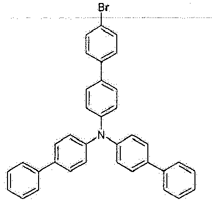 | 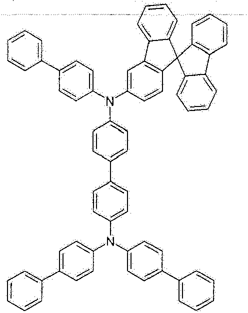 | 80% |
| | | **728039-63-2** | | |
| **6 *** | 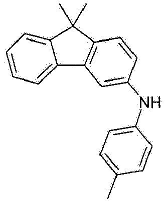 | 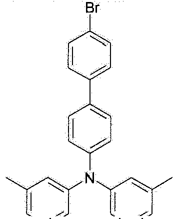 | 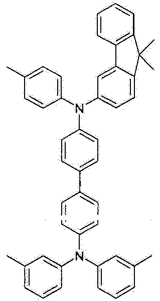 | 77% |
| | | **765271-17-8** | | |
| **7** | 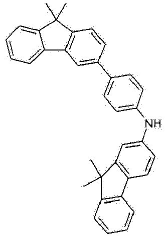 | 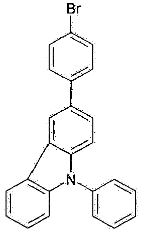 | 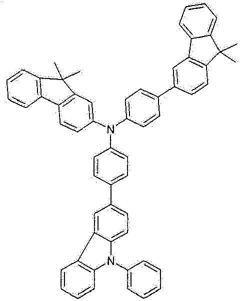 | 72% |
| | | **1028647-93-9** | | |
| **8** | 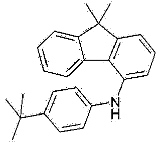 | 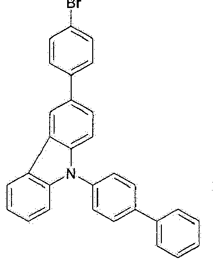 | 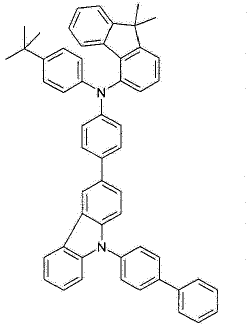 | 65% |
| | | **1028648-25-0** | | |
| **9** | 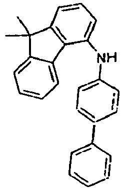 | 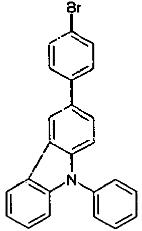 | 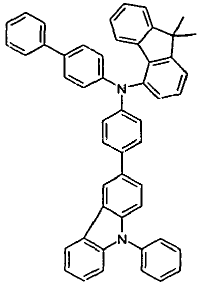 | 84% |
| | | **1028647-93-9** | | |
| **10** | 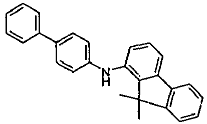 | 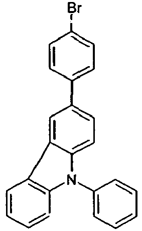 | 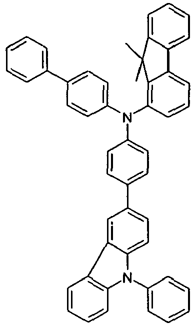 | 78% |
| | | **1028647-93-9** | | |
| **11** | 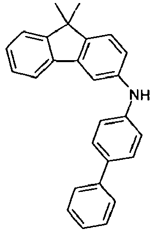 | 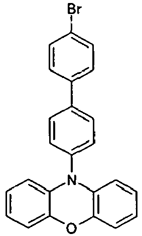 | 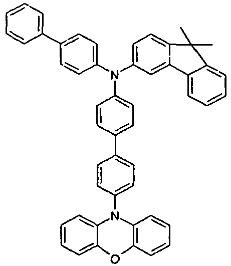 | 69% |
| | | **308144-66-3** | | |
| **12** | 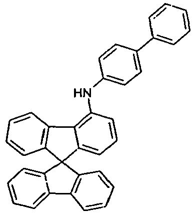 | 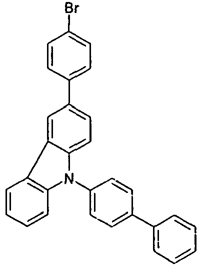 **1028648-25-0** | 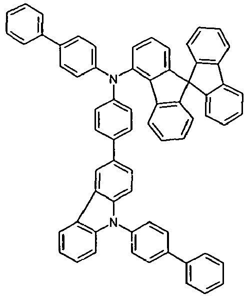 | 63% |
| **13** | 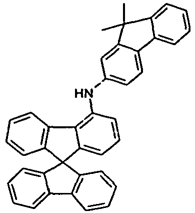 | 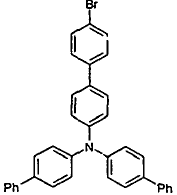 | 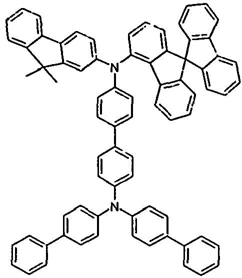 | 73% |
| | | **728039-63-2** | | |
| **14** | 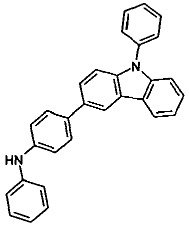 | 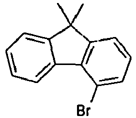 | 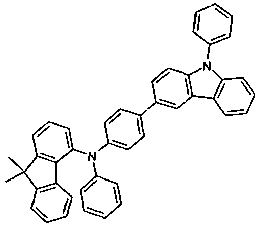 | 76% |
| | **1072194-21-8** | **942615-32-9** | | |
| **15** | 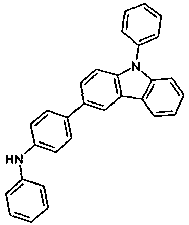 | 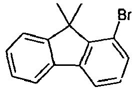 | 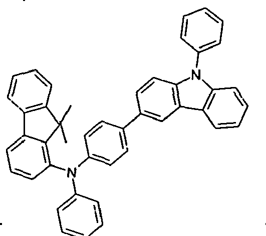 | 77% |
| | **1072194-21-8** | **1225053-54-2** | | |
| **16** | 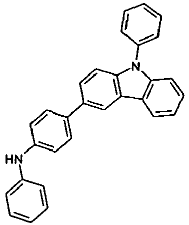 | 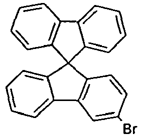 | 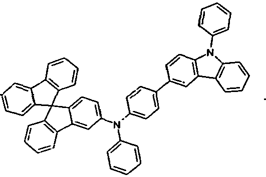 | 79% |
| | **1072194-21-8** | | | |
| **17** | 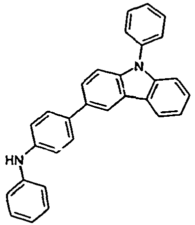 | 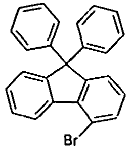 | 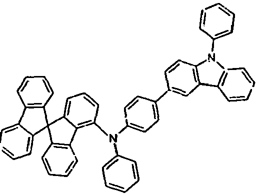 | 73% |
| | **1072194-21-8** | **713125-22-5** | | |
| **18** | 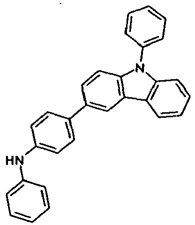 | 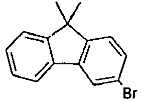 | 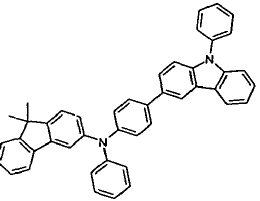 | 76% |
| | **1072194-21-8** | | | |

### Devicebeispiele: Herstellung der OLEDs

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen E1 bis E16 (siehe Tabellen 3 und 5 mit Devicedaten und Tabellen 2 und 4 mit den entsprechenden Angaben zu den Devicestrukturen) werden die Daten verschiedener OLEDs vorgestellt. Glasplättchen, die mit strukturiertem ITO (Indium Zinn Oxid) der Dicke 150 nm beschichtet sind, werden zur verbesserten Prozessierung mit 20 nm PEDOT beschichtet (Poly(3,4-ethylendioxy-2,5-thiophen), aus Wasser aufgeschleudert; bezogen von H. C. Starck, Goslar, Deutschland). Diese beschichteten Glasplättchen bilden die Substrate, auf welche die OLEDs aufgebracht werden. Die OLEDs haben prinzipiell folgenden Schichtaufbau: Substrat / Optionale Lochinjektionsschicht (HIL) / Lochtransportschicht (HTL) / Optionale Zwischenschicht (IL) / Elektronenblockerschicht (EBL) / Emissionsschicht (EML) / Optionale Lochblockierschicht (HBL) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist den Tabellen 2 und 4 zu entnehmen. Die zur Herstellung der OLEDs benötigten Materialien sind in Tabelle 1 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB1 (95%:5%) bedeutet hierbei, dass das Material H1 in einem Volumenanteil von 95% und SEB1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U @ 1000 cd/m² in Tabelle 3 und 5 bezeichnet die Spannung, die für eine Leuchtdichte von 1000 cd/m² benötigt wird. EQE @ 1000 cd/m² schließlich bezeichnet die externe Quanteneffizienz bei einer Betriebsleuchtdichte von 1000 cd/m². LD80 @ 6000 cd/m2 ist die Lebensdauer bis das OLED bei einer Helligkeit von 6000 cd/m² auf 80 % der Anfangsintensität, also auf 4800 cd/m² abgefallen ist. Die Devicedaten der verschiedenen OLEDs sind in Tabelle 3 und 5 zusammengefasst, während die Tabellen 2 und 4 die entsprechenden Device-Aufbauten wiedergeben.

### Verwendung von erfindungsgemäßen Verbindungen als Elektronenblockiermaterialien / Lochtransportmaterialien in fluoreszierenden und phosphoreszierenden OLEDs

Insbesondere eignen sich erfindungsgemäße Verbindungen als Lochtransportmaterial (HTM), als Matrixmaterial oder als Elektronenblockiermaterial (EBM) in OLEDs. Sie eignen sich zur Verwendung als Einzelmaterialien in einer Schicht, aber auch zur Verwendung in Mischung mit einer oder mehreren weiteren Komponenten in der Lochtransportschicht (HTL), Elektronenblockierschicht (EBL) oder emittierenden Schicht (EML).

Verglichen mit Vorrichtungen enthaltend NPB als EBM (V1 und V5) zeigen die Vorrichtungen enthaltend erfindungsgemäße Verbindungen (E1 bis E16) sowohl höhere Effizienzen als auch verbesserte Lebensdauern.

Verglichen mit dem Material gemäß dem Stand der Technik HTMV1 (V2), bei welchem die Carbazol- und die Fluorengruppe direkt über eine Aminogruppe miteinander verbunden sind, zeigen die erfindungsgemäßen Verbindungen ähnliche oder bessere Effizienzen und deutlich bessere Lebensdauern. So ist die Lebensdauer der Referenz-Vorrichtung V2 im Vergleich zu E1 und E2 (blau fluoreszierende Vorrichtungen) nahezu 10-fach länger und auch in der grün phosphoreszierenden Vorrichtung (V6 verglichen mit E9 und E10) ergibt sich noch nahezu eine Verdopplung der Lebensdauer.

Der Vorteil einer 3-Fluoren-Substitution im Vergleich zu einer 2-Fluoren-Substitution ist gut im Vergleich zwischen HTMV2 und HTM6 zu erkennen. In den blau fluoreszierenden Vorrichtungen (V3 und E6) und insbesondere in den grün phosphoreszierenden Vorrichtungen (V7 und E14) zeigen sich bessere Effizienzen und bessere Lebensdauern. Das gleiche ist auch gut im Vergleich von HTMV3 (V8) gegen HTM7 (E15) und HTM8 (E16) zu erkennen. Auch hier zeigt sich insbesondere bei grün phosphoreszierenden Vorrichtungen eine deutlich verbesserte Effizienz und Lebensdauer.

Die oben diskutierten Vorrichtungen wurden lediglich beispielhaft hervorgehoben. Ähnliche Effekte können auch bei den anderen, nicht explizit diskutierten Vorrichtungen beobachtet werden, wie aus den Tabellen mit den Devicedaten ersichtlich ist. Allgemein stellen die mit V1 bis V8 bezeichneten Vorrichtungen Vergleichsbeispiele enthaltend Verbindungen gemäß dem Stand der Technik dar. Die mit E1 bis E16 bezeichneten Vorrichtungen enthalten Verbindungen gemäß der vorliegenden Erfindung und sind somit erfindungsgemäße Beispiele.

**Tabelle 1: Strukturen der verwendeten Materialien**

| | | |
|---|---|---|
| 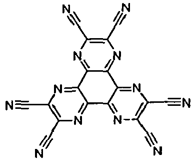 | 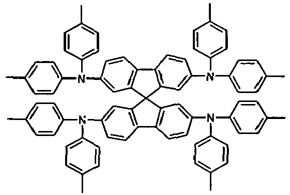 | 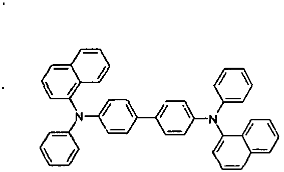 |
| HIL1 | HIL2 | NPB |
| 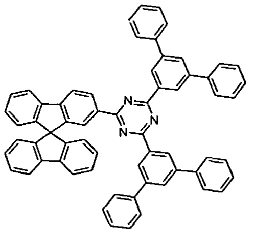 | 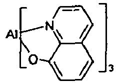 | 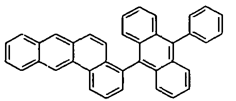 |
| ETM1 | Alq3 | H1 |
| 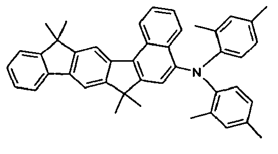 | 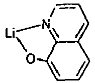 | 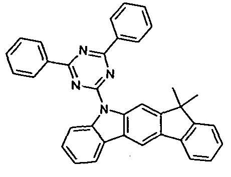 |
| SEB1 | LiQ | H2 |
| 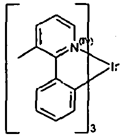 | | |
| Irpy | | |
| 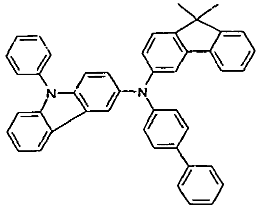 | 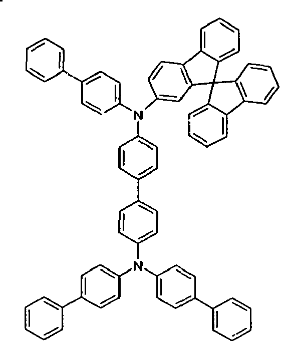 | 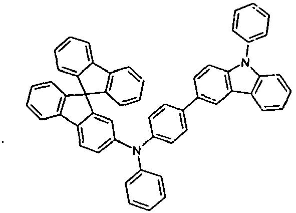 |
| HTMV1 | HTMV2 | HTMV3 |
| 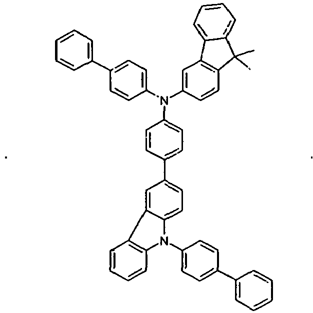 | 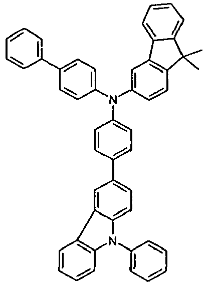 | 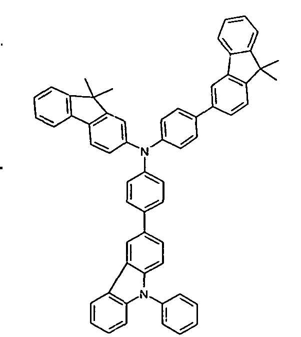 |
| HTM1 (Verbindung 3) | HTM2 (Verbindung 4) | HTM3 (Verbindung 7) |
| 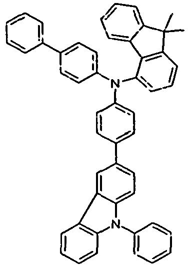 | 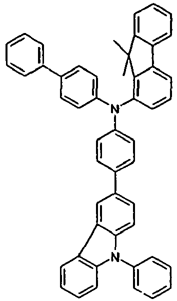 | 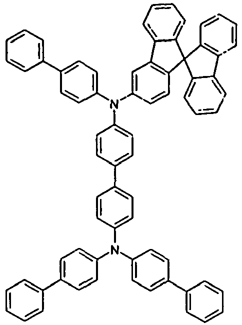 |
| HTM4 (Verbindung 9) | HTM5 (Verbindung 10) | HTM6 (Verbindung 5) |
| 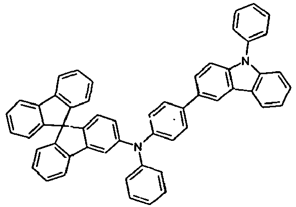 | 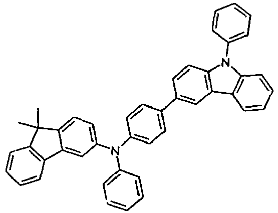 | |
| HTM7 (Verbindung 16) | HTM8 (Verbindung 18) | |

| **Tabelle 2: Aufbau der OLEDs** | | | | | | | |
|---|---|---|---|---|---|---|---|
| ***Bsp.*** | ***IL*** | ***HTL*** | ***IL*** | ***HTL2*** | ***EBL*** | ***EML*** | ***ETL*** |
| | *Dicke* / *nm* | *Dicke* / *nm* | *Dicke* / *nm* | *Dicke* / *nm* | *Dicke* / *nm* | *Dicke* / *nm* | *Dicke* / *nm* |
| *V1* | *HIL1* | *HIL2* | *HIL1* | | *NPB* | *H1(95%):SEB1(5%)* | *ETM1(50%):LiQ(50%)* |
| | *5 nm* | *140 nm* | *5 nm* | | *20 nm* | *20 nm* | *30 nm* |
| *V2* | *HIL1* | *HIL2* | *HIL1* | | *HTMV1* | *H1(95%):SEB1(5%)* | *ETM1(50%):LiQ(50%)* |
| | *5 nm* | *140 nm* | *5 nm* | | *20 nm* | *20 nm* | *30 nm* |
| *V3* | *HIL1* | *HIL2* | *HIL1* | | *HTMV2* | *H1(95%):SEB1(5%)* | *ETM1(50%):LiQ(50%)* |
| | *5 nm* | *140 nm* | *5 nm* | | *20 nm* | *20 nm* | *30 nm* |
| *V4* | *HIL1* | *HIL2* | *HIL1* | | *HTMV3* | *H1(95%):SEB1(5%)* | *ETM1(50%):LiQ(50%)* |
| | *5 nm* | *140 nm* | *5 nm* | | *20 nm* | *20 nm* | *30 nm* |
| *E1* | *HIL1* | *HIL2* | *HIL1* | | *HTM1* | *H1(95%):SEB1(5%)* | *ETM1(50%):LiQ(50%)* |
| | *5 nm* | *140 nm* | *5 nm* | | *20 nm* | *20 nm* | *30 nm* |
| *E2* | *HIL1* | *HIL2* | *HIL1* | | *HTM2* | *H1(95%):SEB1(5%)* | *ETM1(50%):LiQ(50%)* |
| | *5 nm* | *140 nm* | *5 nm* | | *20 nm* | *20 nm* | *30 nm* |
| *E3* | *HIL1* | *HIL2* | *HIL1* | | *HTM3* | *H1(95%):SEB1(5%)* | *ETM1(50%):LiQ(50%)* |
| | *5 nm* | *140 nm* | *5 nm* | | *20 nm* | *20 nm* | *30 nm* |
| *E4* | *HIL1* | *HIL2* | *HIL1* | *NPB* | *HTM4* | *H1(95%):SEB1(5%)* | *ETM1(50%):LiQ(50%)* |
| | *5 nm* | *130 nm* | *5 nm* | *10 nm* | *20 nm* | *20 nm* | *30 nm* |
| *E5* | *HIL1* | *HIL2* | *HIL 1* | *NPB* | *HTM5* | *H1(95%):SEB1(5%)* | *ETM1(50%):LiQ(50%)* |
| | *5 nm* | *130 nm* | *5 nm* | *10 nm* | *20 nm* | *20 nm* | *30 nm* |
| *E6* | *HIL1* | *HIL2* | *HIL1* | | *HTM6* | *H1(95%):SEB1(5%)* | *ETM1(50%):LiQ(50%)* |
| | *5 nm* | *140 nm* | *5 nm* | | *20 nm* | *20 nm* | *30 nm* |
| *E7* | *HIL1* | *HIL2* | *HIL1* | | *HTM7* | *H1(95%):SEB1(5%)* | *ETM1(50%):LiQ(50%)* |
| | *5 nm* | *140 nm* | *5 nm* | | *20 nm* | *20 nm* | *30 nm* |
| *E8* | *HIL1* | *HIL2* | *HIL1* | | *HTM8* | *H1(95%):SEB1(5%)* | *ETM1(50%):LiQ(50%)* |
| | *5 nm* | *140 nm* | *5 nm* | | *20 nm* | *20 nm* | *30 nm* |

| **Tabelle 3: Daten der OLEDs** | | | | | |
|---|---|---|---|---|---|
| ***Bsp.*** | ***U @ 1000 cd*/*m2*** | ***EQE* @ *1000 cd*/*m2*** | ***LD80 @ 6000 cd*/*m²*** | ***CIE*** | |
| | *V* | *%* | *[h]* | *x* | y |
| *V1* | *4.7* | *4.8* | *70* | 0.14 | 0.17 |
| *V2* | *4.4* | *6.5* | *10* | 0.14 | 0.16 |
| *V3* | *4.6* | *5.9* | *60* | 0.14 | 0.16 |
| *V4* | *4.5* | *6.7* | *90* | 0.14 | 0.16 |
| *E1* | *4.5* | *6.8* | *105* | 0.14 | 0.16 |
| *E2* | *4.4* | *6.9* | *110* | 0.14 | 0.16 |
| *E3* | *4.5* | *6.8* | *105* | 0.14 | 0.15 |
| *E4* | *4.6* | 7.0 | *120* | 0.14 | 0.16 |
| *E5* | *4.6* | *6.9* | *110* | 0.14 | 0.16 |
| *E6* | *4.5* | *6.6* | *80* | 0.14 | 0.16 |
| *E7* | *4.5* | *6.8* | *100* | 0.14 | 0.16 |
| *E8* | *4.5* | 6.9 | *95* | 0.14 | 0.16 |

| **Tabelle 4: Aufbau der OLEDs** | | | | | | |
|---|---|---|---|---|---|---|
| ***Bsp.*** | ***HTL*** | ***IL*** | ***HTL2*** | ***EBL*** | ***EML*** | ***ETL*** |
| | *Dicke*/ *nm* | *Dicke*/ *nm* | *Dicke* / *nm* | *Dicke* / *nm* | *Dicke* / *nm* | *Dicke* / *nm* |
| *V5* | *HIL2* | *HIL1* | | *NPB* | *H2(88%):Irpy(12%)* | *ETM1(50%):LiQ(50%)* |
| | *70 nm* | *5 nm* | | *90 nm* | *30 nm* | *40 nm* |
| *V6* | *HIL2* | *HIL1* | | *HTMV1* | *H2(88%):Irpy(12%)* | *ETM1(50%):LiQ(50%)* |
| | *70 nm* | *5 nm* | | *90 nm* | *30 nm* | *40 nm* |
| *V7* | *HIL2* | *HIL1* | | *HTMV2* | *H2(88%):Irpy(12%)* | *ETM1(50%):LiQ(50%)* |
| | *70 nm* | *5 nm* | | *90 nm* | *30 nm* | *40 nm* |
| *V8* | *HIL2* | *HIL1* | | *HTMV3* | *H2(88%):Irpy(12%)* | *ETM1(50%):LiQ(50%)* |
| | *70 nm* | *5 nm* | | *90 nm* | *30 nm* | *40 nm* |
| *E9* | *HIL2* | *HIL1* | | *HTM1* | *H2(88%):Irpy(12%)* | *ETM1(50%):LiQ(50%)* |
| | *70 nm* | *5 nm* | | *90 nm* | *30 nm* | *40 nm* |
| *E10* | *HIL2* | *HIL1* | | *HTM2* | *H2(88%):Irpy(12%)* | *ETM1(50%):LiQ(50%)* |
| | *70 nm* | *5 nm* | | *90 nm* | *30 nm* | *40 nm* |
| *E11* | *HIL2* | *HIL1* | | *HTM3* | *H2(88%):Irpy(12%)* | *ETM1(50%):LiQ(50%)* |
| | *70 nm* | *5 nm* | | *90 nm* | *30 nm* | *40 nm* |
| *E12* | *HIL2* | *HIL1* | *NPB* | *HTM4* | *H2(88%):Irpy(12%)* | *ETM1(50%):LiQ(50%)* |
| | *70 nm* | *5 nm* | *10 nm* | *80 nm* | *30 nm* | *40 nm* |
| *E13* | *HIL2* | *HIL1* | *NPB* | *HTM5* | *H2(88%):Irpy(12%)* | *ETM1(50%):LiQ(50%)* |
| | *70 nm* | 5 *nm* | *10 nm* | *80 nm* | *30 nm* | *40 nm* |
| *E14* | *HIL2* | *HIL1* | | *HTM6* | *H2(88%):Irpy(12%)* | *ETM1(50%):LiQ(50%)* |
| | *70 nm* | *5 nm* | | *90 nm* | *30 nm* | *40 nm* |
| *E15* | *HIL2* | *HIL1* | | *HTM7* | *H2(88%):Irpy(12%)* | *ETM1(50%):LiQ(50%)* |
| | *70 nm* | *5 nm* | | *90 nm* | *30 nm* | *40 nm* |
| *E16* | *HIL2* | *HIL1* | | *HTM8* | *H2(88%):Irpy(12%)* | *ETM1(50%):LiQ(50%)* |
| | *70 nm* | *5 nm* | | *90 nm* | *30 nm* | *40 nm* |

| **Tabelle 5: Daten der OLEDs** | | | | | |
|---|---|---|---|---|---|
| ***Bsp.*** | ***U* @ *1000 cd*/*m2*** | ***Effizienz* @ *1000 cd*/*m2*** | ***LD80* @ *8000 cd*/*m²*** | ***CIE*** | |
| | *V* | *%* | *[h]* | *x* | y |
| *V5* | *3.6* | *14.4* | *85* | 0.32 | 0.63 |
| *V6* | *3.4* | *16.8* | *70* | 0.33 | 0.64 |
| *V7* | *3.4* | *15.6* | *120* | 0.33 | 0.64 |
| *V8* | *3.5* | *16.7* | *135* | 0.33 | 0.63 |
| *E9* | *3.3* | *17.6* | *155* | 0.34 | 0.62 |
| *E10* | *3.3* | *17.8* | *160* | 0.33 | 0.63 |
| *E11* | *3.4* | *17.0* | *145* | 0.33 | 0.64 |
| *E12* | *3.5* | *17.5* | *155* | 0.34 | 0.63 |
| *E13* | *3.5* | *17.8* | *160* | 0.34 | 0.63 |
| *E14* | *3.4* | *16.4* | *145* | 0.33 | 0.63 |
| *E15* | *3.5* | *17.6* | *155* | 0.33 | 0.64 |
| *E16* | *3.4* | *17.8* | *150* | 0.33 | 0.63 |

## Patentansprüche

1. Verbindung der Formel (I), (II) oder (III) 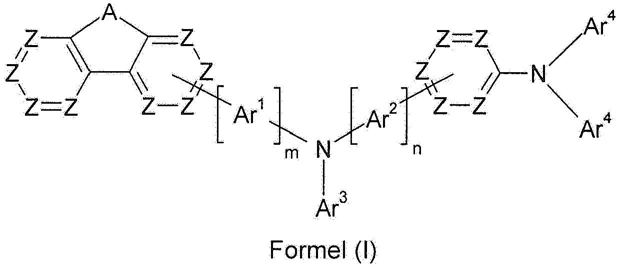 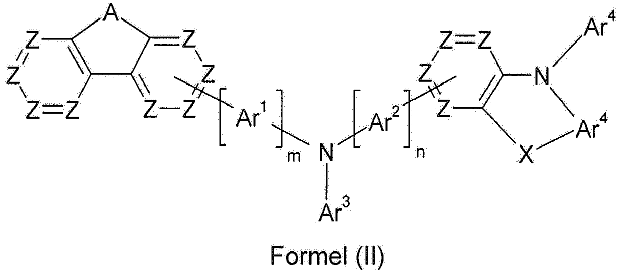 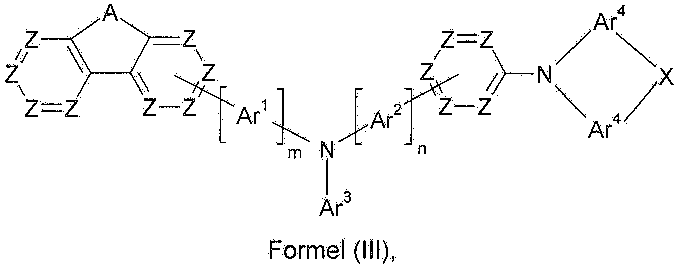 wobei für die auftretenden Symbole und Indices gilt:
A ist für Formel (I) gleich 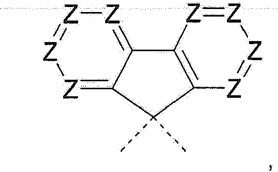 wobei die gestrichelten Linien die Bindungen ausgehend von der Gruppe A darstellen; und
für Formel (II) und (III) gleich C(R¹)₂ oder gleich 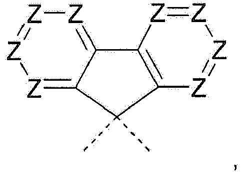 wobei die gestrichelten Linien die Bindungen ausgehend von der Gruppe A darstellen;
Z ist gleich CR¹ oder, wenn eine Gruppe in der betreffenden Position gebunden ist, gleich C;
Ar¹ ist bei jedem Auftreten gleich oder verschieden ein aromatisches oder heteroaromatisches Ringsystem mit 5 bis 30 aromatischen Ringatomen, welches mit einem oder mehreren Resten R¹ substituiert sein kann;
Ar³ ist ein aromatisches Ringsystem mit 6 bis 18 aromatischen Ringatomen, das mit einem oder mehreren Resten R¹ substituiert sein kann;
Ar² ist bei jedem Auftreten gleich oder verschieden eine Arylengruppe mit 6 bis 30 aromatischen Ringatomen oder eine Heteroarylengruppe mit 5 bis 14 aromatischen Ringatomen, welche mit einem oder mehreren Resten R¹ substituiert sein kann;
Ar⁴ ist bei jedem Auftreten gleich oder verschieden eine Arylgruppe mit 6 bis 30 aromatischen Ringatomen, welche mit einem oder mehreren Resten R¹ substituiert sein kann, wobei Reste R¹ an Gruppen Ar⁴ keine Ringe bilden dürfen;
X ist ausgewählt aus einer Einfachbindung, C(R¹)₂, C=O, Si(R¹)₂, NR¹, O, S, S=O und S(=O)₂;
R¹ ist bei jedem Auftreten gleich oder verschieden H, eine geradkettige Alkylgruppe mit 1 bis 20 C-Atomen oder eine verzweigte oder cyclische Alkyllgruppe mit 3 bis 20 C-Atomen, wobei die oben genannten Gruppen jeweils mit einem oder mehreren Resten R² substituiert sein können und wobei ein oder mehrere H-Atome in den oben genannten Gruppen durch D, F, Cl, Br, I, CN oder NO₂ ersetzt sein können, oder ein aromatisches Ringsystem mit 5 bis 30 aromatischen Ringatomen, das jeweils durch einen oder mehrere Reste R² substituiert sein kann, wobei zwei oder mehr Reste R¹ miteinander verknüpft sein können und einen Ring bilden können;
R² ist bei jedem Auftreten gleich oder verschieden H, eine geradkettige Alkylgruppe mit 1 bis 20 C-Atomen oder eine verzweigte oder cyclische Alkylgruppe mit 3 bis 20 C-Atomen, wobei die oben genannten Gruppen jeweils mit einem oder mehreren Resten R³ substituiert sein können und wobei ein oder mehrere H-Atome in den oben genannten Gruppen durch D, F, Cl, Br, I, CN oder NO₂ ersetzt sein können, oder ein aromatisches Ringsystem mit 5 bis 30 aromatischen Ring-atomen, das jeweils durch einen oder mehrere Reste R³ substituiert sein kann, wobei zwei oder mehr Reste R² miteinander verknüpft sein können und einen Ring bilden können;
R³ ist bei jedem Auftreten gleich oder verschieden H oder ein aliphatischer oder aromatischer organischer Rest mit 1 bis 20 C-Atomen, in dem auch ein oder mehrere H-Atome durch D oder F ersetzt sein können; dabei können zwei oder mehr Substituenten R³ miteinander verknüpft sein und einen Ring bilden;
m ist gleich 0, 1, 2 oder 3, wobei m=0 bedeutet, dass die betreffende Gruppe nicht vorhanden ist;
n ist gleich 0, 1, 2 oder 3, wobei n=0 bedeutet, dass die betreffende Gruppe nicht vorhanden ist;
wobei die Gruppe Ar¹ bzw. das Stickstoffatom in 1-Position, in 3-Position oder in 4-Position am Fluoren-Ringsystem gebunden ist;
wobei n in Formel (II) gleich 1 sein muss, wenn die Gruppe X eine Einfachbindung darstellt; und
wobei die Verbindung keine Heteroarylgruppe enthalten darf, welche mehr als 14 aromatische Ringatome enthält.

2. Verbindung nach Anspruch 1, **dadurch gekennzeichnet, dass** die Gruppe Ar¹ oder, für den Fall dass m=0 gilt, die Gruppe N(Ar³), in 3-Position am Fluorenyl-Ringsystem gebunden ist.

3. Verbindung nach Anspruch 1 oder 2, **dadurch gekennzeichnet, dass** A für Formel (II) und Formel (III) gleich C(R¹)₂ ist.

4. Verbindung nach einem oder mehreren der Ansprüche 1 bis 3, **dadurch gekennzeichnet, dass** Ar¹ ein aromatisches Ringsystem mit 6 bis 12 aromatischen Ringatomen darstellt, welches mit einem oder mehreren Resten R¹ substituiert sein kann.

5. Verbindung nach einem oder mehreren der Ansprüche 1 bis 4, **dadurch gekennzeichnet, dass** Ar² eine Phenylengruppe darstellt, welche mit einem oder mehreren Resten R¹ substituiert sein kann.

6. Verbindung nach einem oder mehreren der Ansprüche 1 bis 5, **dadurch gekennzeichnet, dass** die Gruppe X ausgewählt ist aus einer Einfachbindung, C(R¹)₂, O oder S.

7. Verbindung nach einem oder mehreren der Ansprüche 1 bis 6, **dadurch gekennzeichnet, dass** m gleich Null ist.

8. Verbindung nach einem oder mehreren der Ansprüche 1 bis 7, **dadurch gekennzeichnet, dass** n gleich 1 oder 2 ist.

9. Verbindung nach einem oder mehreren der Ansprüche 1 bis 8, **dadurch gekennzeichnet, dass** keine kondensierte Arylgruppe mit mehr als 14 aromatischen Ringatomen in der Verbindung vorhanden ist.

10. Verbindung nach einem oder mehreren der Ansprüche 1 bis 9, **dadurch gekennzeichnet, dass** sie nicht in einer spiegelsymmetrischen Strukturformel dargestellt werden kann.

11. Verfahren zur Herstellung einer Verbindung nach einem oder mehreren der Ansprüche 1 bis 10, **dadurch gekennzeichnet, dass** ein Fluorenyl- oder Spirobifluorenyl-Derivat in einer ersten Kupplungsreaktion mit einer Arylaminoverbindung umgesetzt wird, und das erhaltene Produkt in einer zweiten Kupplungsreaktion mit einer Triarylamino- oder Carbazolverbindung umgesetzt wird.

12. Oligomer, Polymer oder Dendrimer enthaltend eine oder mehrere Verbindungen nach einem oder mehreren der Ansprüche 1 bis 10, wobei die Bindungen zum Oligomer, Polymer oder Dendrimer an beliebigen, in Formel (I), (II) oder (III) mit R¹ substituierten Positionen lokalisiert sein können.

13. Formulierung enthaltend mindestens eine Verbindung nach einem oder mehreren der Ansprüche 1 bis 10 oder mindestens ein Polymer, Oligomer oder Dendrimer gemäß Anspruch 12 und mindestens ein Lösungsmittel.

14. Elektronische Vorrichtung, enthaltend mindestens eine Verbindung gemäß einem oder mehreren der Ansprüche 1 bis 10 oder mindestens ein Polymer, Oligomer oder Dendrimer gemäß Anspruch 12.

15. Elektronische Vorrichtung gemäß Anspruch 14, **dadurch gekennzeichnet, dass** sie ausgewählt ist aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs).

16. Elektronische Vorrichtung gemäß Anspruch 15, gewählt aus organischen Elektrolumineszenzvorrichtungen (OLEDs), **dadurch gekennzeichnet, dass** die Verbindung gemäß einem oder mehreren der Ansprüche 1 bis 10 oder das Polymer, Oligomer oder Dendrimer gemäß Anspruch 12 in einer oder mehreren der folgenden Funktionen eingesetzt wird:
- als Lochtransportmaterial in einer Lochtransport- oder Lochinjektionsschicht,
- als Matrixmaterial in einer emittierenden Schicht,
- als Elektronenblockiermaterial,
- als Excitonenblockiermaterial,
- als Material für eine Zwischenschicht (Interlayer).

## Claims

1. Compound of the formula (I), (II) or (III) 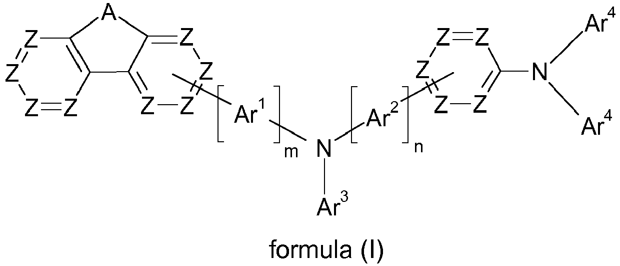 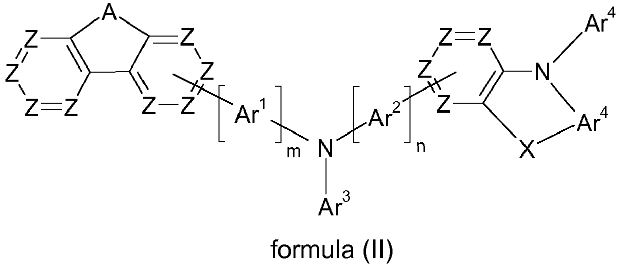 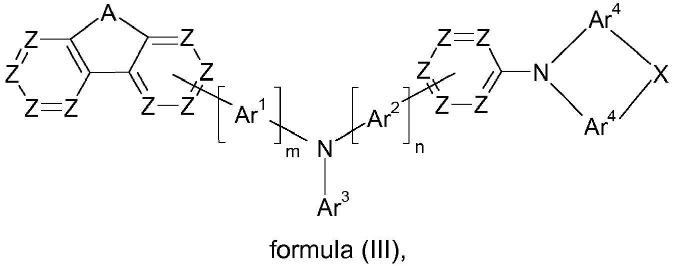 where the following applies to the symbols and indices occurring:
A for formula (I) is equal to 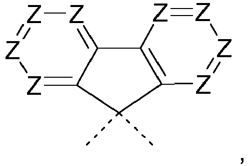 where the dashed lines represent the bonds starting from group A; and
for formulae (II) and (III) is equal to C(R¹)₂ or equal to 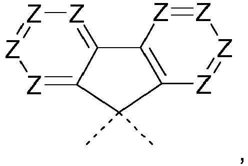 where the dashed lines represent the bonds starting from group A;
Z is equal to CR¹ or, if a group is bonded in the position in question, is equal to C;
Ar¹ is on each occurrence, identically or differently, an aromatic or heteroaromatic ring system having 5 to 30 aromatic ring atoms, which may be substituted by one or more radicals R¹;
Ar³ is an aromatic ring system having 6 to 18 aromatic ring atoms, which may be substituted by one or more radicals R¹;
Ar² is on each occurrence, identically or differently, an arylene group having 6 to 30 aromatic ring atoms or a heteroarylene group having 5 to 14 aromatic ring atoms, which may be substituted by one or more radicals R¹;
Ar⁴ is on each occurrence, identically or differently, an aryl group having 6 to 30 aromatic ring atoms, which may be substituted by one or more radicals R¹, where radicals R¹ on groups Ar⁴ cannot form rings;
X is selected from a single bond, C(R¹)₂, C=O, Si(R¹)₂, NR¹, O, S, S=O and S(=O)₂;
R¹ is on each occurrence, identically or differently, H, a straight-chain alkyl group having 1 to 20 C atoms or a branched or cyclic alkyl group having 3 to 20 C atoms, where the above-mentioned groups may in each case be substituted by one or more radicals R² and where one or more H atoms in the above-mentioned groups may be replaced by D, F, Cl, Br, I, CN or NO₂, or an aromatic ring system having 5 to 30 aromatic ring atoms, which may in each case be substituted by one or more radicals R², where two or more radicals R¹ may be linked to one another and may form a ring;
R² is on each occurrence, identically or differently, H, a straight-chain alkyl group having 1 to 20 C atoms or a branched or cyclic alkyl group having 3 to 20 C atoms, where the above-mentioned groups may in each case be substituted by one or more radicals R³ and where one or more H atoms in the above-mentioned groups may be replaced by D, F, Cl, Br, I, CN or NO₂, or an aromatic ring system having 5 to 30 aromatic ring atoms, which may in each case be substituted by one or more radicals R³, where two or more radicals R² may be linked to one another and may form a ring;
R³ is on each occurrence, identically or differently, H or an aliphatic or aromatic organic radical having 1 to 20 C atoms, in which, in addition, one or more H atoms may be replaced by D or F; two or more substituents R³ may be linked to one another and form a ring;
m is equal to 0, 1, 2 or 3, where m = 0 means that the group in question is not present;
n is equal to 0, 1, 2 or 3, where n = 0 means that the group in question is not present;
where the group Ar¹ or the nitrogen atom is bonded in position 1, in position 3 or in position 4 on the fluorene ring system;
where n in formula (II) must be equal to 1 if the group X represents a single bond; and
where the compound cannot contain a heteroaryl group which contains more than 14 aromatic ring atoms.

2. Compound according to Claim 1, **characterised in that** the group Ar¹ or, in the case where m = 0, the group N(Ar³), is bonded in position 3 on the fluorenyl ring system.

3. Compound according to Claim 1 or 2, **characterised in that** A for formula (II) and formula (III) is equal to C(R¹)₂.

4. Compound according to one or more of Claims 1 to 3, **characterised in that** Ar¹ represents an aromatic ring system having 6 to 12 aromatic ring atoms, which may be substituted by one or more radicals R¹.

5. Compound according to one or more of Claims 1 to 4, **characterised in that** Ar² represents a phenylene group, which may be substituted by one or more radicals R¹.

6. Compound according to one or more of Claims 1 to 5, **characterised in that** the group X is selected from a single bond, C(R¹)₂, O or S.

7. Compound according to one or more of Claims 1 to 6, **characterised in that** m is equal to zero.

8. Compound according to one or more of Claims 1 to 7, **characterised in that** n is equal to 1 or 2.

9. Compound according to one or more of Claims 1 to 8, **characterised in that** no condensed aryl group having more than 14 aromatic ring atoms is present in the compound.

10. Compound according to one or more of Claims 1 to 9, **characterised in that** it cannot be represented in a mirror-symmetrical structural formula.

11. Process for the preparation of a compound according to one or more of Claims 1 to 10, **characterised in that** a fluorenyl or spirobifluorenyl derivative is reacted with an arylamino compound in a first coupling reaction, and the product obtained is reacted with a triarylamino or carbazole compound in a second coupling reaction.

12. Oligomer, polymer or dendrimer containing one or more compounds according to one or more of Claims 1 to 10, where the bonds to the oligomer, polymer or dendrimer may be localised at any desired positions in formula (I), (II) or (III) that are substituted by R¹.

13. Formulation comprising at least one compound according to one or more of Claims 1 to 10 at least one polymer, oligomer or dendrimer according to Claim 12 and at least one solvent.

14. Electronic device containing at least one compound according to one or more of Claims 1 to 10 at least one polymer, oligomer or dendrimer according to Claim 12.

15. Electronic device according to Claim 14, **characterised in that** it is selected from organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs).

16. Electronic device according to Claim 15, selected from organic electroluminescent devices (OLEDs), **characterised in that** the compound according to one or more of Claims 1 to 10 or the polymer, oligomer or dendrimer according to Claim 12 is employed in one or more of the following functions:
- as hole-transport material in a hole-transport or hole-injection layer,
- as matrix material in an emitting layer,
- as electron-blocking material,
- as exciton-blocking material,
- as material for an interlayer.

## Revendications

1. Composé de la formule (I), (II) ou (III) : 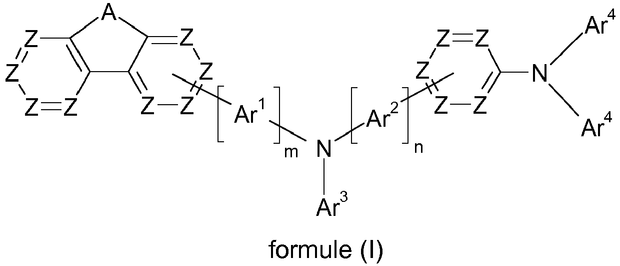 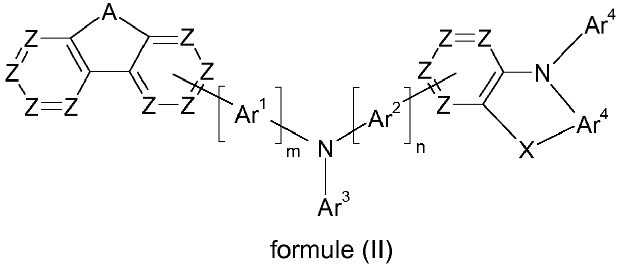 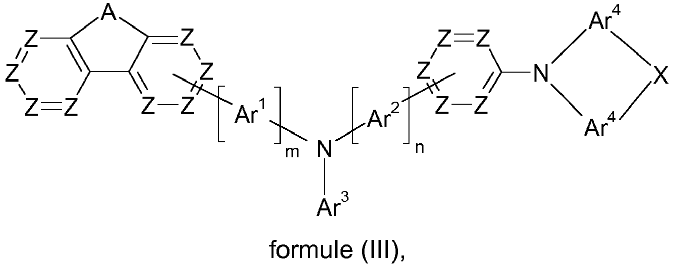 dans lesquelles ce qui suit s'applique aux symboles et aux indices qui sont rencontrés :
A pour la formule (I), est égal à 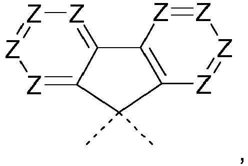 où les lignes en pointillés représentent les liaisons qui partent du groupe A ; et
pour les formules (II) et (III), est égal à C(R¹)₂ ou est égal à 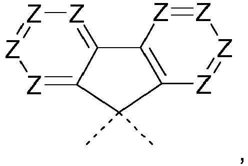 où les lignes en pointillés représentent les liaisons qui partent du groupe A ;
Z est égal à CR¹ ou, si un groupe est lié au niveau de la position en question, est égal à C ;
Ar¹ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux R¹ ;
Ar³ est un système de cycle aromatique qui comporte de 6 à 18 atomes de cycle aromatique, lequel peut être substitué par un radical ou par plusieurs radicaux R¹ ;
Ar² est pour chaque occurrence, de manière identique ou différente, un groupe arylène qui comporte de 6 à 30 atomes de cycle aromatique ou un groupe hétéroarylène qui comporte de 5 à 14 atomes de cycle aromatique, lequel peut être substitué par un radical ou par plusieurs radicaux R¹ ;
Ar⁴ est pour chaque occurrence, de manière identique ou différente, un groupe aryle qui comporte de 6 à 30 atomes de cycle aromatique, lequel peut être substitué par un radical ou par plusieurs radicaux R¹, où des radicaux R¹ sur les groupes Ar⁴ ne peuvent pas former de cycles ;
X est sélectionné parmi une liaison simple, C(R¹)₂, C=O, Si(R¹)₂, NR¹, O, S, S=O et S(=O)₂ ;
R¹ est pour chaque occurrence, de manière identique ou différente, H, un groupe alkyle en chaîne droite qui comporte de 1 à 20 atome(s) de C ou un groupe alkyle ramifié ou cyclique qui comporte de 3 à 20 atomes de C, où les groupes qui ont été mentionnés ci-avant peuvent dans chaque cas être substitués par un radical ou par plusieurs radicaux R² et où un ou plusieurs atome(s) de H dans les groupes qui ont été mentionnés ci-avant peut/peuvent être remplacé(s) par D, F, Cl, Br, I, CN ou NO₂, ou un système de cycle aromatique qui comporte de 5 à 30 atomes de cycle aromatique, lequel peut dans chaque cas être substitué par un radical ou par plusieurs radicaux R², où deux radicaux R¹ ou plus peuvent être liés l'un à l'autre ou les uns aux autres et peuvent former un cycle ;
R² est pour chaque occurrence, de manière identique ou différente, H, un groupe alkyle en chaîne droite qui comporte de 1 à 20 atome(s) de C ou un groupe alkyle ramifié ou cyclique qui comporte de 3 à 20 atomes de C, où les groupes qui ont été mentionnés ci-avant peuvent dans chaque cas être substitués par un radical ou par plusieurs radicaux R³ et où un ou plusieurs atomes de H dans les groupes qui ont été mentionnés ci-avant peut/peuvent être remplacé(s) par D, F, Cl, Br, I, CN ou NO₂, ou un système de cycle aromatique qui comporte de 5 à 30 atomes de cycle aromatique, lequel peut dans chaque cas être substitué par un radical ou par plusieurs radicaux R³, où deux radicaux R² ou plus peuvent être liés l'un à l'autre ou les uns aux autres et peuvent former un cycle ;
R³ est pour chaque occurrence, de manière identique ou différente, H ou un radical organique aliphatique ou aromatique qui comporte de 1 à 20 atome(s) de C, où, en outre, un ou plusieurs atome(s) de H peut/peuvent être remplacé(s) par D ou F ; deux substituants R³ ou plus peuvent être liés l'un à l'autre ou les uns aux autres et peuvent former un cycle ;
m est égal à 0, 1, 2 ou 3, où m = 0 signifie que le groupe en question n'est pas présent ;
n est égal à 0, 1, 2 ou 3, où n = 0 signifie que le groupe en question n'est pas présent ;
où le groupe Ar¹ ou l'atome d'azote est lié au niveau de la position 1, de la position 3 ou de la position 4 sur le système de cycle fluorène ;
où n dans la formule (II) doit être égal à 1 si le groupe X représente une liaison simple ; et
où le composé ne peut pas contenir un groupe hétéroaryle qui contient plus de 14 atomes de cycle aromatique.

2. Composé selon la revendication 1, **caractérisé en ce que** le groupe Ar¹ ou, dans le cas où m = 0, le groupe N(Ar³), est lié au niveau de la position 3 sur le système de cycle fluorényle.

3. Composé selon la revendication 1 ou 2, **caractérisé en ce que** A pour la formule (II) et pour la formule (III) est égal à C(R¹)₂.

4. Composé selon une ou plusieurs des revendications 1 à 3, **caractérisé en ce que** Ar¹ représente un système de cycle aromatique qui comporte de 6 à 12 atomes de cycle aromatique, lequel peut être substitué par un radical ou par plusieurs radicaux R¹.

5. Composé selon une ou plusieurs des revendications 1 à 4, **caractérisé en ce que** Ar² représente un groupe phénylène, lequel peut être substitué par un radical ou par plusieurs radicaux R¹.

6. Composé selon une ou plusieurs des revendications 1 à 5, **caractérisé en ce que** le groupe X est sélectionné parmi une liaison simple, C(R¹)₂, O ou S.

7. Composé selon une ou plusieurs des revendications 1 à 6, **caractérisé en ce que** m est égal à zéro.

8. Composé selon une ou plusieurs des revendications 1 à 7, **caractérisé en ce que** n est égal à 1 ou 2.

9. Composé selon une ou plusieurs des revendications 1 à 8, **caractérisé en ce qu'**aucun groupe aryle condensé qui comporte plus de 14 atomes de cycle aromatique n'est présent dans le composé.

10. Composé selon une ou plusieurs des revendications 1 à 9, **caractérisé en ce qu'**il ne peut pas être représenté selon une formule structurelle en symétrie miroir.

11. Procédé pour la préparation d'un composé selon une ou plusieurs des revendications 1 à 10, **caractérisé en ce qu'**un dérivé de fluorényle ou un dérivé de spirobifluorényle est amené à réagir avec un composé arylamino selon une première réaction de couplage, et le produit qui est obtenu est amené à réagir avec un composé triarylamino ou carbazole selon une seconde réaction de couplage.

12. Oligomère, polymère ou dendrimère contenant un ou plusieurs composé(s) selon une ou plusieurs des revendications 1 à 10, où les liaisons sur l'oligomère, le polymère ou le dendrimère peuvent être localisées au niveau de n'importe quelles positions souhaitées dans la formule (I), (II) ou (III) qui sont substituées par R¹.

13. Formulation comprenant au moins un composé selon une ou plusieurs des revendications 1 à 10, au moins un polymère, un oligomère ou un dendrimère selon la revendication 12 et au moins un solvant.

14. Dispositif électronique contenant au moins un composé selon une ou plusieurs des revendications 1 à 10, au moins un polymère, un oligomère ou un dendrimère selon la revendication 12.

15. Dispositif électronique selon la revendication 14, **caractérisé en ce qu'**il est sélectionné parmi les circuits intégrés organiques (O-IC), les transistors à effet de champ organiques (O-FET), les transistors à film mince organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC), les diodes laser organiques (O-laser) et les dispositifs électroluminescents organiques (OLED).

16. Dispositif électronique selon la revendication 15, sélectionné parmi les dispositifs électroluminescents organiques (OLED), **caractérisé en ce que** le composé selon une ou plusieurs des revendications 1 à 10 ou le polymère, l'oligomère ou le dendrimère selon la revendication 12 est utilisé selon une ou plusieurs des fonctions qui suivent :
- en tant que matériau de transport de trous dans une couche de transport de trous ou d'injection de trous ;
- en tant que matériau de matrice dans une couche d'émission ;
- en tant que matériau de blocage d'électrons ;
- en tant que matériau de blocage d'excitons ;
- en tant que matériau pour une intercouche.
